(19) Europäisches Patentamt / European Patent Office / Office européen des brevets

(11) **EP 1 723 255 B1**

(12) **EUROPEAN PATENT SPECIFICATION**

(45) Date of publication and mention of the grant of the patent: **29.12.2010 Bulletin 2010/52**

(21) Application number: **05700655.3**

(22) Date of filing: **17.02.2005**

(51) Int Cl.: ***C12Q 1/68*** *(2006.01)* ***C12N 15/10*** *(2006.01)*

(86) International application number: **PCT/DK2005/000106**

(87) International publication number: **WO 2005/078122 (25.08.2005 Gazette 2005/34)**

# (54) METHOD FOR ENRICHMENT INVOLVING ELIMINATION BY MISMATCH HYBRIDISATION

ANREICHERUNGSVERFAHREN MIT ELIMINIERUNG DURCH FEHLPAARUNGSHYBRIDISIERUNG

METHODE D'ELIMINATION VISANT A UN ENRICHISSEMENT PAR HYBRIDATION DE MESAPPARIEMENTS

(84) Designated Contracting States:
**AT BE BG CH CY CZ DE DK EE ES FI FR GB GR HU IE IS IT LI LT LU MC NL PL PT RO SE SI SK TR**

(30) Priority: **17.02.2004 DK 200400240**
**17.02.2004 US 544343 P**

(43) Date of publication of application: **22.11.2006 Bulletin 2006/47**

(73) Proprietor: **Nuevolution A/S**
**2100 Copenhagen (DK)**

(72) Inventors:
- **FRESKAARD, Per-Ola**
  **S-603 79 Norrköping (SE)**
- **LUNDORF, Mikkel, Dybro**
  **DK-4000 Roskilde (DK)**
- **FRANCH, Thomas**
  **DK-3070 Snekkersten (DK)**

(74) Representative: **Høiberg A/S**
**St. Kongensgade 59 A**
**1264 Copenhagen K (DK)**

(56) References cited:
**WO-A-00/23458** **WO-A-02/34948**
**WO-A-96/41011** **WO-A-98/01562**
**US-A1- 2002 048 760**

- **FACK FRED ET AL: "Heteroduplex Mobility Assay (HMA) pre-screening: An improved strategy for the rapid identification of inserts selected from phage-displayed peptide libraries" MOLECULAR DIVERSITY, vol. 5, no. 1, 2000, pages 7-12, XP002342761 ISSN: 1381-1991**
- **BROUDE NATALIA E: "Stem-loop oligonucleotides: A robust tool for molecular biology and biotechnology" TRENDS IN BIOTECHNOLOGY, vol. 20, no. 6, June 2002 (2002-06), pages 249-256, XP002342762 ISSN: 0167-7799**
- **BRENNER S; LERNER R A: "ENCODED COMBINATORIAL CHEMISTRY" PROCEEDINGS OF THE NATIONAL ACADEMY OF SCIENCES OF USA, vol. 89, no. 12, 1 June 1992 (1992-06-01), pages 5381-5383, XP000647936**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field of the Invention

The present invention relates to a method for obtaining the identity of one or more display molecules capable of associating with a molecular target. The display molecules initially form part of a library and the present invention devises a method for identifying such display molecules that possess certain properties relative to a target.

### Background

Methods for obtaining information about a display molecule that possesses a binding characteristic towards a molecular target are used in the phage display area and are generally known as panning. A typical panning protocol includes a library of phages displaying certain specific polypeptides and a target. When the library and the target are mixed, polypeptides that have an ability to bind to the target will form an association complex. Polypeptides that do not bind are washed away.

In theory, it should be possible to rank the members of the library in accordance with their binding affinity in an elution step. Thus, the best binders could easily be identified following a single mixing step. However, practical experiments show that libraries of many members cannot properly be partitioned for good and bad binders and a second contacting with the target is necessary. Generally, libraries of the size $10^8$ to $10^{12}$ are used to increase the chance of finding a successfully binding polypeptide. Therefore, several rounds are generally required. To obtain a sufficient quantity of phages for performing a second contacting with a molecular target, the phages harbouring the binding polypeptides are amplified using the genetic material (DNA or RNA) of the eluted binding phages. It is usual to perform 3 to 12 rounds of contact between the increasingly enriched libraries and the target before a minor group of binding peptides can be identified.

Systems that resemble the phage panning have been evolved by the present applicant (WO 02/103008 A2) however with the possibility of displaying other molecules than polypeptides. Other systems using the same principles as the phage panning include WO 98/31700, WO 93/03172, and WO 00/23458.

For some systems, e.g. as disclosed in EP 643 778 B1 and WO 93/06121 A1, it is not immediately possible to perform amplification of the complexes after elution of binding display molecules associated with an identifying nucleic acid.

One known method for selecting useful molecules from a library is described in PCT application with publication no. WO 2004/099441 (Hyscite Discovery A/S). This method makes use of a primary library, which comprises the candidate molecules of the library marked with nucleic acid tags, and a secondary library, which is used for amplifying and identifying the nucleic acid tags of the molecules in the primary library.

The present invention aims at providing a method that only requires a single initial contact between a target and a library of display molecules associated with an identifying nucleic acid even for large libraries. Thus, reiterated amplification of display molecules associated with an identifying or coding nucleic acid between each round of contact with the target can be avoided. Moreover, encoding methods not previous available for large libraries becomes at the disposal of the person skilled in the art.

Further background art includes WO 96/41011, wherein a method for tracking and identifying sub-populations of molecules using oligonucleotide tags is described as well as Fack, F., et al. (2000), "Heteroduplex mobility assay (HMA) pre-screening: An improved strategy for the rapid identification of inserts selected from phage displayed peptide libraries" (Mol. Divers.; 5(1): 7-12), which is concerned with the selection of positive colonies from a phage library, without having to carry out extensive sequence analysis.

Additionally, in the article "Encoded combinatorial chemistry" (Proc. Natl. Acad. Sci. (US) 1992; 89:5381-5383) Brenner, S. and Lerner, R. A. describes bifunctional libraries where decoding is done by PCR but does not describe or suggest separation of homoduplexes from heteroduplexes.

### Summary of the Invention

The present invention relates to a method for obtaining display molecule(s) having affinity towards a target, comprising the steps of: a) providing a library comprising a plurality of different display molecules, each display molecule being associated with an identifier oligonucleotide, which codes for the identity of said display molecule, b) contacting the library with a target to allow for an interaction between the display molecules of the library with the target, c) partitioning a fraction preferably enriched with identifier oligonucleotides of display molecules interacting with the target, d) subjecting the fraction to conditions at which hetero-duplexes and homo-duplexes are formed, e) recovering the homo-duplexes, and f) deducing from the homo-duplexes the identity of the display molecule(s) interacting with the target.

An ideal initial library comprises an equal amount of each display molecule associated with the identifier oligonucleotide. The display molecule associated with the identifier oligonucleotide is also for short termed "complex" in the following. It is estimated that a standard vial can comprise around $10^{14}$ complexes. Therefore a library of $10^8$ complexes ideally comprises $10^6$ copies of each complex.

The contacting between the library and the molecular target, and the subsequent partitioning of a fraction of the complexes or just the identifier oligonucleotides thereof, interacting with the target forms an imbalance in the amount of the individual members of the library. If elution is used for the partitioning, complexes binding with high affinity to the target will be eluted in a relatively higher amounts compared to low affinity binding complexes, which will be eluted in a correspondingly minor amount.

The obtained imbalance at the nucleic acid level is used in the subsequent steps. The identifier oligonucleotide parts of the binding complexes are usually, but not necessarily, amplified by PCR or similar to obtain a higher total amount of oligonucleotides while retaining the proportion between the individual oligonucleotides.

The PCR amplification provides a homo-duplex product. A mixture of homo- and hetero-duplexes is appropriately initiated by subjecting the PCR amplification product to a denaturing process to separate the duplexes into single stranded oligonucleotides. Subsequently, the mixture of single stranded oligonucleotides is allowed to hybridise to form the mixture of homo- and hetero-duplexes. The denaturing step is suitably obtained by heating the nucleic acids above the melting temperature of the duplexes and the hybridisation step is suitably conducted by lowering the temperature below the melting temperature of the duplexes. Due to the presence of various different single stranded oligonucleotides some oligonucleotides may find a perfect matching partner and form a homo-duplex, while other oligonucleotides will hybridise to non-complementary binding partners and form hetero-duplexes. The present invention takes advantage of the fact that the oligonucleotides most abundant easier will find perfect binding partners and form homo-duplexes.

After the formation of the mixture of homo- and hetero-duplexes has been allowed the homo-duplexes are recovered. Recovery is usually conducted by eliminating or reducing the amount of hetero-duplexes. Several methods are available for reducing the amount of hetero-duplexes, including dHPLC as disclosed in US 5,795,976, and enzymatic degradation. In some aspects of the invention enzymatic degradation is preferred due to the availability of enzymes specifically locating one or more mis-match pairing nucleobases.

The recovered pool of homo-duplexes may not fully be depleted for hetero-duplexes and/or the diversity of the pool may still be too high for a meaningful decoding. In an aspect of the invention, the step of forming a mixture of homo- and hetero-duplexes is therefore repeated one or more times, i.e. the recovered pool is, optionally after nucleic acid amplification, subjected to denaturing conditions and subsequent to hybridisation conditions to allow for the formation of a new mixture of homo- and hetero-duplexes. The new mixture is treated as stated above to recover the homo-duplexes.

The identity of the display molecules that possessed the ability of binding to the molecular target is finally revealed by decoding the homo-duplexes, using conventional methods.

**Brief Description of the Figures**

Fig. 1 discloses a schematic representation of a selection process.
Fig. 2 depicts a homo- and a hetero-duplex.
Fig. 3 shows a library treated in accordance with the method of the invention.
Fig. 4 discloses the overall principle of mis-match selection.

**Detailed Description of the Invention**

The target may be of a biological origin or may be synthetic molecular target. Typically, the molecular target stems from an organism selected from human and animals, especially vertebras. However, in other embodiments the target may originate from a plant. In the quest for a compound with therapeutical effect on the human or animal body, the target is usually expected to have an importance in a therapeutically theory that combats a certain disease. In the quest for discovering compounds with plant protective effect, the target is usually expected to originate from an organism that harms the crop or a competing undesired plant. The organism may be a fungus when a compound with fungicide effect is searched for or an insect when a compound having insecticide effect is desired. Optionally, a protein target stemming from a biological origin may be derivatised by altering, adding, or deleting one or more amino acids.

The target may be a protein, a small molecular hormone, a lipid, a polysaccharide, a whole cell, a nucleic acid, a metabolite, a heme group, etc. In a preferred aspect the target is a protein. The protein may serve the function in the organism of being an enzyme, a hormone, a structural element, a regulatory protein, a membrane channel or pump, a part of a signal transducing cascade, an antibody, etc. Suitable target enzymes include kinases, phosphatates, and proteases. The protein may occur as an independent entity or may be dimers, trimers, tetramers, or polymers and the protein may comprise a prosthetic group. Furthermore, the molecular target may be a soluble or insoluble agglomerate

of one or more proteins and one or more substituents occurring in the body or artificial components. In another preferred embodiment, the molecular target is a nucleic acid, such as DNA or RNA aptamer or ribozyme.

The target may be immobilized to a solid support. The solid support can be a bead or the surfaces of a well. The target immobilized on the solid support may also form a stable or quasi-stable dispersion in the media; for example the immobilized target may be covalently (or otherwise) coupled to beads or matrices formed by a polymer such as a polymer selected from the group consisting of:

polystyrene, polyethyleneglycol, α-peptides, β-peptides, γ-peptides, ω-peptides, mono-, di- and tri-substituted α-peptides, β-peptides, γ-peptides, ω-peptides, peptides wherein the amino acid residues are in the L-form or in the D-form, vinylogous polypeptides, glycopoly-peptides, polyamides, vinylogous sulfonamide peptides, polysulfonamides, conjugated peptides comprising e.g. prosthetic groups, polyesters, polysaccharides, polycarbamates, polycarbonates, polyureas, polypeptidylphosphonates, polyurethanes, azatides, oligo N-substituted glycines, polyethers, ethoxyformacetal oligomers, poly-thioethers, polyethylene glycols (PEG), polyethylenes, polydisulfides, polyarylene sulfides, polynucleotides, PNAs, LNAs, morpholinos, oligo pyrrolinones, polyoximes, polyimines, polyethyleneimines, polyimides, polyacetals, polyacetates, polystyrenes, polyvinyl, lipids, phospholipids, glycolipids, polycyclic compounds comprising e.g. aliphatic or aromatic cycles, including polyheterocyclic compounds, proteoglycans, and polysiloxanes, including any combination thereof.

More preferably, said polymer is polystyrene or polyethyleneglycol

In a certain embodiment, the target is in solution and all the interaction occur in the solution too. The absence of an immobilization step generally reduces the background noise because there is no background surface to associate to. Thus the result of the assay may be more sensitive. In solution, the only background noise imaginable is when the oligonucleotides or display molecules of the library of complexes binds unspecific to the target molecules. The absence of an immobilization step generally necessitates a subsequent recovery step, e.g. by chromatography.

In certain aspects of the invention, it is preferred to immobilize the target on a solid support. The solid support may be beads of a column or the surface of a container. The immobilisation of the molecular target may ease the removal of the non-binding complexes by washing or similar means. In a certain embodiment, a cleavable linkage between the molecular target and the solid support is present. The cleavable linker is preferably selectively cleavable, that is, the linkage can be cleaved without cleaving other linkages in the target or the complexes. The cleavage of the linkage between the molecular target and the solid support may reduce the contribution from the background, such as complexes associated with the surface of the solid support and not binding to the molecular target.

The target may be obtained in any suitable way. A variety of targets are commercially available, either as purified protein or as the corresponding cDNA. Other protein or peptide targets may be isolated from tissues or mRNA (or the corresponding cDNA) may be extracted from a tissue. Smaller peptides may be synthesised chemically using the standard solid-phase Fmoc peptide synthesis. When nucleic acids are used or included in the molecular target, it may be synthesised using the standard amedite synthesis method or by using the natural machinery.

It may be an advantage to have all or at least a part of the identifier oligonucleotides on a double stranded form during the contacting with the molecular target, as certain nucleic acids may perform a binding interaction or a catalytical action on the components present during the contacting step. Thus, in one embodiment of the invention, the identifier oligonucleotide partly or fully is hybridised to a complementing oligonucleotide.

The identifier oligonucleotide comprises the information necessary for decoding the identity of the display molecule. The identifier oligonucleotide may be analysed directly in some instances to reveal the identity of the display molecules that have performed an interaction with the target. The informative part can be decoded in a standard sequencing machine. In general however, it is preferred to include the informative part of the coupled product in to a suitable vector and transfer the vector to a host organism. The host organisms may then be cultivated on a suitable substrate and allowed to form colonies. Samples from the colonies may be used for sequencing in a sequencing machine. Also, the identification may be conducted using any sequencing method known in the art, including QPCR, microarrays, etc.

<u>Display molecule and identifier oligonucleotide association</u>

The display molecule associated with the identifier oligonucleotide is sometimes herein referred to as a bifunctional complex to indicate that a physical connection between the display molecule and the identifier oligonucleotide normally is present. However, in certain embodiments of the present invention the association between the display molecule and the identifier oligonucleotide may be spatial, i.e. an identifier oligonucleotide specifies the spatial position of a display molecule. The term "bifunctional complex" is intended also to cover the latter embodiment. The identifier oligonucleotide comprises identifying moieties that identify the display molecule. Preferably, the identifier oligonucleotide identifies the molecule uniquely, i.e. in a library of complexes a particular identifier oligonucleotide is capable of distinguishing the molecule it is associated with from the rest of the display molecules.

The display molecule and the identifier oligonucleotide may be attached directly to each other or through a bridging moiety. In one aspect of the invention, the bridging moiety is a selectively cleavable linkage.

The identifier oligonucleotide may comprise one, two or more codons. The codon sequences can be decoded to identify reactants used in the formation of the display molecule. When the identifier oligonucleotide comprises more than one codon, each member of a pool of chemical entities can be identified and the order of codons is informative of the synthesis step each member has been incorporated in.

The sequence of the nucleotides in each codon may have any suitable length. The codon may be a single nucleotide or a plurality of nucleotides. In some aspects of the invention, it is preferred that each codon independently comprises four or more nucleotides, more preferred 4 to 30 nucleotides.

The identifier oligonucleotide will in general have at least two codons arranged in sequence, i.e. next to each other. Two neighbouring codons may be separated by a framing sequence. Depending on the display molecule formed, the identifier oligonucleotide may comprise further codons, such as 3, 4, 5, or more codons. Each of the further codons may be separated by a suitable framing sequence. Preferably, all or at least a majority of the codons of the identifier oligonucleotide are separated from a neighbouring codon by a framing sequence. The framing sequence may have any suitable number of nucleotides, e.g. 1 to 20. Alternatively, codons on the identifier oligonucleotide may be designed with overlapping sequences.

The framing sequence, if present, may serve various purposes. In one setup of the invention, the framing sequence identifies the position of the codon.

Usually, the framing sequence either upstream or downstream of a codon comprises information, which allows determination of the position of the codons. In another setup, the frames have alternating sequences, allowing for addition of building blocks from two pools in the formation of the library. The framing sequence may also or in addition provide for a region of high affinity. The high affinity region may ensure that the hybridisation of the template with an anti-codon will occur in frame. Moreover, the framing sequence may adjust the annealing temperature to a desired level.

By a molecule having an "affinity" for molecule X is meant herein that a molecule with affinity for molecule X will bind to molecule X in a certain detectable amount under certain conditions but will not (optionally detectably) bind other, different molecules (for which it does not have affinity for) to the same extent under identical conditions. One measure to describe a molecule's affinity to another molecule is a dissociation constant, Kd. The smaller the Kd, the stronger the affinity. Dissociation constants can be determined using methods well-known in the art, such as surface plasmon resonance analysis. Herein, it is preferred that a molecule with "affinity" for another molecule X has a Kd for said molecule X that is less than 100 mM, such as less than 10 mM, for example less than 5 mM, such as less than 1 mM, for example less than 0.1 mM, such as less than 0.01 mM, for example less than 1 μM, such as less than 100 nM, for example less than 10 nM, such as less than 1 nM, , for example less than 100 pM , such as less than 10 pM, for example less than 1 pM. Furthermore, it is herein preferred that a molecule that "does not have an affinity" to molecule X has a dissociation constant, Kd with respect to binding molecule X that is at least 10 fold larger, such as at least 20 fold larger, for example at least 30 fold larger, such as at least 40 fold larger, for example at least 50 fold larger, such as at least 60 fold larger, for example at least 70 fold larger, such as at least 80 fold larger, for example at least 90 fold larger, such as at least 100 fold larger, than the Kd of the binding (to molecule X) of a molecule that does have affinity to molecule X. Most preferably, there is at least a ten-fold difference in Kd between those molecules considered to have an affinity and those deemed not to have an affinity to a molecule X.

A framing sequence with high affinity can be provided by incorporation of one or more nucleobases forming three hydrogen bonds to a cognate nucleobase. Examples of nucleobases having this property are guanine and cytosine. Alternatively, or in addition, the framing sequence may be subjected to backbone modification. Several back bone modifications provides for higher affinity, such as 2'-O-methyl substitution of the ribose moiety, peptide nucleic acids (PNA), and 2'-4' O-methylene cyclisation of the ribose moiety, also referred to as LNA (Locked Nucleic Acid).

The identifier oligonucleotide may comprise one or two flanking regions. The flanking region can encompass a signal group, such as a flourophor or a radioactive group to allow for detection of the presence or absence of a complex or the flanking region may comprise a label that may be detected, such as biotin. When the identifier oligonucleotide comprises a biotin moiety, the identifier oligonucleotide may easily be recovered.

The flanking region(s) can also serve as priming sites for amplification reactions, such as PCR. The identifier oligonucleotide may in certain embodiments comprise an affinity region having the property of being able to hybridise to a building block. The priming sites for PCR amplification may be identical for all the identifier oligonucleotides to allow for a proportional amplification of the individual identifier oligonucleotides at various stages of the present method. Alternatively, the different priming sites with corresponding primers may be used to favour the amplification of certain groups of complexes in the library.

It is to be understood that when the term identifier oligonucleotide is used in the present description and claims, the identifier oligonucleotide may be in the sense or the anti-sense format, i.e. the identifier oligonucleotide can be a sequence of codons, which actually codes for the molecule or can be a sequence complementary thereto. Moreover,

the identifier oligonucleotide may be single-stranded or double-stranded, as appropriate.

During the contacting step the identifier oligonucleotide usually is in double stranded form to minimise any interaction relative to the target. However, it may be suitable to establish the identifier oligonucleotide in single stranded form prior to the formation of the mixture of the hetero- and homo-duplexes. Starting with a double stranded identifier oligonucleotide, a single stranded identifier oligonucleotide may easily be prepared by extension of a forward primer annealed at a priming site of the identifier oligonucleotide.

The display molecule part of the complex is generally of a chemical structure expected of having an effect on the target. When the target is of pharmaceutical importance, the molecule is generally a possible drug candidate. The complex may be formed by tagging a library of different possible drug candidates with a tag, e.g. a nucleic acid tag identifying each possible drug candidate. In another embodiment of the invention, the molecule is encoded, i.e. formed by a variety of reactants, which have reacted with each other and/or a scaffold molecule. Optionally, this reaction product may be post-modified to obtain the final molecule displayed on the complex. The post-modification may involve the cleavage of one or more chemical bonds attaching the encoded molecule to the identifier in order more efficiently to display the encoded molecule. In still another embodiment the display molecule is a polypeptide formed using the natural machinery, such as the methods disclosed in WO 92/02536, WO 91/05058, and US 6,194,550.

A variety or methods for association of an oligonucleotide to a polypeptide display molecule is available for the skilled person in the art. An option involves the association of a display molecule protein with the mRNA responsible for the formation thereof. This method is generally referred to as mRNA display. Optionally, the mRNA may be substituted with the corresponding cDNA. A method for generation such a single or a library of fusions between a protein and the mRNA responsible for the formation thereof is disclosed in WO 98/31700. The corresponding DNA strand may be attached to the protein using the method disclosed in WO 00/32823. The method of WO 98/31700 includes providing a RNA stand comprising a translation initiation sequence, a start codon operable linked to a protein encoding sequence, and a peptide acceptor at the 3’ end and translating the protein encoding sequence to produce a RNA-protein fusion. According to WO 00/32823 a DNA primer is covalently connected to the 3’ end of the mRNA strand and extended by reverse transcriptase a to prepare the complementing DNA strand. The original RNA strand may be digested by RNase H. Another suitable method for generating a library is disclosed in WO 01/90414.

In accordance with another option, the identifier oligonucleotide is associated with a polypeptide display molecule using a method generally referred to as ribosome display. Ribosome display is disclosed in WO 93/03172. A still further option for association is phage display, in which a polypeptide display molecule is presented on the capsule of the phage and the same capsule harbours the RNA or DNA responsible for the formation of the polypeptide.

A further option for associating the display molecule with an identifier oligonucleotide includes the method disclosed in M. Yonezawa et al, Nucleic acid research, 2003, vol. 31, No. 19 e118. The method includes the initial provision of an oligonucleotide connected to biotin and compartmentalization thereof together with a transcription and translation system. The oligonucleotide comprises a fusion gene coding for streptavidin and a polypeptide display molecule. After the formation of the fusion protein in each compartment, the streptavidin part of the fusion protein binds to the biotin moiety of the oligonucleotide, thereby associating the display molecule with the oligonucleotide coding for the identity thereof.

In case the display molecule is a nucleic acid, it may be of the aptamer type, i.e. a library of aptamers comprising constant nucleic acid regions flanking a random oligonucleotide part. The random oligonucleotide part serves the dual function of a nucleic acid display molecule and the identifying oligonucleotide.

The formation of a synthetic encoded molecule generally starts by a scaffold, i.e. a chemical unit having one or more reactive groups capable of forming a connection to another reactive group positioned on a chemical entity, thereby generating an addition to the original scaffold. A second chemical entity may react with a reactive group also appearing on the original scaffold or a reactive group incorporated by the first chemical entity. Further chemical entities may be involved in the formation of the final reaction product. The formation of a connection between the chemical entity and the nascent encoded molecule may be mediated by a bridging molecule. As an example, if the nascent encoded molecule and the chemical entity both comprise an amine group a connection between these can be mediated by a dicarboxylic acid. A display molecule is in general produced in vitro and may be a naturally occurring or an artificial substance. In an aspect of the invention, a display molecule is not produced using the natural translation system in an *in vitro* process. In other aspects of the invention, the display molecule is a polypeptide produced using the natural translation machinery.

The chemical entities that are precursors for structural additions or eliminations of the encoded molecule may be attached to a building block prior to the participation in the formation of the reaction product leading to the final display molecule. Besides the chemical entity, the building block generally comprises an anti-codon. In some embodiments the building blocks also comprise an affinity region providing for affinity towards the nascent complex.

In a certain aspect of the invention, the reactants or chemical entities are suitably mediated to the nascent encoded molecule by a building block, which further comprises an anticodon. The anti-codon serves the function of transferring the genetic information of the building block in conjunction with the transfer of a chemical entity. The transfer

of genetic information and chemical entity may occur in any order, however, it is important that a correspondence is maintained in the complex. The chemical entities are preferably reacted without enzymatic interaction in some aspects of the invention. Notably, ribosomes or enzymes having similar activity do preferably not mediate the reaction of the chemical entities. In another aspect of the invention a ribosome is used to translate an mRNA into a protein using a tRNA loaded with a natural or unnatural amino acid. In still another aspect of the invention, enzymes having catalytic activities different from that of ribosomes are used in the formation of the display molecule.

According to certain aspects of the invention the genetic information of the anti-codon is transferred by specific hybridisation to a codon on a nucleic acid template. Another method for transferring the genetic information of the anti-codon to the nascent complex is to anneal an oligonucleotide complementary to the anti-codon and attach this oligonucleotide to the complex, e.g. by ligation. A still further method involves transferring the genetic information of the anti-codon to the nascent complex by an extension reaction using a polymerase and a mixture of dNTPs.

The chemical entity of the building block may in certain cases be regarded as a precursor for the structural entity eventually incorporated into the encoded molecule. In other cases the chemical entity provides for the eliminations of chemical units of the nascent encoded molecule. Therefore, when it in the present application with claims is stated that a chemical entity is reacted with a nascent encoded molecule it is to be understood that not necessarily all the atoms of the original chemical entity is to be found in the eventually formed encoded molecule. Also, as a consequence of the reactions involved in the connection, the structure of the chemical entity can be changed when it appears on the nascent encoded molecule. Especially, the cleavage resulting in the release of the entity may generate a reactive group, which in a subsequent step can participate in the formation of a connection between a nascent complex and a chemical entity.

The chemical entity of the building block comprises at least one reactive group capable of participating in a reaction, which results in a connection between the chemical entity of the building block and another chemical entity or a scaffold associated with the nascent complex. The number of reactive groups, which appears on the chemical entity, is suitably one to ten. A building block featuring only one reactive group is used *i.a*. in the end positions of polymers or scaffolds, whereas building blocks having two reactive groups are suitable for the formation of the body part of a polymer or scaffolds capable of being reacted further. One, two or more reactive groups intended for the formation of connections are typically present on scaffolds. Non-limiting examples of scaffolds are opiates, steroids, benzodiazepines, hydantoines, and peptidylphosphonates.

The reactive group of the chemical entity may be capable of forming a direct connection to a reactive group of the nascent complex or the reactive group of the building block may be capable of forming a connection to a reactive group of the nascent complex through a bridging fill-in group. It is to be understood that not all the atoms of a reactive group are necessarily maintained in the connection formed. Rather, the reactive groups are to be regarded as precursors for the structure of the connection.

The subsequent cleavage step to release the chemical entity from the building block can be performed in any appropriate way. In an aspect of the invention the cleavage involves usage of a chemical reagent or an enzyme. The cleavage results in a transfer of the chemical entity to the nascent encoded molecule or in a transfer of the nascent encoded molecule to the chemical entity of the building block. In some cases it may be advantageous to introduce new chemical groups as a consequence of linker cleavage. The new chemical groups may be used for further reaction in a subsequent cycle, either directly or after having been activated. In other cases it is desirable that no trace of the linker remains after the cleavage.

In another aspect, the formation of connection between chemical entity and nascent encoded molecule and the cleavage between chemical entity and the remainder of the building block is conducted as a simultaneous reaction, i.e. either the chemical entity of the building block or the nascent encoded molecule is a leaving group of the reaction. In some aspects of the invention, it is appropriate to design the system such that the connection and the cleavage occur simultaneously because this will reduce the number of steps and the complexity. The simultaneous connection and cleavage can also be designed such that either no trace of the linker remains or such that a new chemical group for further reaction is introduced, as described above.

The attachment of the chemical entity to the building block, optionally via a suitable spacer can be at any entity available for attachment, e.g. the chemical entity can be attached to a nucleobase or the backbone. In general, it is preferred to attach the chemical entity at the phosphor of the internucleoside linkage or at the nucleobase. When the nucleobase is used for attachment of the chemical entity, the attachment point is usually at the 7 position of the purines or 7-deaza-purins or at the 5 position of pyrimidines. The nucleotide may be distanced from the reactive group of the chemical entity by a spacer moiety. The spacer may be designed such that the conformational spaced sampled by the reactive group is optimized for a reaction with the reactive group of the nascent encoded molecule.

The display molecules of the invention may have any chemical structure. In a preferred aspect, the display molecule can be any compound that may be synthesized in a component-by-component fashion. In some aspects the display molecule is a linear or branched polymer. In another aspect the display molecule is a scaffolded molecule. The term "display molecule" also comprises naturally occurring molecules like $\alpha$-polypeptides etc, however produced *in vitro*

usually in the absence of enzymes, like ribosomes. In certain aspects, the display molecule of the library is a non-α-polypeptide.

The display molecule may have any molecular weight. However, in order to be orally available, it is in this case preferred that the display molecule has a molecular weight less than 2000 Daltons, preferably less than 1000 Dalton, and more preferred less than 500 Daltons.

The size of the library may vary considerably pending on the expected result of the inventive method. In some aspects, it may be sufficient that the library comprises two, three, or four different complexes. However, in most events, more than two different complexes are desired to obtain a higher diversity. In some aspects, the library comprises 1,000 or more different complexes; more preferred 1,000,000 or more different complexes. The upper limit for the size of the library is only restricted by the size of the vessel in which the library is comprised. It may be calculated that a vial may comprise up to $10^{14}$ different complexes.

Methods for forming libraries of complexes

The complexes comprising an identifier oligonucleotide having two or more codons that codes for reactants that have reacted in the formation of the molecule part of the complex may be formed by a variety of processes. Generally, the preferred methods can be used for the formation of virtually any kind of encode molecule. Suitable examples of processes include prior art methods disclosed in WO 93/20242, WO 93/06121, WO 00/23458, WO 02/074929, and WO 02/103008, as well as methods of the present applicant not yet public available, including the methods disclosed in PCT/DK03/00739 filed 30 October 2003, and DK PA 2003 00430 filed 20 March 2003.

Below five presently preferred embodiments are described. A first embodiment disclosed in more detail in WO 02/103008 is based on the use of a polymerase to incorporate unnatural nucleotides as building blocks. Initially, a plurality of template oligonucleotides is provided. Subsequently primers are annealed to each of the templates and a polymerase is extending the primer using nucleotide derivatives, which have appended chemical entities. Subsequent to or simultaneously with the incorporation of the nucleotide derivatives, the chemical entities are reacted to form a reaction product. The encoded molecule may be post-modified by cleaving some of the linking moieties to better present the encoded molecule.

Several possible reaction approaches for the chemical entities are apparent. First, the nucleotide derivatives can be incorporated and the chemical entities subsequently polymerised. In the event the chemical entities each carry two reactive groups, the chemical entities can be attached to adjacent chemical entities by a reaction of these reactive groups. Exemplary of the reactive groups are amine and carboxylic acid, which upon reaction form an amide bond. Adjacent chemical entities can also be linked together using a linking or bridging moiety. Exemplary of this approach is the linking of two chemical entities each bearing an amine group by a bi-carboxylic acid. Yet another approach is the use of a reactive group between a chemical entity and the nucleotide building block, such as an ester or a hoister group. An adjacent building block having a reactive group such as an amine may cleave the interspaced reactive group to obtain a linkage to the chemical entity, e.g. by an amide linking group.

A second embodiment for obtainment of complexes disclosed in WO 02/103008 pertains to the use of hybridisation of building blocks to a template and reaction of chemical entities attached to the building blocks in order to obtain a reaction product. This approach comprises that templates are contacted with a plurality of building blocks, wherein each building block comprises an anti-codon and a chemical entity. The anti-codons are designed such that they recognise a sequence, i.e. a codon, on the template. Subsequent to the annealing of the anti-codon and the codon to each other a reaction of the chemical entity is effected.

The template may be associated with a scaffold. Building blocks bringing chemical entities in may be added sequentially or simultaneously and a reaction of the reactive group of the chemical entity may be effected at any time after the annealing of the building blocks to the template.

A third embodiment for the generation of a complex includes chemical or enzymatic ligation of building blocks when these are lined up on a template. Initially, templates are provided, each having one or more codons. The templates are contacted with building blocks comprising anti-codons linked to chemical entities. The two or more anti-codons annealed on a template are subsequently ligated to each other and a reaction of the chemical entities is effected to obtain a reaction product. The method is disclosed in more detail in DK PA 2003 00430 filed 20 March 2003.

A fourth embodiment makes use of the extension by a polymerase of an affinity sequence of the nascent complex to transfer the anti-codon of a building block to the nascent complex. The method implies that a nascent complex comprising a scaffold and an affinity region is annealed to a building block comprising a region complementary to the affinity section. Subsequently, the anti-codon region of the building block is transferred to the nascent complex by a polymerase. The transfer of the chemical entity may be transferred prior to, simultaneously with or subsequent to the transfer of the anti-codon. This method is disclosed in detail in PCT/DK03/00739.

A fifths embodiment also disclosed in PCT/DK03/00739 comprises reaction of a reactant with a site reaction site on nascent bifunctional molecule and addition of a nucleic acid tag to the nascent bifunctional molecule using an

enzyme, such as a ligase. When a library is formed, usually an array of compartments is used for reaction of reactants and enzymatic addition of tags with the nascent bifunctional molecule.

Thus, the codons are either pre-made into one or more templates before the encoded molecules are generated or the codons are transferred simultaneously with the formation of the encoded molecules.

After or simultaneously with the formation of the reaction product some of the linkers to the template may be cleaved, however, usually at least one linker is maintained to provide for the complex.

Nucleotides

The nucleotides used in the present invention may be linked together in a sequence of nucleotides, i.e. an oligonucleotide. Each nucleotide monomer is normally composed of two parts, namely a nucleobase moiety, and a backbone. The backbone may in some cases be subdivided into a sugar moiety and an internucleoside linker.

The nucleobase moiety may be selected among naturally occurring nucleobases as well as non-naturally occurring nucleobases. Thus, "nucleobase" includes not only the known purine and pyrimidine heterocycles, but also heterocyclic analogues and tautomers thereof. Illustrative examples of nucleobases are adenine, guanine, thymine, cytosine, uracil, purine, xanthine, diaminopurine, 8-oxo-$N^6$-methyladenine, 7-deazaxanthine, 7-deazaguanine, $N^4$,$N^4$-ethanocytosin, $N^6$,$N^6$-ethano-2,6-diamino-purine, 5-methylcytosine, 5-($C^3$-$C^6$)-alkynylcytosine, 5-fluorouracil, 5-bromouracil, pseudoisocytosine, 2-hydroxy-5-methyl-4-triazolopyridine, isocytosine, isoguanine, inosine and the "non-naturally occurring" nucleobases described in Benner et al., U.S. Pat No. 5,432,272. The term "nucleobase" is intended to cover these examples as well as analogues and tautomers thereof. Especially interesting nucleobases are adenine, guanine, thymine, cytosine, 5-methylcytosine, and uracil, which are considered as the naturally occurring nucleobases in relation to therapeutic and diagnostic application in humans.

Examples of suitable specific pairs of nucleobases are shown below:

**Natural Base Pairs**

R=H: Uracil
R=$CH_3$: Thymine

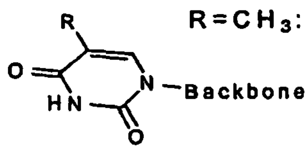

Cytosine

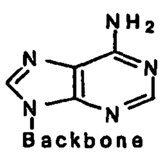

Adenine

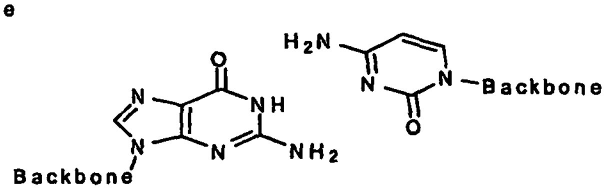

Guanine

**Synthetic Base Pairs**

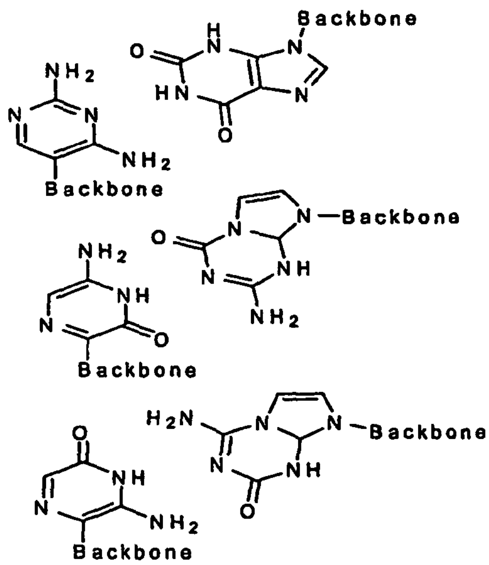

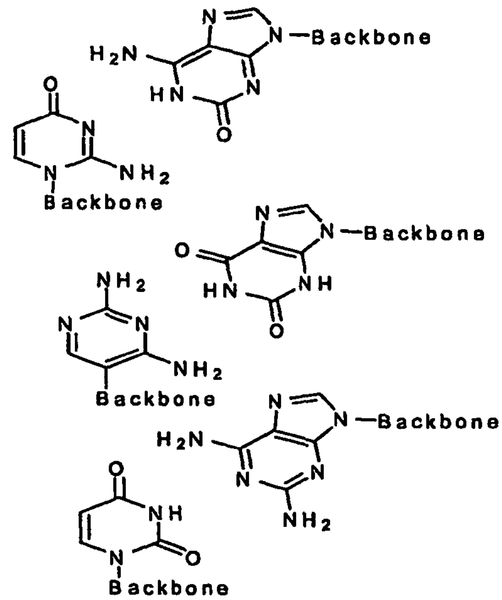

**Synthetic purine bases pairring with natural pyrimidines**

R=H: Uracil
$R=CH_3$: Thymine

Cytosine

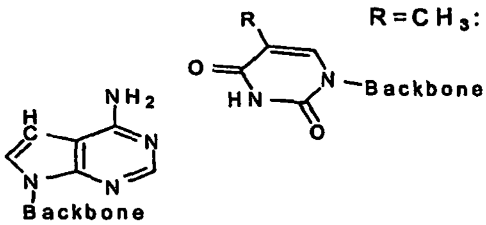

7-deaza adenine

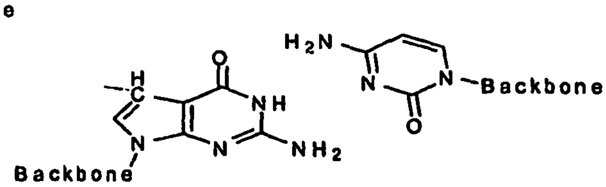

7-deaza guanine

Suitable examples of backbone units are shown below (B denotes a nucleobase):

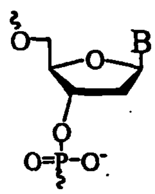

DNA

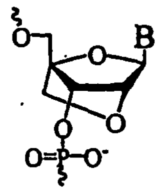

Oxy-LNA

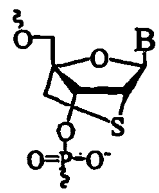

Thio-LNA

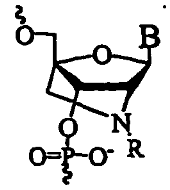

Amino-LNA
R = -H, -$CH_3$

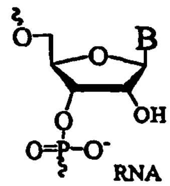

RNA

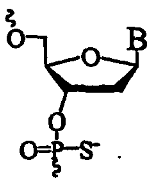

Phosphorthioate

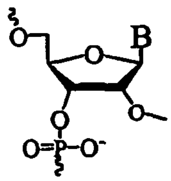

2'-O-Methyl

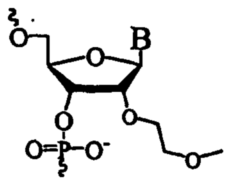

2'-MOE

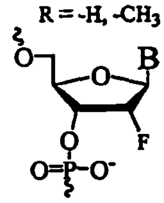

2'-Fluoro

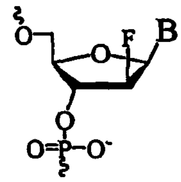

2'-F-ANA

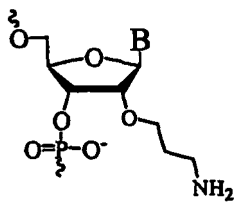

2'-AP

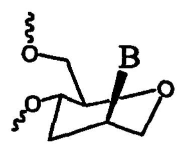

HNA

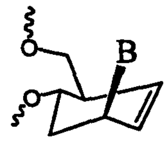

CeNA

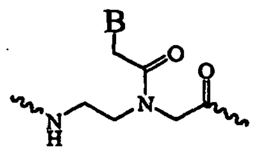

PNA

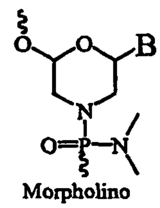

Morpholino

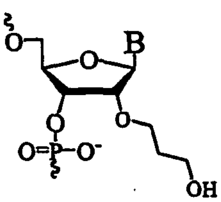

2'-(3-hydroxy)propyl

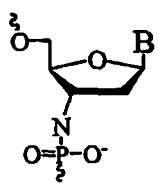

3'-Phosphoramidate

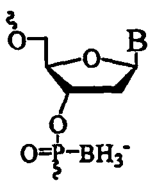

Boranophosphates

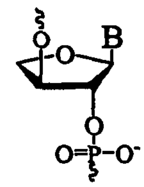

TNA

The sugar moiety of the backbone is suitably a pentose but may be the appropriate part of a PNA or a six-member ring. Suitable examples of possible pentoses include ribose, 2'-deoxyribose, 2'-O-methyl-ribose, 2'-flour ribose, and 2'-4'-O-methylene-ribose (LNA). Suitably the nucleobase is attached to the 1' position of the pentose entity.

An internucleoside linker connects the 3' end of preceding monomer to a 5' end of a succeeding monomer when the sugar moiety of the backbone is a pentose, like ribose or 2-deoxyribose. The internucleoside linkage may be the natural occurring phospodiester linkage or a derivative thereof. Examples of such derivatives include phosphorothioate, methylphosphonate, phosphoramidate, phosphotriester, and phosphodithioate. Furthermore, the internucleoside linker can be any of a number of non-phosphorous-containing linkers known in the art.

Preferred nucleic acid monomers include naturally occurring nucleosides forming part of the DNA as well as the RNA family connected through phosphodiester linkages. The members of the DNA family include deoxyadenosine, deoxyguanosine, deoxythymidine, and deoxycytidine. The members of the RNA family include adenosine, guanosine, uridine, cytidine, and inosine. Inosine is a non-specific pairing nucleoside and may be used as universal base because inosine can pair nearly isoenergetically with A, T, and C. Other compounds having the same ability of non-specifically base-pairing with natural nucleobases have been formed. Suitable compounds which may be utilized in the present invention includes among others the compounds depicted below

**Examples of Universal Bases:**

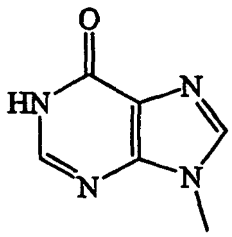
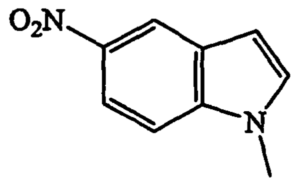
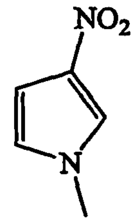
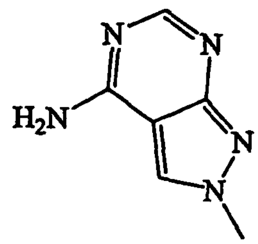

Inosine 5-Nitroindole 3-Nitropyrrole $N^8$-8aza-7deazaadenine

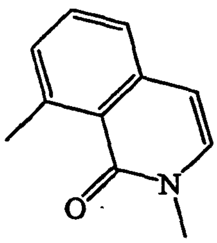
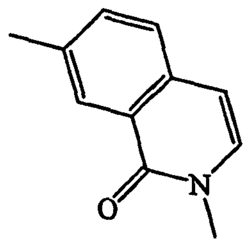
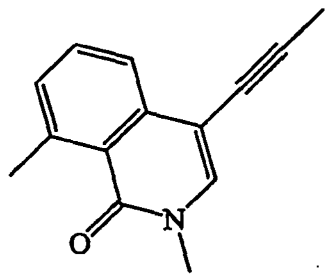

MICS 5MICS PIM

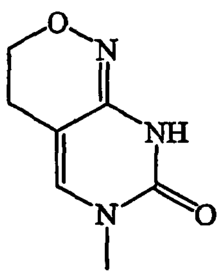
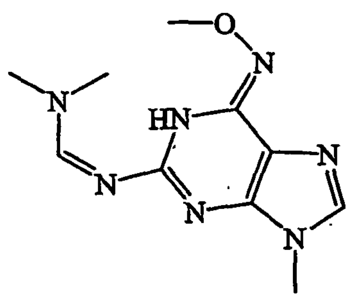
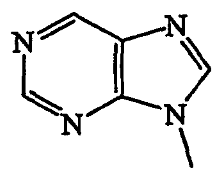

dP dK Nebularine

Building block

The chemical entities or reactants that are precursors for structural additions or eliminations of the encoded molecule may be attached to a building block prior to the participation in the formation of the reaction product leading to the final encoded molecule. Besides the chemical entity, the building block generally comprises an anti-codon.

The chemical entity of the building block comprises at least one reactive group capable of participating in a reaction, which results in a connection between the chemical entity of the building block and another chemical entity or a scaffold associated with the nascent complex. The connection is facilitated by one or more reactive groups of the chemical entity. The number of reactive groups, which appear on the chemical entity, is suitably one to ten. A building block featuring only one reactive group is used i.a. in the end positions of polymers or scaffolds, whereas building blocks having two reactive groups are suitable for the formation of the body part of a polymer or scaffolds capable of being

reacted further. One, two or more reactive groups intended for the formation of connections are typically present on scaffolds.

The reactive group of the building block may be capable of forming a direct connection to a reactive group of the nascent complex or the reactive group of the building block may be capable of forming a connection to a reactive group of the nascent complex through a bridging fill-in group. It is to be understood that not all the atoms of a reactive group are necessarily maintained in the connection formed. Rather, the reactive groups are to be regarded as precursors for the structure of the connection.

The subsequent cleavage step to release the chemical entity from the building block can be performed in any appropriate way. In an aspect of the invention the cleavage involves usage of a reagent or an enzyme. The cleavage results in a transfer of the chemical entity to the nascent encoded molecule or in a transfer of the nascent encoded molecule to the chemical entity of the building block. In some cases it may be advantageous to introduce new chemical groups as a consequence of linker cleavage. The new chemical groups may be used for further reaction in a subsequent cycle, either directly or after having been activated. In other cases it is desirable that no trace of the linker remains after the cleavage.

In another aspect, the connection and the cleavage are conducted as a simultaneous reaction, i.e. either the chemical entity of the building block or the nascent encoded molecule is a leaving group of the reaction. In general, it is preferred to design the system such that the connection and the cleavage occur simultaneously because this will reduce the number of steps and the complexity. The simultaneous connection and cleavage can also be designed such that either no trace of the linker remains or such that a new chemical group for further reaction is introduced, as described above.

The attachment of the chemical entity to the building block, optionally via a suitable spacer can be at any entity available for attachment, e.g. the chemical entity can be attached to a nucleobase or the backbone. In general, it is preferred to attach the chemical entity at the phosphor of the internucleoside linkage or at the nucleobase. When the nucleobase is used for attachment of the chemical entity, the attachment point is usually at the 7 position of the purines or 7-deaza-purins or at the 5 position of pyrimidines. The nucleotide may be distanced from the reactive group of the chemical entity by a spacer moiety. The spacer may be designed such that the conformational space sampled by the reactive group is optimized for a reaction with the reactive group of the nascent encoded molecule or reactive site.

The anticodon complements the codon of the identifier oligonucleotide sequence and generally comprises the same number of nucleotides as the codon. The anticodon may be adjoined with a fixed sequence, such as a sequence complementing a framing sequence.

Various specific building blocks are envisaged. Building blocks of particular interest are shown below.

*Building blocks transferring a chemical entity to a recipient nucleophilic group* The building block indicated below is capable of transferring a chemical entity (CE) to a recipient nucleophilic group, typically an amine group. The bold lower horizontal line illustrates the building block comprising an anti-codon and the vertical line illustrates a spacer. The 5-membered substituted N-hydroxysuccinimid (NHS) ring serves as an activator, i.e. a labile bond is formed between the oxygen atom connected to the NHS ring and the chemical entity. The labile bond may be cleaved by a nucleophilic group, e.g. positioned on a scaffold

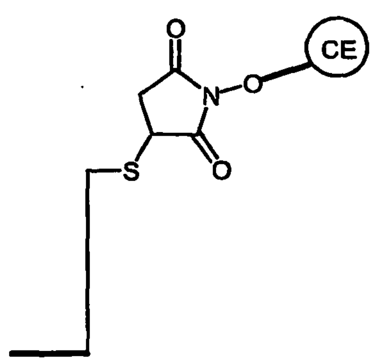

The 5-membered substituted N-hydroxysuccinimid (NHS) ring serves as an activator, i.e. a labile bond is formed between the oxygen atom connected to the NHS ring and the chemical entity. The labile bond may be cleaved by a nucleophilic group, e.g. positioned on a scaffold, to transfer the chemical entity to the scaffold, thus converting the remainder of the fragment into a leaving group of the reaction. When the chemical entity is connected to the activator through a carbonyl group and the recipient group is an amine, the bond formed on the scaffold will an amide bond. The above building block is the subject of WO03078627A2.

Another building block, which may form an amide bond, is

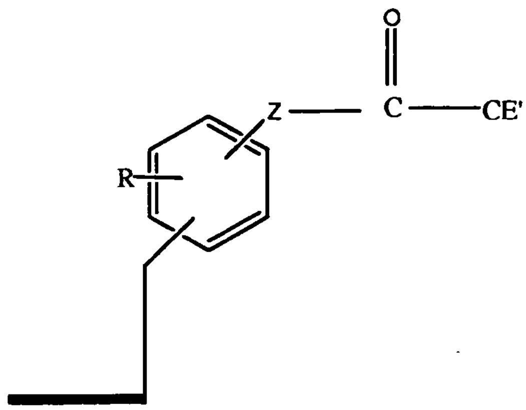

R may be absent or $NO_2$, $CF_3$, halogen, preferably Cl, Br, or I, and Z may be S or O. This type of building block is disclosed in WO03078626A2. A nucleophilic group can cleave the linkage between Z and the carbonyl group thereby transferring the chemical entity -(C=O)-CE' to said nucleophilic group.

*Building blocks transferring a chemical entity to a recipient reactive group forming a C=C bond*

A building block as shown below is able to transfer the chemical entity to a recipient aldehylde group thereby forming a double bond between the carbon of the aldehyde and the chemical entity

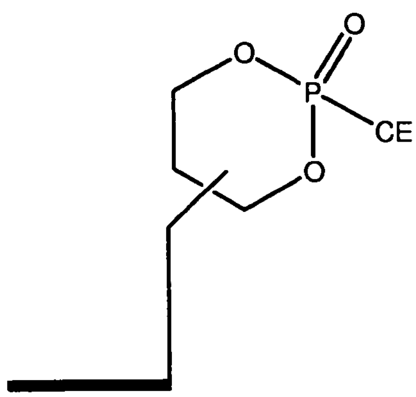

The above building block is disclosed in WO03078445A2.

*Building blocks transferring a chemical entity to a recipient reactive group forming a C-C bond*

The below building block is able to transfer the chemical entity to a recipient group thereby forming a single bond between the receiving moiety, e.g. a scaffold, and the chemical entity.

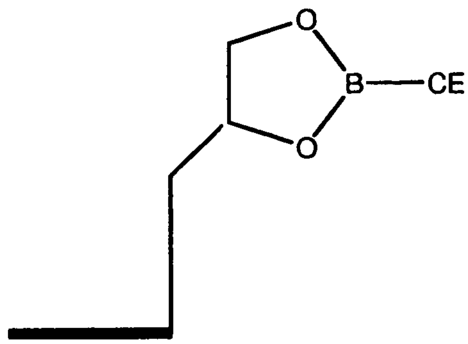

The above building block is disclosed in WO03078445A2.

Another building block capable of transferring a chemical entity to a receiving reactive group forming a single bond is

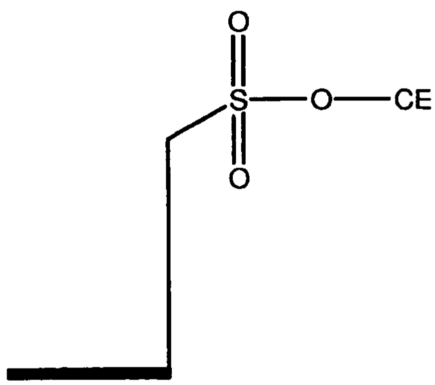

The receiving group may be a nucleophile, such as a group comprising a hetero atom, thereby forming a single bond between the chemical entity and the hetero atom, or the receiving group may be an electronegative carbon atom, thereby forming a C-C bond between the chemical entity and the scaffold. The above building block is disclosed in WO03078446A2.

The chemical entity attached to any of the above building blocks may be a selected from a large arsenal of chemical structures. Examples of chemical entities are

H or entities selected among the group consisting of a $C_1$-$C_6$ alkyl, $C_2$-$C_6$ alkenyl, $C_2$-$C_6$ alkynyl, $C_4$-$C_8$ alkadienyl, $C_3$-$C_7$ cycloalkyl, $C_3$-$C_7$ cycloheteroalkyl, aryl, and heteroaryl, said group being substituted with 0-3 $R^4$, 0-3 $R^5$ and 0-3 $R^9$ or $C_1$-$C_3$ alkylene-$NR^4_2$, $C_1$-$C_3$ alkylene-$NR^4C(O)R^8$, $C_1$-$C_3$alkylene-$NR^4C(O)OR^8$, $C_1$-$C_2$ alkylene-O-$NR^4_2$, $C_1$-$C_2$ alkylene-O-$NR^4C(O)R^8$, $C_1$-$C_2$ alkylene-O-$NR^4C(O)OR^8$ substituted with 0-3 $R^9$.

where $R^4$ is.H or selected independently among the group consisting of $C_1$-$C_6$ alkyl, $C_2$-$C_6$ alkenyl, $C_2$-$C_6$ alkynyl, $C_3$-$C_7$ cycloalkyl, $C_3$-$C_7$ cycloheteroalkyl, aryl, heteroaryl, said group being substituted with 0-3 $R^9$ and

$R^5$ is selected independently from -$N_3$, -CNO, -C(NOH)$NH_2$, -NHOH, -NHNH$R^6$, -C(O)$R^6$, -Sn$R^6_3$, -B($OR^6$)$_2$, -P(O)($OR^6$)$_2$ or the group consisting of $C_2$-$C_6$ alkenyl, $C_2$-$C_6$ alkynyl, $C_4$-$C_8$ alkadienyl said group being substituted with 0-2 $R^7$, where $R^6$ is selected independently from H, $C_1$-$C_6$ alkyl, $C_3$-$C_7$ cycloalkyl, aryl or $C_1$-$C_6$ alkylene-aryl substituted with 0-5 halogen atoms selected from -F, -Cl, -Br, and -I; and

$R^7$ is independently selected from -$NO_2$, -COO$R^6$, -CO$R^6$, -CN, -OSi$R^6_3$, -O$R^6$ and -N$R^6_2$.

$R^8$ is H, $C_1$-$C_6$ alkyl, $C_2$-$C_6$ alkenyl, $C_2$-$C_6$ alkynyl, $C_3$-$C_7$ cycloalkyl, aryl or $C_1$-$C_6$ alkylene-aryl substituted with 0-3 substituents independently selected from -F, -Cl, -$NO_2$, -$R^3$, -O$R^3$, -Si$R^3_3$

$R^9$ is =O, -F, -Cl, -Br, -I, -CN, -$NO_2$, -O$R^6$, -N$R^6_2$, -N$R^6$-C(O)$R^8$, -N$R^6$-C(O)O$R^8$, -S$R^6$, -S(O)$R^6$, -S(O)$_2R^6$, -COO$R^6$, -C(O)N$R^6_2$ and -S(O)$_2$N$R^6_2$.

*Cross-link cleavage building blocks*

It may be advantageous to split the transfer of a chemical entity to a recipient reactive group into two separate steps, namely a cross-linking step and a cleavage step because each step can be optimized. A suitable building block

for this two-step process is illustrated below:

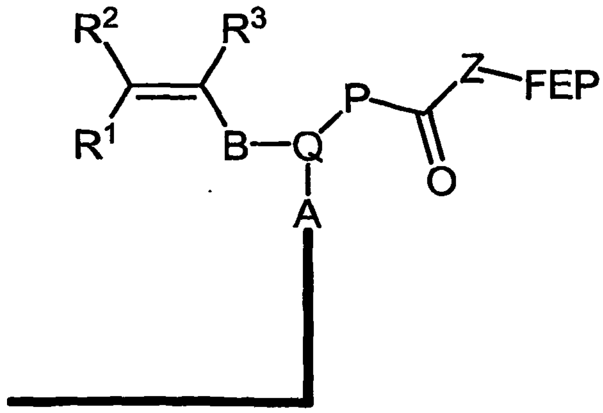

Initially, a reactive group appearing on the functional entity precursor (abbreviated FEP) reacts with a recipient reactive group, e.g. a reactive group appearing on a scaffold, thereby forming a cross-link. Subsequently, a cleavage is performed, usually by adding an aqueous oxidising agent such as $I_2$, $Br_2$, $Cl_2$, $H^+$, or a Lewis acid. The cleavage results in a transfer of the group HZ-FEP- to the recipient moiety, such as a scaffold.

In the above formula

Z is O, S, $NR^4$

Q is N, $CR^1$

P is a valence bond, O, S, $NR^4$, or a group $C_{5-7}$arylene, $C_{1-6}$alkylene, $C_{1-6}$O-alkylene, $C_{1-6}$S-alkylene, $NR^1$-alkylene, $C_{1-6}$alkylene-O, $C_{1-6}$alkylene-S option said group being substituted with 0-3 $R^4$, 0-3 $R^5$ and 0-3 $R^9$ or $C_1$-$C_3$ alkylene-$NR^4_2$, $C_1$-$C_3$ alkylene-$NR^4C(O)R^8$, $C_1$-$C_3$ alkylene-$NR^4C(O)OR^8$, $C_1$-$C_2$ alkylene-O-$NR^4_2$, $C_1$-$C_2$ alkylene-O-$NR^4C(O)R^8$, $C_1$-$C_2$ alkylene-O-$NR^4C(O)OR^8$ substituted with 0-3 $R^9$,

B is a group comprising D-E-F, in which

D is a valence bond or a group $C_{1-6}$alkylene, $C_{1-6}$alkenylene, $C_{1-6}$alkynylene, $C_{5-7}$arylene, or $C_{5-7}$heteroarylene, said group optionally being substituted with 1 to 4 group $R^{11}$,

E is, when present, a valence bond, O, S, $NR^4$, or a group $C_{1-6}$alkylene, $C_{1-6}$alkenylene, $C_{1-6}$alkynylene, $C_{5-7}$arylene, or $C_{5-7}$heteroarylene, said group optionally being substituted with 1 to 4 group $R^{11}$,

F is, when present, a valence bond, O, S, or $NR^4$,

A is a spacing group distancing the chemical structure from the complementing element, which may be a nucleic acid, $R^1$, $R^2$, and $R^3$ are independent of each other selected among the group consisting of H, $C_1$-$C_6$ alkyl, $C_2$-$C_6$ alkenyl, $C_2$-$C_6$ alkynyl, $C_4$-$C_8$ alkadienyl, $C_3$-$C_7$ cycloalkyl, $C_3$-$C_7$ cycloheteroalkyl, aryl, and heteroaryl, said group being substituted with 0-3 $R^4$, 0-3 $R^5$ and 0-3 $R^9$ or $C_1$-$C_3$ alkylene-$NR^4_2$, $C_1$-$C_3$ alkylene-$NR^4C(O)R^8$, $C_1$-$C_3$ alkylene-$NR^4C(O)OR^8$, $C_1$-$C_2$ alkylene-O-$NR^4_2$, $C_1$-$C_2$ alkylene-O-$NR^4C(O)R^8$, $C_1$-$C_2$ alkylene-O-$NR^4C(O)OR^8$ substituted with 0-3 $R^9$,

FEP is a group selected among the group consisting of H, $C_1$-$C_6$ alkyl, $C_2$-$C_6$ alkenyl, $C_2$-$C_6$ alkynyl, $C_4$-$C_8$ alkadienyl, $C_3$-$C_7$ cycloalkyl, $C_3$-$C_7$ cycloheteroalkyl, aryl, and heteroaryl, said group being substituted with 0-3 $R^4$, 0-3 $R^5$ and 0-3 $R^9$ or $C_1$-$C_3$ alkylene-$NR^4_2$, $C_1$-$C_3$ alkylene-$NR^4C(O)R^8$, $C_1$-$C_3$ alkylene-$NR^4C(O)OR^8$, $C_1$-$C_2$ alkylene-O-$NR^4_2$, $C_1$-$C_2$ alkylene-O-$NR^4C(O)R^8$, $C_1$-$C_2$ alkylene-O-$NR^4C(O)OR^8$ substituted with 0-3 $R^9$,

where $R^4$ is H or selected independently among the group consisting of $C_1$-$C_6$ alkyl, $C_2$-$C_6$ alkenyl, $C_2$-$C_6$ alkynyl, $C_3$-$C_7$ cycloalkyl, $C_3$-$C_7$ cycloheteroalkyl, aryl, heteroaryl, said group being substituted with 0-3 $R^9$ and

$R^5$ is selected independently from $-N_3$, -CNO, $-C(NOH)NH_2$, -NHOH, $-NHNHR^6$, $-C(O)R^6$, $-SnR^6_3$, $-B(OR^6)_2$, $-P(O)(OR^6)_2$ or the group consisting of $C_2$-$C_6$ alkenyl, $C_2$-$C_6$ alkynyl, $C_4$-$C_8$ alkadienyl said group being substituted with 0-2 $R^7$, where $R^6$ is selected independently from H, $C_1$-$C_6$ alkyl, $C_3$-$C_7$ cycloalkyl, aryl or $C_1$-$C_6$ alkylene-aryl substituted with 0-5 halogen atoms selected from -F, -Cl, -Br, and -I; and $R^7$ is independently selected from $-NO_2$, $-COOR^6$, $-COR^6$, -CN, $-OSiR^6_3$, $-OR^6$ and $-NR^6_2$.

$R^8$ is H, $C_1$-$C_6$ alkyl, $C_2$-$C_6$ alkenyl, $C_2$-$C_6$ alkynyl, $C_3$-$C_7$ cycloalkyl, aryl or $C_1$-$C_6$ alkylene-aryl substituted with 0-3 substituents independently selected from -F, -Cl, $-NO_2$, $-R^3$, $-OR^3$, $-SiR^3_3$

$R^9$ is =O, -F, -Cl, -Br, -I, -CN, $-NO_2$, $-OR^6$, $-NR^6_2$, $-NR^6-C(O)_R{}^8$, $-NR^6-C(O)OR^8$, $-SR^6$, $-S(O)R^6$, $-S(O)_2R^6$, $-COOR^6$, $-C(O)NR^6_2$ and $-S(O)_2NR^6_2$.

In a preferred embodiment Z is O or S, P is a valence bond, Q is CH, B is $CH_2$, and $R^1$, $R^2$, and $R^3$ is H. The bond between the carbonyl group and Z is cleavable with aqueous $I_2$.

Contacting between target and library

The contacting step, by which the library of bifunctional molecules is subjected under binding conditions to a target, may be referred to as the enrichment step or the selection step, as appropriate, and includes the screening of the library for display molecules having predetermined desirable characteristics. Predetermined desirable characteristics can include binding to a target, catalytically changing the target, chemically reacting with a target in a manner which alters/modifies the target or the functional activity of the target, and covalently attaching to the target as in a suicide inhibitor.

In theory, display molecules of interest can be selected based on their properties using either physical or physiological procedures. The method preferred according to the present invention is to enrich molecules with respect to binding affinity towards a target of interest. In a certain embodiment, the basic steps involve mixing the library of complexes with the target of interest. The target can be attached to a column matrix or microtitre wells with direct immobilization or by means of antibody binding or other high-affinity interactions. In another embodiment, the target and displayed molecules interact without immobilisation of the target. Displayed molecules that bind to the target will be retained on this surface, while nonbinding displayed molecules in a certain aspect of the invention will be removed during a single or a series of wash steps. The identifier oligonucleotides of complexes bound to the target can then be recovered. It may be considered advantageously to perform a chromatography step after or instead of the washing step, notably in cases where the target is not immobilized. After the recovery of the identifier oligonucleotides they are optionally amplified before the decoding step.

A significant reduction in background binders may be obtained with increased washing volumes, repeating washing steps, higher detergent concentrations and prolonged incubation during washing. Thus, the more volume and number of steps used in the washing procedure together with more stringent conditions the more efficiently the non-binders and background binders will be removed. The right stringency in the washing step can also be used to remove low-affinity specific binders. However, the washing step will also remove wanted binders if too harsh conditions are used.

A blocking step, such as incubation of solid phase with skimmed milk proteins or other inert proteins and/or mild detergent such as Tween-20 and Triton X-100, may also be used to reduce the background. The washing conditions should be as stringent as possible to remove background binding but to retain specific binders that interact with the target. Generally, washing conditions are adjusted to maintain the desired affinity binders, e.g. binders in the micro molar, nanomolar, or picomolar range.

The present invention takes advantages of the fact that the identifier oligonucleotides of low-binding complexes will be in a low concentration compared to the identifier oligonucleotides of complexes binding with high affinity. The generated imbalance can be enhanced in the subsequent formation of a mixture of homo- and hetoro-duplexes and the recovery of homo-duplexes.

The target can be any compound of interest. E.g. the target can be a protein, peptide, carbohydrate, polysaccharide, glycoprotein, hormone, receptor, antigen, antibody, virus, substrate, metabolite, transition state analogue, cofactor, inhibitor, drug, dye, nutrient, growth factor, cell, tissue, etc. without limitation. Suitable targets include, but are not limited to, angiotensin converting enzyme, renin, cyclooxygenase, 5-lipoxygenase, IIL-1 0 converting enzyme, cytokine receptors, PDGF receptor, type II inosine monophosphate dehydrogenase, β-lactamases, integrin, proteases like factor VIIa, kinases like Bcr-Abl/Her, phosphotases like PTP-1B, and fungal cytochrome P-450. Targets can include, but are not limited to, bradykinin, neutrophil elastase, the HIV proteins, including *tat, rev, gag, int,* RT, nucleocapsid etc., VEGF, bFGF, TGFβ, KGF, PDGF, GPCR, thrombin, substance P, IgE, sPLA2, red blood cells, glioblastomas, fibrin clots, PBMCs, hCG, lectins, selectins, cytokines, ICP4, complement proteins, etc.

A target can also be a surface of a non-biological origin, such as a polymer surface or a metal surface. The method of the invention may then be used to identify suitable coatings for such surfaces.

In a preferred embodiment, the desirable display molecule acts on the target without any interaction between the nucleic acid attached to the desirable encoded molecule and the target. In one embodiment, the bound complex-target aggregate can be partitioned from unbound complexes by a number of methods. The methods include nitrocellulose filter binding, column chromatography, filtration, affinity chromatography, centrifugation, and other well known methods. A preferred method is size-exclusion chromatography.

Briefly, the library of complexes is subjected to the target, which may include contact between the library and a column onto which the target is immobilised. Identifier oligonucleotides associated with undesirable display molecules, i.e. display molecules not bound to the target under the stringency conditions used, will pass through the column. Additional undesirable display molecules (e.g. display molecules which cross-react with other targets) may be removed by counter-selection methods. Desirable complexes are bound to the column. The target may be immobilized in a number of ways. In one embodiment, the target is immobilized through a cleavable physical link, such as one more chemical bonds.

The complex may be provided with a cleavable linker at a position between the display molecule and the identifier oligonucleotide. When the target is immobilized, the cleavable linker of the complex is preferable orthogonal to the cleavable linker that attached the target to the solid support. The cleavable linker may be cleaved to separate the

identifier oligonucleotides of complexes having affinity towards the targets. Just to mention a single type of orthogonal cleavable linkages, one could attached to target to the solid support through a linkage that can be cleaved by a chemical agent, and the linker separating the display molecule and the identifier oligonucleotide may be selected as a photocleavable linkage. More specifically, the former linkage may be a disulphide bond that can be cleaved by a suitable reducing agent like DTT (dithiothreitol) and the latter linkage may be an o-nitrophenyl group.

There are other partitioning and screening processes which are compatible with this invention that are known to one of ordinary skill in the art. Such known process may be used in combination with the present inventive method. In one embodiment, the complex-target aggregate can be fractionated by common methods and then each fraction is assayed for activity. The fractionization methods can include size, pH, hydrophobicity, etc.

Inherent in the present method is the selection of encoded molecules on the basis of a desired function; this can be extended to the selection of molecules with a desired function and specificity. Specificity can be required during the selection process by first extracting complexes which are capable of interacting with a non-desired "target" (negative selection, or counter-selection), followed by positive selection with the desired target. As an example, inhibitors of fungal cytochrome P-450 are known to cross-react to some extent with mammalian cytochrome P-450 (resulting in serious side effects). Highly specific inhibitors of the fungal cytochrome could be selected from a library by first removing those complexes capable of interacting with the mammalian cytochrome, followed by retention of the remaining products which are capable of interacting with the fungal cytochrome.

In a certain embodiment, a binding platform may be constructed that can be used for almost any target. The binding platform should preferably be small enough to only allow association of a few or a single target molecule. This to ensure a solution based selection procedure with adjustable target concentration. The binding platform is primarily composed of two components; a small surface allowing association of the target molecule, and an association area/site for the target oligonucleotide. This binding platform may be designed to mediate the association of the target and target oligonucleotide to allow proximity selection in solution.

Cleavable linkers

A cleavable linker may be positioned between the target and a solid support, between the display molecule and the identifier oligonucleotide, or any other position that can provide for a separation of the identifier oligonucleotides of successful complexes from non-specific binding complexes. The cleavable linker may be selectively cleavable, i.e. conditions may selected that only cleave that particular linker.

The cleavable linkers may be selected from a large plethora of chemical structures. Examples of linkers include, but are not limited to, linkers having an enzymatic cleavage site, linkers comprising a chemical degradable component, and linkers cleavable by electromagnetic radiation, such as light.

*Examples of linkers cleavable by electromagnetic radiation (light)*

o-nitrobenzyl

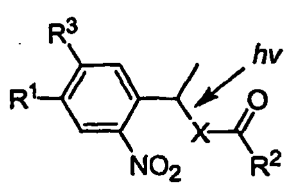

p-alkoxy

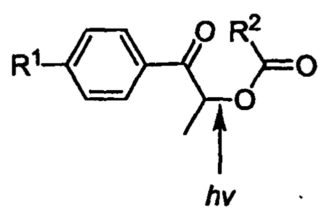

o-nitrobenzyl in exo position

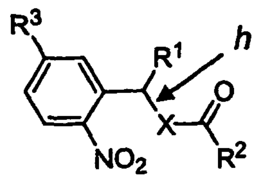

For more details see Holmes CP. J. Org. Chem. 1997, 62, 2370-2380

3-nitrophenyloxy

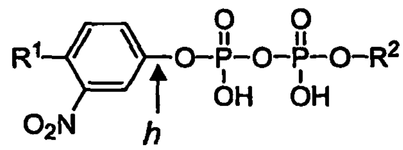

For more details see Rajasekharan Pillai, V. N. Synthesis. 1980, 1-26

Dansyl derivatives:

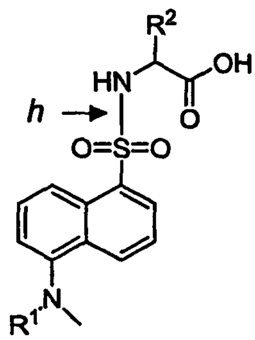

For more details see Rajasekharan Pillai, V. N. Synthesis. 1980, 1-26

Coumarin derivatives

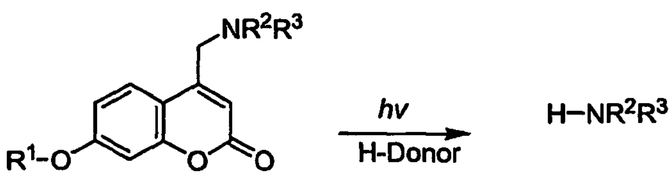


For more details see R. O. Schoenleber, B. Giese. Synlett 2003, 501-504

$R^1$ and $R^2$ can be either of the potential drug candidate and the identifier oligonucleotide, respectively. Alternatively, $R^1$ and $R^2$ can be either of the target or a solid support, respectively.
$R^3$ = H or $OCH_3$
If X is O then the product will be a carboxylic acid
If X is NH the product will be a carboxamide

One specific example is the PC Spacer Phosphoramidite (Glen research catalog # 10-4913-90) which can be introduced in an oligonucleotide during synthesis and cleaved by subjecting the sample in water to UV light (~ 300-350 nm) for 30 seconds to 1 minute.

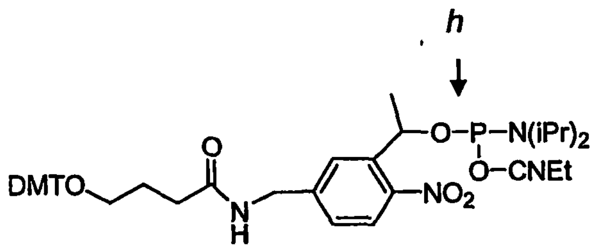


DMT = 4,4'-Dimethoxytrityl
iPr = Isopropyl
CNEt = Cyanoethyl

The above PC spacer phosphoamidite is suitable incorporated in a library of complexes at a position between the indentifier and the potential drug candidate. The spacer may be cleaved according to the following reaction.

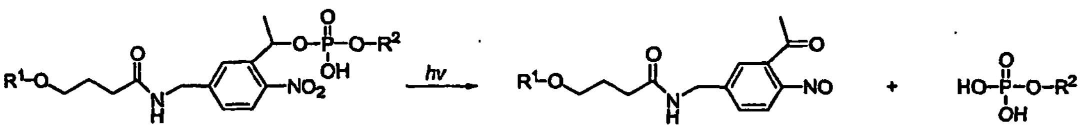


$R^1$ and $R^2$ can be either of the encoded molecule and the identifying molecule, respectively. In a preferred aspect $R^2$ is an oligonucleotide identifier and the $R^1$ is the potential drug candidate. When the linker is cleaved a phosphate group is generated allowing for further biological reactions. As an example, the phosphate group may be positioned in the 5'end of an oligonucleotide allowing for an enzymatic ligation process to take place.

*Examples of linkers cleavable by chemical agents:*

Ester linkers can be cleaved by nucleophilic attack using e.g. hydroxide ions. In practice this can be accomplished by subjecting the target-ligand complex to a base for a short period.

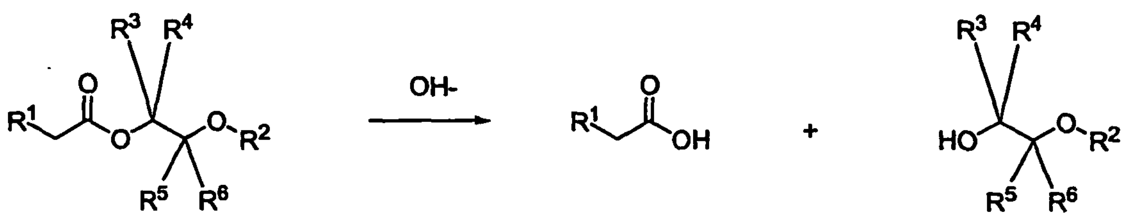


$R^1$ and $R^2$ can be the either of be the potential drug candidate or the identifier oligonucleotide, respectively. $R^{4-6}$ can be any of the following: H, CN, F, $NO_2$, $SO_2NR_2$.

Disulfide linkers can efficiently be cleaved / reduced by Tris (2-carboxyethyl) phosphine (TCEP). TCEP selectively and completely reduces even the most stable water-soluble alkyl disulfides over a wide pH range. These reductions frequently required less than 5 minutes at room temperature. TCEP is a nonvolatile and odorless reductant and unlike most other reducing agents, it is resistant to air oxidation. Trialkylphosphines such as TCEP are stable in aqueous solution, selectively reduce disulfide bonds, and are essentially unreactive toward other functional groups commonly found in proteins.

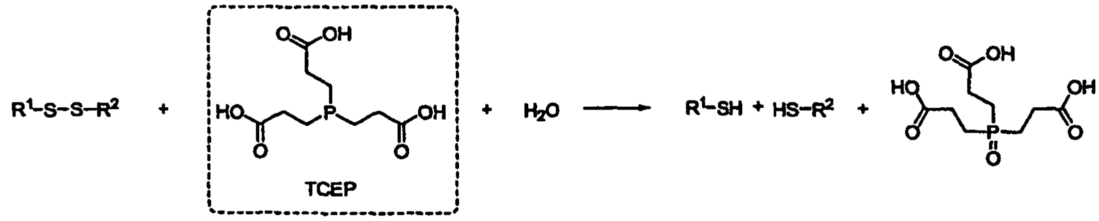

More details on the reduction of disulfide bonds can be found in Kirley, T.L.(1989), Reduction and fluorescent labeling of cyst(e)ine-containing proteins for subsequent structural analysis, Anal. Biochem. 180, 231 and Levison, M.E., et al. (1969), Reduction of biological substances by water-soluble phosphines: Gamma-globulin. Experentia 25, 126-127.

*Linkers cleavable by enzymes*

The linker connecting the potential drug candidate with the identifier oligonucleotide or the solid support and the target can include a peptide region that allows a specific cleavage using a protease. This is a well-known strategy in molecular biology. Site-specific proteases and their cognate target amino acid sequences are often used to remove the fusion protein tags that facilitate enhanced expression, solubility, secretion or purification of the fusion protein. Various proteases can be used to accomplish a specific cleavage. The specificity is especially important when the cleavage site is presented together with other sequences such as for example the fusion proteins. Various conditions have been optimized in order to enhance the cleavage efficiency and control the specificity. These conditions are available and know in the art.

Enterokinase is one example of an enzyme (serine protease) that cut a specific amino acid sequence. Enterokinase recognition site is Asp-Asp-Asp-Asp-Lys (DDDDK), and it cleaves C-terminally of Lys. Purified recombinant Enterokinase is commercially available and is highly active over wide ranges in pH (pH 4.5-9.5) and temperature (4-45˚C).

The nuclear inclusion protease from tobacco etch virus (TEV) is another commercially available and well-characterized proteases that can be used to cut at a specific amino acid sequence. TEV protease cleaves the sequence Glu-Asn-Leu-Tyr-Phe-Gln-Gly/Ser (ENLYFQG/S) between Gln-Gly or Gln-Ser with high specificity.

Another well-known protease is thrombin that specifically cleaves the sequence Leu-Val-Pro-Arg-Gly-Ser (LVPAGS) between Arg-Gly. Thrombin has also been used for cleavage of recombinant fusion proteins. Other sequences can also be used for thrombin cleavage; these sequences are more or less specific and more or less efficiently cleaved by thrombin. Thrombin is a highly active protease and various reaction conditions are known to the public.

Activated coagulation factor FX (FXa) is also known to be a specific and useful protease. This enzyme cleaves C-terminal of Arg at the sequence Ile-Glu-Gly-Arg (IEGR). FXa is frequently used to cut between fusion proteins when producing proteins with recombinant technology. Other recognition sequences can also be used for FXa.

Other types of proteolytic enzymes can also be used that recognize specific amino acid sequences. In addition, proteolytic enzymes that cleave amino acid sequences in an un-specific manner can also be used if only the linker contains an amino acid sequence in the complex molecule.

Other type of molecules such as ribozymes, catalytically active antibodies, or lipases can also be used. The only prerequisite is that the catalytically active molecule can cleave the specific structure used as the linker, or as a part of the linker, that connects the encoding region and the displayed molecule or, in the alternative the solid support and the target.

A variety of endonucleases are available that recognize and cleave a double stranded nucleic acid having a specific sequence of nucleotides. The endonuclease Eco RI is an example of a nuclease that efficiently cuts a nucleotide sequence linker comprising the sequence GAATTC also when this sequence is close to the nucleotide sequence length. Purified recombinant Eco RI is commercially available and is highly active in a range of buffer conditions. As an example the Eco RI is working in in various protocols as indicted below (NEBuffer is available from New England Biolabs):

NEBuffer 1 : [10 mM Bis Tris Propane-HCl, 10 mM MgCl2, 1 mM dithiothreitol (pH 7.0 at 25˚C)],
NEBuffer 2 : [50 mM NaCl, 10 mM Tris-HCl, 10 mM MgCl2, 1 mM dithiothreitol (pH 7.9 at 25˚C)],
NEBuffer 3 : [100 mM NaCl, 50 mM Tris-HCl, 10 mM MgCl2, 1 mM dithiothreitol (pH 7.9 at 25˚C)],
NEBuffer 4 : [50 mM potassium acetate, 20 mM Tris-acetate, 10 mM magnesium acetate, 1 mM dithiothreitol (pH 7.9 at 25˚C)].
Extension buffer: mM KCl, 20 mM Tris-HCl(Ph 8.8 at 25˚ C), 10 mM (NH4 )2 SO4 , 2 mM MgSO 4 and 0.1 % Triton X-100, and 200 μM dNTPs.

Formation of a mixture of homo- and hetero-duplexes

After the identifier oligonucleotides of complexes comprising display molecules interacting in a certain desired fashion with a target are partitioned, a nucleic acid amplification is usually conducted. The nucleic acid amplification method may be PCR or a method aquivalent thereto. The amplification is preferably conducted such that the relative destribution of the individual identifier oligonucleotides are retained. According to a certain embodiment, identical PCR priming sites to obtain a proportional amplification of the identifier oligonucleotides surround the coding section.

After the formation of duplexes, a denaturing step usually follows. A denaturing can be obtained by a variety of methods, including increased temperature, high salt concentration, presence of organic solvents, certain duplex disrupting chemicals, etc. Generally it is preferred to use an elevated temperature. When the temperature is increased above the melting temperature of the duplex the single stranded oligonucleotides are formed. At the elevated temperature, single stranded oligonucleotides complementing the partitioned identifier oligonucleotides optionally may be added.

A mixture of homo- and hetero-duplexes is formed by subjecting the single stranded mixture to hybridisation conditions. When the denaturing is obtained by elevating the temperature, the hybridisation conditions may appropriately be obtained by decreasing the temperature below the melting temperature of the homo-duplex. The temperature decrease rate may be adjusted according to the specific conditions used to obtain the optimal condition for the formation of the homo-duplexes. If a high temperature decrease rate is selected a lower tendency of homo-duplex formation is obtained. Conversely, a low temperature decrease rate implies a less tendency of hetero-duplex formation. When the denaturing is obtained using hybridisation modifying agents, like salt, solvents etc, hybridisation conditions may be obtained by desalination in case of salt and evaporation in case of solvents.

As mentioned above, the mixture of denatured single stranded oligonucleotides may be added complementing oligonucleotides to obtain certain desired effects. In an aspect of the invention random oligonucleotides are added to the single stranded oligonucleotides to lower the general tendency of duplex formation. A less tendency may be desirable because the selectivity for the best binding display molecule increases. In another aspect of the invention, certain specific complementing oligonucleotides are added to increase the probability of duplex formation for certain kind of identifier oligonucleotides. Alternatively, the addition of certain complementing oligonucleotides may compensate for an initial library not having an even distribution of the concentration of individual members.

In certain applications of the invention it is preferred to use amplification of only one strand, thereby to produce single stranded identifier oligonucleotides. The single stranded identifier oligonucleotides may then be mixed with a mixture of oligonucleotides complementing the original oligonucleotides of the library. The more frequent abundant single stranded identifier oligonucleotides will be more inclined to form a higher portion of homo-duplex compared to the less frequently occurring, which will tend to produce a higher portion of hetero-duplex. It may be desired to spike the partitioned fraction of single stranded identifier oligonucleotides with a higher concentration of certain complementing oligonucleotides in order to bias the formation of homo- and hetero-duplexes. Alternatively, certain complementing oligonucleotides may be added in a lower amount. As an example, the identifier oligonucleotide coding for a known ligand may be extinguished because it is desired to find unknown ligands, by avoiding the addition of an oligonucleotide complementing said identifier oligonucleotide.

In some aspects of the invention, a library of different display molecules, each being associated with an identifying oligonucleotide, is divided into two or more portions. A first portion may be contacted with a target and the identifier oligonucleotides of successful display molecules harvested, while a second portion may be contacted with a blank vessel or a second target and the identifier oligonucleotides of successful display molecules of the second portion collected. Prior to the hetero- and homo-duplex formation, the identifier oligonucleotides from the two portions are mixed. The advantages of screening two or more portions of a library individually include changing the profile of the background, obtaining subtype selectivity of the display molecule etc. It is also possible to combine two or more libraries before the contacting with the target to obtain an altered profile of binding display molecules.

Recovery of homo-duplexes

The recovery of homo-duplex is typically obtained by removing the hetero-duplexes form the mixture of hetero-duplexes and homo-duplexes. In another aspect of the invention, the homo-duplexes are extracted form the mixture. When the hetero-duplexes are removed from the reaction mixture, any convenient method can be used, such as a mis-match binding, enzymatic or chemical mis-match cleavage, or physical method.

*Mis-match binding*

In a certain aspect of the invention, hetero-duplexes are removed by binding to prokaryotic or eukaryotic mis-match binding proteins. An example is MutS, a mismatch binding protein isolated from E. coli, which recognises regions of double-stranded DNA containing a mismatched base pair (Wagner el al., 1995, Nucleic Acids Research, 22,

1541-1547) as well as 1 to 4 base pair insertion-deletion loops. MutS is allowed to bind to the hetero-duplexes and bound hetero-duplex/MutS complexes are removed from the reaction mixture using, for example, powdered nitrocellulose. A convenient alternative is to use MutS conjugated to magnetic beads, allowing bound heteroduplexes to be removed from the reaction mixture with a magnet. MutS may also be conjugated to biotin and the bound hetero-duplexes removed from the mixture using streptavidin-coated beads.

MutY has considerable potential for mismatch detection, as its *in vivo* function is to repair mismatched G:A base pairing by cleavage of the adenine-containing strand. Similar proteins thought to be involved in G:T and G:U mismatch repair have also been described. Hsu (Carcinogenesis 1994;15:1657-62) described the use of *E. coli* MutY protein for the detection of mismatched G:A in p53. As low as 1-2% mutant DNA in a sample of mutant and wild-type DNA could be detected using a synthetic DNA oligonucleotide to create G/A mis-pairing.

*Enzymatic cleavage*

Chemical and enzymatical cleavage methods used for degradation of hetero-duplexes must differentially cleave these sequences and retain the homo-duplexes. Any sequence difference will result in the formation of a mispairing, causing localized distortion of the double helix. Cleavage techniques exploit this structural change by selectively degrading or modifying DNA at the site of the mismatch. Ideally, little or no cleavage would be seen in a perfectly matched DNA fragment, and all distortions of the helix generated by base mismatches would result in cleavage. In practice neither criteria a fully met, and the utility of a technique becomes a trade-off between ease of use, sensitivity and specificity.

In certain aspect of the invention mammalian or bacterial endonucleases are used to recognise and cleave the hetero-duplexes at mismatched nucleobases (see U.S. Pat. No. 5,824,471). Examples of preferred enzymes include bacteriophage resolvases such as T4 endonuclease VII or T7 endonuclease I. In a preferred aspect of the invention, thermostable cleavage enzymes would be used in order to avoid the necessity of adding fresh enzyme during each round of heteroduplex formation and removal.

An enzyme called "Cleavase" from Third Wave Technologies relies on the endonucleolytic cleavage of stem-loop structures. The precise nature of the thermostable enzyme "Cleavase" has not been published. Since the stem-loop profile is dependent upon the primary sequence of the DNA, sequence changes in some cases result in a change in the cleavage profile. This method is unique in that unlike all other enzymatic techniques, it does not require the formation of a mismatch heteroduplex to generate a site for enzymatic cleavage.

Ribonuclease A cleavage was originally described by Myers et al (Science 1985; 230:1242-6) using DNA:RNA hybrids. Sensitivity was reported to be around 60% per strand cleaved. Grange et al (Nucleic Acids Research 1990; 18: 4227-36) described improved sensitivity by screening both strands of RNA. A Non-Isotopic RNase Cleavage Assay (NIRCA) has also been described. This assay (commercially available from Ambion) utilized PCR primers with phage RNA polymerase promoters so that large quantities of RNA were produced. Cleaved products could be detected on agarose gels and fragments of up to 1 kilobase were analyzed. Additional RNase enzymes such as RNase 1 and RNase T1 increase the sensitivity of the assay. The commercial kit includes a helix modifying reagent that makes the mismatches more sensitive to cleavage.

T4 endonuclease VII (T4E7) and T7E1 are small proteins from bacteriophages that bind as homodimers and cleave aberrant DNA structures including Holliday Junctions (and are hence sometimes called "resolvases") though it is far from clear that they perform such a role *in vivo*. Mashal et al (Nature Genetics 1995; 9:177-83) observed that they preferentially cleaved mismatched hetero-duplexes, leading to the possibility of an enzymatic equivalent to the chemical cleavage of mismatch. DNA requires no special preparation after amplification like GC clamping or including primers with 'phage promoters. Background peaks which are seen are highly reproducible and may therefore amenable to background subtraction algorithms like those applied to DNA sequencing traces. A commercially available T4E7-based mutation detection kit is available from Amersham-Pharmacia.

A plant endonuclease (CEL I) with similar activity has also been described. CEL I is one of series of plant endonucleases with similar activity to nuclease S1 but at neutral pH instead of pH 4 or 5. Like T4E7, the cleavage efficiency varies according to the mismatch examined and background cleavage is dependent on the template being examined.

Uracil glycosylase and photo-activated guanine modification reagent have been used to develop a cleavage method that essentially produces T or G sequencing tracks. DNA synthesis by PCR requires the incorporation of a proportion of uracil bases in place of thymines. These can be removed by uracil glycosylase and the abasic site then cleaved by heat or enzymatic treatment.

The present invention also includes any combination of enzymes.

*Chemical cleavage*

Chemical cleavage of mismatches was developed as a modification of the Maxam-Gilbert DNA sequencing

method by Cotton et al (Proc Natl Acad Sci (US) 1988;85: 4397-401). Mismatched thymines are susceptible to modification by osmium tetroxide (or potassium permanganate and tetraethyl ammonium acetate) and mismatched cytosines can be modified by hydroxylamine. The modified bases are then cleaved by hot piperidine treatment.

*Physical method*

The physical method of separating the hetero-duplex and homo-duplex molecules involves physical separation, such as achieved by chromatography or electrophoresis. Suitable chromatography methods include column chromatography, affinity chromatography, size-exclusion chromatography and gel chromatography. Suitable gels for gel chromatography include non-denaturing gels, such as agarose or polyacrylamide gels. The chromatography can be performed at elevated temperatures, e.g. above ambient temperatures, to favour the formation of homo-duplexes. In a certain aspect of the invention, the temperature is selected between the average melting temperature of the homo-duplexes and the melting temperature of the hetero-duplexes having the least amount of mis-matches. Generally, a temperature for performing gel chromatography is selected in the range of 45 to 80 degrees Celsius.

Suitable physical methods notably include denaturing high performance liquid chromatography (DHPLC) and chemical or temperature denaturing electrophoresis. Denaturing HPLC is a chromatographic technique capable of separating hetero-duplex and homo-duplex DNA molecules in a mixture. The mixture is applied to a stationary reverse-phase support and the homo- and hetero-duplex molecules are eluted (under thermal or chemical conditions capable of partially denaturing hetero-duplexes) with a mobile phase containing an ion-pairing reagent (e.g. triethylammonium acetate; TEAA) and an organic solvent (e.g. acetonitrile; AcN). DHPLC can also allow the direct quantisation of relative homo-duplex and hetero-duplex concentrations by the detection of ultraviolet absorbance or fluorescent emission of/from the separated species. The area under the absorbance/emission peak is proportional to the amount of product, which therefore allows quantitative assessment of the relative proportions. DHPLC is described in Liu W et al. (Nucleic Acids Research. 26:1396-1400, 1998 and O'Donovan MC et al. Genomics. 52:4449, 1998).

A preferred method for use in the instant invention to separate hetero-duplex and homo-duplex molecules is as described in U.S. Pat. No. 5,795,976.

<u>Determining the identifier oligonucleotide sequence</u>

The nucleotide sequence of the identifier sequence is determined to identify the identity of the binding display molecule(s). In a certain embodiment of the invention, chemical entities that participated in the formation of the display molecules that binds to the target are identified. The synthesis method of the display molecule may be established if information on the chemical entities as well as the point in time they have been incorporated in the display molecule can be deduced from the identifier oligonucleotide. It may be sufficient to obtain information on the chemical structure of the various chemical entities that have participated in the formation of the display molecule to deduce the full molecule due to structural constraints during the formation. As an example, the use of different kinds of attachment chemistries may ensure that a chemical entity can only be reacted at a single position on a scaffold. Another kind of chemical constrains may be present due to steric hindrance on the scaffold molecule or the chemical entity to be transferred. In general however, it is preferred that information can be inferred from the identifier oligonucleotide sequence that enable the identification of each of the chemical entities that have participated in the formation of the encoded molecule along with the point in time in the synthesis history the chemical entities have been incorporated in the (nascent) display molecule.

Although conventional DNA sequencing methods are readily available and useful for this determination, the amount and quality of isolated bifunctional molecules may require additional manipulations prior to a sequencing reaction.

Where the amount is low, it is preferred to increase the amount of the oligonucleotide sequences by polymerase chain reaction (PCR) using PCR primers directed to primer binding sites present in the identifier oligonucleotide sequence.

In one embodiment, the different identifier oligonucleotide sequences are cloned into separate sequencing vectors prior to determining their sequence by DNA sequencing methods. This is typically accomplished by amplifying the different identifier oligonucleotide sequences by PCR and then using a unique restriction endonuclease sites on the amplified product to directionally clone the amplified fragments into sequencing vectors. The cloning and sequencing of the amplified fragments then is a routine procedure that can be carried out by any of a number of molecular biological methods known in the art.

Alternatively, the bifunctional complex or the PCR amplified identifier oligonucleotide sequence can be analysed in a microarray. The array may be designed to analyse the presence of a single codon or multiple codons in a identifier oligonucleotide sequence.

Another approach, the identifier oligonucleotide product is analysed by QPCR. Preferably, the QPCR affords information as to the chemical moieties that has participated in the formation of the display molecules. The QPCR approach also allows a direct investigation of the enrichment factor if two samples are analysed in parallel, said samples being collected before and after the use of the present method. Various conditions can be investigated to obtain the

most optimal selection procedure before the sequences are analysed to identify the precise structures of the binding molecules.

A still further method for decoding the identifier oligonucleotides comprises high throughput sequencing, such as the Pyrosequencing method of 454 Lifesciences.

According to the invention, the homo-duplexes are used to deduce the identity of the display molecule(s) interacting with a target. The homoduplexes may be used directly from the recovery step or modified by any biotechnological technique prior to decoding. Notably, the modification can include total or partial amplification of the homo-duplexes to produce a double stranded or single stranded product. Also the modification may include fragmentation, e.g. digestion by a restriction enzyme or another nucleic acid active enzyme. In a certain embodiment, a restriction site is positioned between codons to allow for a separation of codons of the identifier oligonucleotides. The fragmentation may facilitate the subsequent decoding, as small nucleic acids usually are easier to decode.

The recovered duplexes of step e may one or more times be recycled to step d. The recycling may reduce the diversity at the nucleic acid level and at the same time increase the probability that an identifier oligonucleotide from a display molecule interacting with a target is identified when sequencing a limited number of homo-duplexes. Usually, when two or more sequences of a single identifier are detected during the sequencing, this is an indication that a display molecule performing an interaction with the target is identified. The repetition of the hetero- and homo-duplexes formation and recovery of the homo-duplexes may be conducted a suitable number of times until two or more identifier oligonucleotides in a sequencing step of a limited number of homo-duplexes is revealed.

In a certain embodiment of the invention, therefore, a decoding of the homo-duplex is effected prior to a consecutive repetition. The information of the decoding step may be used to modify the composition of the identifier oligonucleotides and strands complementary thereto. Notably, the composition can be modified by removing certain identifier oligonucleotides from the pool. As an example, identifiers of known display molecules interacting with a target can be removed to reveal other display molecules in the library having an ability to perform the same interaction albeit, to a lesser extend.

Various methods for excluding certain identifier oligonucleotides are available, including removal by an immobilized probe, digestion with a sequence specific nuclease etc. An appealing method includes that the partitioned fraction of identifier oligonucleotides is split in two portions. A first portion is then treated as described above to recover homo-duplexes. The recovered homo-duplexes are used to treat the second portion. In a certain aspect of the invention it is preferred to amplify the recovered homo-duplexes using primers conjugated to biotin. When the amplification product containing biotin is mixed with the second portion of the partitioned identifiers under denaturing conditions and subsequently subject to at least partly renaturing conditions, the strands with attached biotin may anneal with a complement in the mixture. A subsequent treatment with streptavidin or avidin allows the biotin labelled duplexes selectively to be removed from the mixture.

## Preferred embodiments of the present invention

The present invention relates to a method for identifying a display molecule of a bifunctional complex further comprising an identifier oligonucleotide linked to the display molecule, said method comprising the steps of

i) separating the complementary single stranded identifier oligonucleotides from an enriched and/or partitioned fraction of bifunctional complexes having an affinity for a target molecule, said bifunctional complexes comprising a duplex identifier oligonucleotide comprising said complementary single stranded identifier oligonucleotides,
ii)hybridising the single stranded identifier oligonucleotides obtained in step i), thereby generating a composition comprising homo-duplex identifier oligonucleotides and hetero-duplex identifier oligonucleotides, wherein homo-duplex identifier oligonucleotides hybridise without generating any mis-matches between the complementary oligonucleotide strands, and wherein one or more mis-matches are present when complementary identifier oligonucleotides form hetero-duplexes,
iii) separating homo-duplexes from hetero-duplexes,
iv) obtaining a fraction comprising a higher percentage of homoduplexes than in the fraction used in step i), such as obtaining a fraction comprising predominantly homo-duplexes,
v) decoding the identifier oligonucleotides of the homo-duplexes, and
vi) identifying based on the decoding in step v) the one or more display molecules identifier by the decoded identifier oligonucleotides.

Said enriched and/or partitioned fraction of bifunctional complexes is preferably obtained from a naïve "starting library" which has been through at least one partitioning and/or selecting step, such as 2, 3, 4, 5, 6 or 7 partitioning and/or selecting steps, such as 4 partitioning and/or selecting steps, to form the enriched and/or partitioned fraction of bifunctional complexes.

Steps i) to iii) described above may be repeated one or more times to further enrich the enriched and partitioned fraction.

After step iv), an optional step may be added in which the identifier nucleotides from the fraction obtained in step iv) are (optionally after amplification) used to contact either the fraction used in step i) (or a partitioned variant thereof), and/or the original naïve library used to generate the fraction used in step i) (or a partitioned variant thereof). Those bifunctional molecules from these earlier libraries identified as having affinity to the nulceotide tag of the fraction obtained in step iv) (or to the complementary sequence of the nucleotide tag) may then be used to enrich the library obtained in step iv). This optional step may be repeated as many times as is required to obtain the required enrichment of the library, optionally after repeating steps i)-iii) again.

Preferably, the duplex identifier oligonucleotide comprising complementary single stranded identifier oligonucleotides is obtained by hybridisation of a complementary identifier oligonucleotide with the identifier oligonucleotide linked to the display molecule of the bifunctional complex.

Equally preferably, the duplex identifier oligonucleotide comprising complementary single stranded identifier oligonucleotides is obtained by hybridising a probe oligonucleotide with the identifier oligonucleotide of the bifunctional complex, enzymatically extending said probe oligonucleotide, thereby obtaining a complementary identifier oligonucleotide hybridised with the identifier oligonucleotide linked to the display of the bifunctional complex.

The duplex identifier oligonucleotide comprising complementary single stranded identifier oligonucleotides may be obtained prior to, during, concomitantly with or after the step of partitioning the bifunctional complexes.

Preferably the identifier oligonucleotide linked to the display molecule is selected from the group consisting of a) a single stranded identifier oligonucleotide and b) a duplex identifier oligonucleotide comprising complementary single stranded identifier oligonucleotides, said method comprising the preliminary steps (i.e. preferably prior to step (i), described above) of

a) providing a composition of bifunctional complexes comprising different display molecules,
b) providing one or more target molecule(s) having an affinity for one or more of said display molecules (wherein said one or more target molecules may be different and/or the same),
c)contacting the composition of bifunctional complexes provided in step i) with the target molecule provided in step ii)
d)obtaining an enriched fraction of bifunctional complexes comprising one or more display molecules having an affinity for said target molecule,
e) partitioning said enriched fraction obtained in step iv) from bifunctional complexes not having an affinity for said target molecule, and
f) complementing single stranded identifier oligonucleotides of different bifunctional complexes in which the display molecule is linked to a) a single stranded identifier oligonucleotide, thereby obtaining a duplex identifier oligonucleotide comprising complementary identifier oligonucleotides, with the proviso that no single stranded complementation occurs for bifunctional complexes comprising b) duplex identifier oligonucleotides comprising complementary identifier oligonucleotides.

After step d), an optional step may be added in which the identifier nucleotides from the fraction obtained in step d) are (optionally after amplification) used to contact either the composition used in step a) (or a partitioned variant thereof), and/or the original naïve library used to generate the composition used in step i) (or a partitioned variant thereof). Those bifunctional molecules from these earlier libraries identified as having affinity to the nucleotide tag of the fraction obtained in step d) (or to the complementary sequence of the nucleotide tag) may then be used to enrich the library obtained in step d). This optional step may be repeated as many times as is required to obtain the required enrichment of the library, optionally after repeating steps a)-d) again.

The display molecule of a bifunctional complex provided in step a) or obtained in step d) may be linked to a single stranded identifier oligonucleotide, wherein said method further comprises the step of complementing said single stranded identifier oligonucleotides of the different bifunctional complexes, thereby obtaining a duplex identifier oligonucleotide comprising complementary identifier oligonucleotides. Said step of complementing said single stranded identifier oligonucleotide may occur prior to, during or concomitantly with, or after partitioning of the bifunctional complexes.

The complementing step may comprise the steps of hybridising one or more oligonucleotide probe(s) to the single stranded identifier oligonucleotide of at least some of the bifunctional complexes, and ligating and/or extending enzymatically said probe(s), thereby generating a duplex identifier oligonucleotide comprising complementary oligonucleotide strands.

Also, the complementation of the single stranded identifier oligonucleotides may comprise the step of hybridising a complementary identifier oligonucleotide to the single stranded identifier oligonucleotide of at least some of the bifunctional complexes.

In one embodiment, the display molecule of at least some of the bifunctional complexes are linked to duplex identifier oligonucleotides comprising complementary oligonucleotide strands.

In one embodiment of the method disclosed herein, hetero-duplexes are selectively removed or eliminated from the composition comprising homo-duplex identifier oligonucleotides and hetero-duplex identifier oligonucleotides. This may e.g. be carried out by enzymatically degrading the mis-matched, single stranded part of the hetero-duplexes. The enzyme used may e.g. comprise nuclease activity or consist of a polypeptide having nuclease activity, for example an enzyme selected from T4 endonuclease VII, T4 endonuclease I, CEL I or nuclease S1, including variants and combinations thereof. Said enzyme is preferably thermostable.

In one embodiment of the present invention, the chemical entities are precursor chemical entities which are processed in the synthesis of the display molecule, wherein the processed chemical entities form part of the display molecule.

In one embodiment of the present invention, each codon comprises 4 or more nucleotides.

The chemical entity reactions may occur without the intervention of an enzyme.

In one preferred embodiment, the linker linking the display molecule and the identifier oligonucleotide is selectively cleavable. For example, the linker may be cleaved by irradiation. The cleavage preferably occurs after the bifunctional complex has contacted the target.

The composition of bifunctional complexes may comprise more than 1000 different display molecules.

In one embodiment of the present invention, the target molecule is of a biological origin. The molecular target may be immobilized on a solid support, for example the immobilized target may form a stable or quasi-stable dispersion. The target molecule may comprise or consist of a polypeptide, such as selected from the group consisting of kinases, proteases, phosphatases, and antibodies. In another embodiment of the present invention, the target molecule may comprise or consist of a nucleic acid, such as consisting of a nucleic acid. Said nucleic acid may e.g. be a DNA or RNA aptamer. In one preferred embodiment of the present invention, the target molecule, such as a polypeptide, is associated with the nucleic acid template having templated the synthesis thereof. Further suitable target molecules for use in the present invention include, but are not limited to :

(1) Receptors, e.g.,andrenergic receptors, histamine receptors, dopamine receptors, alpha-V-beta-3 integrin
(2) Kinases, e.g., ROCK-1, CaM kinase, cdk5, MAPKAP kinase, PKC gamma, p38, PRAK, mTOR, Raf, WNK3
(3) Polymerases, e.g., HIV reverse transcriptase,
(4) Transcription factors, e.g., BRCA1, p53,
(5) Proteases, e.g., HIV protease, thrombin,
(6) Phosphatases, e.g., PTP 1 B, Cdc25A, (7) Cytokines, e.g., TNF-alpha,
(8) Antibodies, e.g., antibodies raised against any of the mentioned targets or other macromolecules.

Furthermore, suitable targets may have two or more of the above functions

In one preferred embodiment, the contacting of the target molecule and the composition of different bifunctional complexes is achieved by mixing the target molecule with the composition of different bifunctional coplexes. In such cases, the target is optionally saturated with a known ligand prior to contacting the different bifunctional complexes.

In one embodiment of the present invention, at least one of the complementary identifier oligonucleotides of the recovered homo-duplexes is amplified prior to the step of decoding the identity of the display molecule.

The partitioned fraction of identifier oligonucleotides may e.g. be amplified by PCR, such as being proportionally amplified.

In one embodiment of the present invention, the complementary identifier oligonucleotide strands of homo-duplexes and hetero-duplexes in the fraction comprising predominantly homo-duplexes are separated and allowed to hybridise, said hybridisation resulting in a further fraction comprising predominantly homo-duplexes. Furthermore, the complementary identifier oligonucleotide strands of homo-duplexes and hetero-duplexes in the fraction comprising predominantly homo-duplexes may optionally be amplified before being re-hybridised and/or the identifier oligonucleotides may be decoded before being re-hybridised. Said decoding may be used to eliminate a subset of identifier oligonucleotides before the step of re-hybridisation.

*Preferred methods for obtaining the display molecule useful in the inventive methods described herein*

Any suitable method may be used for generating the display molecule useful in the inventive methods described herein. In said method for generating the display molecules for use in the present invention, it is preferred that at least 3 chemical entities, such as at least 4 chemical entities, for example at least 5 chemical entities, such as at least 6 chemical entities, for example at least 7 chemical entities, such as at least 8 chemical entities, for example at least 9 chemical entities, such as at least 10 chemical entities, for example at least 11 chemical entities, such as at least 12 chemical entities, for example at least 13 chemical entities, such as at least 14 chemical entities, for example at least 15 chemical entities, such as at least 16 chemical entities, for example at least 17 chemical entities, such as at least 18 chemical entities, for example at least 19 chemical entities, such as at least 20 chemical entities, are reacted.

In one embodiment of the method disclosed herein, the display molecule is obtained by reacting two or more chemical entities, wherein each chemical entity is preferably linked to a building block comprising one or more anti-codons, wherein said anti-codon(s) is complementary to an identifier oligonucleotide codon(s), wherein said codons or anti-codons identifies the chemical entities having reacted to generate the display molecule. Said codons may be separated by a framing sequence.

*Templated molecules and methods for using such molecules*

The display molecule useful in the inventive method described herein may in one embodiment be obtained by a method comprising the steps of

i) providing at least one template comprising a sequence of n coding elements,
wherein each coding element comprises at least one recognition group capable of recognising a predetermined complementing element, and
wherein n is an integer of more than 1,

ii) providing a plurality of building blocks, wherein each building block comprises

a) at least one complementing element comprising at least one recognition group capable of recognising a predetermined coding element,

b) at least one functional entity comprising at least one functional group and at least one reactive group, and

c) at least one linker separating the at least one functional entity from the at least one complementing element,

iii) contacting each of said coding elements with a complementing element capable of recognising said coding element,

iv) optionally obtaining a complementing template by covalently linking the complementing elements, and

v) obtaining a display molecule by reacting functional groups on different building blocks,

wherein the display molecule is linked to a building block or to the complementing template, when formed, or to the template that templated the synthesis of the display molecule. Further embodiments of this method that are suitable for use in the present invention are described in PCT application with publication number WO 02/103008 ("Templated Molecules and Methods for Using such Molecules").

*Ligational encoding of Small Molecules*

In another embodiment of the present invention, said display molecule may be obtained by a method comprising the steps of

i) providing at least one template comprising one or more codons capable of hybridising to an anti-codon, wherein said template is optionally associated with one or more chemical entities, and
ii) providing a plurality of building blocks each comprising an anti-codon associated with one or more chemical entities,
iii) hybridising the anti-codon of one or more of the provided building blocks to the template,
iv) covalently linking said anti-codons and/or linking the at least one template with the anti-codon of at least one building block, thereby generating an identifier oligonucleotide capable of identifying chemical entities having participated in the synthesis of the display molecule,
v) displacing or separating the template from one or more of the anti-codons hybridised thereto, thereby generating an at least partly single stranded identifier oligonucleotide associated with a plurality of chemical entities,
vi) generating a bifunctional complex comprising a display molecule linked to an identifier oligonucleotide identifying the chemical entities having participated in the synthesis of the display molecule,

wherein said display molecule is generated by reacting at least two of said plurality of chemical entities associated with the identifier polynucleotide,
wherein said at least two chemical entities are provided by separate building blocks or provided by the template and at least one building block.

Further embodiments of this method that are suitable for use in the present invention are described in PCT application with publication number WO 2004/083427 ("Ligational encoding of Small Molecules.

*Quasirandom structure and Function Guided Synthesis Methods*

In another embodiment of the present invention, said display molecule may be obtained by a method comprising the steps of

i) providing a plurality of building blocks each comprising an oligonucleotide associated with one or more chemical entities,

ii) providing at least one connector oligonucleotide capable of hybridising with one or more building block oligonucleotides,

iii) immobilising at least one building block to a solid support,

iv) hybridising said immobilised building block oligonucleotide to a first connector oligonucleotide,

v) hybridising at least one additional building block oligonucleotide to said first connector oligonucleotide,

vi) ligating building block oligonucleotides hybridised to the connector oligonucleotide,

vii) separating the connector polynucleotide from the ligated building block oligonucleotides,

viii) reacting one or more chemical entities associated with different building block oligonucleotides, thereby obtaining a first bifunctional complex comprising a first display molecule or first display molecule precursor linked to a first identifier oligonucleotide identifying the chemical entities having participated in the synthesis of the first display molecule or first display molecule precursor, wherein said first bifunctional complex is immobilised to a solid support.

Said chemical entities are optionally reacted in a reaction compartment from which the connector oligonucleotide has been removed in a washing and/or separation step prior to the reaction of said chemical entities. The method for obtaining one or more display molecules may further comprise the steps of:

i) providing a second connector polynucleotide,

ii) hybridising said second connector polynucleotide to the identifier polynucleotide of said first bifunctional complex,

iii) hybridising at least one further oligonucleotide of a building block to said second connector oligonucleotide,

iv) ligating building block oligonucleotides hybridised to the second connector oligonucleotide, wherein at least one of said building block oligonucleotides are hybridised to the first identifier polynucleotide,

v) separating the second connector polynucleotide from the ligated building block oligonucleotides, preferably by diverting the second connector polynucleotide to another compartment,

vi) reacting the first display molecule precursor with the one or more chemical entities associated with the ligated building block oligonucleotide(s), thereby obtaining a second bifunctional complex comprising a second display molecule or second display molecule precursor linked to a second identifier polynucleotide identifying the chemical entities having participated in the synthesis of the display molecule or display molecule precursor,

wherein said second bifunctional complex is immobilised to a solid support.

Furthermore, said steps i) to vi) may be repeated for different connector oligonucleotides and different further building blocks.

In another embodiment of the present invention, said display molecule may be obtained by a method comprising the steps of

i) providing a plurality of building blocks selected from the group consisting of

a) building blocks comprising an identifier oligonucleotide linked to one or more chemical entities,

b) building blocks comprising an identifier oligonucleotide linked to one or more reactive groups, and

c) building blocks comprising an identifier oligonucleotide comprising a spacer region, wherein said building blocks comprising a spacer region are preferably connector polynucleotides to which building blocks of groups a) and b) can hybridise,

ii) generating a hybridisation complex comprising at least n building blocks by hybridising the identifier oligonucleotide of one building block to the identifier oligonucleotide of at least one other building block,
wherein n is an integer of 4 or more
wherein at least 3 of said at least n building blocks comprise a chemical entity,
wherein no single identifier oligonucleotide is hybridised to all of the remaining identifier oligonucleotides,
wherein optionally at least one of said building blocks of group c) is immobilised to a solid support, thereby providing a handle to which an oligonucleotide of at least one building block of groups a) or b) can hybridise,

iii) covalently linking identifier oligonucleotides of building blocks comprising one or more chemical entities, thereby obtaining an identifier oligonucleotide comprising covalently linked identifier oligonucleotides each associated with one or more chemical entities,

iv) optionally separating said identifier oligonucleotide obtained in step iii) from any immobilised connector oligonucleotides hybridised thereto, wherein said separation optionally comprises the step of diverting said identifier polynucleotide comprising covalently linked identifier oligonucleotides each associated with one or more chemical entities to a different reaction compartment, thereby separating said identifier polynucleotide from said immobilised connector oligonucleotides,

v) reacting said at least 3 chemical entities linked to the identifier polynucleotide, and

vi) obtaining a bifunctional complex comprising a display molecule resulting from the reaction of a plurality of chemical entities, wherein said display molecule is linked to an identifier polynucleotide identifying the chemical entities having participated in the synthesis of the molecule.

The method steps may be repeated for different, or the same, building blocks.

Further embodiments of the above methods that are suitable for use in the present invention are described in PCT application with publication number WO 2004/056994 ("Quasirandom structure and function guided synthesis methods").

*Enzymatic Encoding*

In another embodiment of the present invention, said display molecule may be obtained by a method comprising the steps of

i) providing an oligonucleotide comprising a priming site linked to a) a reaction site comprising one or more reactive group(s) and b) a site for tag addition,

ii) providing a plurality of building blocks each comprising a probe oligonucleotide capable of hybridising with the priming site, wherein at least part of said plurality of building blocks comprise a probe oligonucleotide linked to different chemical entities,

iii) sequentially hybridising different building blocks to the priming site of the oligonucleotide provided in step i),

iv) reacting for each hybridised building block a chemical entity with a reactive group of the reaction site of the oligonucleotide provided in step i), and

v) adding for every chemical entity reacted a unique tag residue to the site for tag addition, said unique tag identifying the chemical entity having reacted with a reactive group of the reactive site.

In another embodiment of the present invention, said display molecule may be obtained by a method comprising

the steps of:

i) providing an oligonucleotide comprising a tag addition site and either one or more reactive site for functionalization, or a scaffold comprising one or more reactive site(s) for functionalization,

ii) reacting one or more scaffold reactive sites with two or more chemical entities, and

iii) adding for each chemical entity reacted with a scaffold reactive site a unique tag residue to the tag addition site, thereby obtaining a bifunctional complex comprising a display molecule resulting from the chemical entity reactions, said display molecule being linked to an identifier oligonucleotide comprising the individual unique tag residues.

Further embodiments of the above methods that are suitable for use in the present invention are described in PCT application with publication number WO 2004/039825 ("Enzymatic Encoding").

*Display molecules*

Any suitable display molecule may be used in the methods of the present invention. For example, the plurality of display molecules may be selected from the group consisting of peptides wherein the amino acid residues are in the L-form or in the D-form, vinylogous polypeptides, glycopolypeptides, polyamides, vinylogous sulfonamide peptides, polysulfonamides, conjugated peptides comprising e.g. prosthetic groups, polyesters, polysaccharides, polycarbamates, polycarbonates, polyureas, polypeptidylphosphonates, polyurethanes, azatides, oligo N-substituted glycines, polyethers, ethoxyformacetal oligomers, polythioethers, polyethylene glycols (PEG), polyethylenes, polydisulfides, polyarylene sulfides, polynucleotides, PNAs, LNAs, morpholinos, oligo pyrrolinones, polyoximes, polyimines, polyethyleneimines, polyimides, polyacetals, polyacetates, polystyrenes, polyvinyl, lipids, phospholipids, glycolipids, polycyclic compounds comprising e.g. aliphatic or aromatic cycles, including polyheterocyclic compounds, proteoglycans, and polysiloxanes, including any combination thereof,
wherein each display molecule is synthesised by reacting a plurality of chemical entities preferably in the range of from 2 to 200, for example from 2 to 100, such as from 2 to 80, for example from 2 to 60, such as from 2 to 40, for example from 2 to 30, such as from 2 to 20, for example from 2 to 15, such as from 2 to 10, such as from 2 to 8, for example from 2 to 6, such as from 2 to 4, for example 2, such as from 3 to 100, for example from 3 to 80, such as from 3 to 60, such as from 3 to 40, for example from 3 to 30, such as from 3 to 20, such as from 3 to 15, for example from 3 to 15, such as from 3 to 10, such as from 3 to 8, for example from 3 to 6, such as from 3 to 4, for example 3, such as from 4 to 100, for example from 4 to 80, such as from 4 to 60, such as from 4 to 40, for example from 4 to 30, such as from 4 to 20, such as from 4 to 15, for example from 4 to 10, such as from 4 to 8, such as from 4 to 6, for example 4, for example from 5 to 100, such as from 5 to 80, for example from 5 to 60, such as from 5 to 40, for example from 5 to 30, such as from 5 to 20, for example from 5 to 15, such as from 5 to 10, such as from 5 to 8, for example from 5 to 6, for example 5, such as from 6 to 100, for example from 6 to 80, such as from 6 to 60, such as from 6 to 40, for example from 6 to 30, such as from 6 to 20, such as from 6 to 15, for example from 6 to 10, such as from 6 to 8, such as 6, for example from 7 to 100, such as from 7 to 80, for example from 7 to 60, such as from 7 to 40, for example from 7 to 30, such as from 7 to 20, for example from 7 to 15, such as from 7 to 10, such as from 7 to 8, for example 7, for example from 8 to 100, such as from 8 to 80, for example from 8 to 60, such as from 8 to 40, for example from 8 to 30, such as from 8 to 20, for example from 8 to 15, such as from 8 to 10, such as 8, for example 9, for example from 10 to 100, such as from 10 to 80, for example from 10 to 60, such as from 10 to 40, for example from 10 to 30, such as from 10 to 20, for example from 10 to 15, such as from 10 to 12, such as 10, for example from 12 to 100, such as from 12 to 80, for example from 12 to 60, such as from 12 to 40, for example from 12 to 30, such as from 12 to 20, for example from 12 to 15, such as from 14 to 100, such as from 14 to 80, for example from 14 to 60, such as from 14 to 40, for example from 14 to 30, such as from 14 to 20, for example from 14 to 16, such as from 16 to 100, such as from 16 to 80, for example from 16 to 60, such as from 16 to 40, for example from 16 to 30, such as from 16 to 20, such as from 18 to 100, such as from 18 to 80, for example from 18 to 60, such as from 18 to 40, for example from 18 to 30, such as from 18 to 20, for example from 20 to 100, such as from 20 to 80, for example from 20 to 60, such as from 20 to 40, for example from 20 to 30, such as from 20 to 25, for example from 22 to 100, such as from 22 to 80, for example from 22 to 60, such as from 22 to 40, for example from 22 to 30, such as from 22 to 25, for example from 25 to 100, such as from 25 to 80, for example from 25 to 60, such as from 25 to 40, for example from 25 to 30, such as from 30 to 100, for example from 30 to 80, such as from 30 to 60, for example from 30 to 40, such as from 30 to 35, for example from 35 to 100, such as from 35 to 80, for example from 35 to 60, such as from 35 to 40, for example from 40 to 100, such as from 40 to 80, for example from 40 to 60, such as from 40 to 50, for example from 40 to 45, such as from 45 to 100, for example from 45 to 80, such as from 45 to 60, for example from 45 to 50, such as from 50 to 100, for example from 50 to 80, such as from 50 to 60, for example from 50 to 55, such as from 60 to 100, for example from 60 to 80, such as from 60 to 70, for example

from 70 to 100, such as from 70 to 90, for example from 70 to 80, such as from 80 to 100, for example from 80 to 90, such as from 90 to 100.

The display molecules may be polypeptides that are not alpha-polypeptides for example. In another embodiment of the present invention, the display molecules are natural or non-natural nucleic acids. In one embodiment, it is preferred that the display molecules have a molecular weight of less than 2000 Dalton, such as less than 1000 Dalton, for example less than 500 Dalton.

One or more chemical entities may be transferred to a display molecule or an intermediate thereof by a chemical building block further comprising an anti-codon, such as whereby the information of the anti-codon is transferred in conjunction with the chemical entity to the bifunctional complex.

Any of the methods described herein may be repeated one or more time to increase the level of partitioning and/or enrichment, such as by repeating said method once, twice, thrice, four times, five times, six times, seven times or more than seven times.

## DETAILED DISCLOSURE OF THE FIGURES

Figure 1 discloses the common selection process in schematic form. In diagram A an ideal library is shown that comprises a variety of different members of a library (Diversity) in the same concentration, i.e. with the same number of each bifunctional complex in the mixture.

The library represented in diagram A is subjected to a selection process. Generally, the selection process implies that the library is contacted with a target to allow for an interaction, usually a binding interaction, to take place. Ideally, the non-binding members of the library are discharged and the bifunctional complexes able to perform an interaction with the target are selected. However, in reality a certain amount of non-binding complexes are eluted together with the binding complexes. The non-binding complexes are referred to a background in diagram B. The background usually increases with the diversity, i.e. a large library generates a higher background relative to small library.

To increase the probability of finding a ligand, it is generally desired to apply a library as large as possible. The high background however generates a high level of noise so that a detection of the hits is difficult or even impossible. The present invention suggests a method to reduce the background so as to be able to identify the hit. In diagram C the background is broken down to individual molecules to illustrate that the amount of the binding ligand is higher than each of the molecules in the selected library. The imbalance formed due to the selection process between the hit and the remainder of the library members is then used in the subsequent steps of the present method.

Fig. 2 schematically discloses a homo-duplex and a hetero-duplex. A homo-duplex is an identifier oligonucleotide hybridised to a fully complementing oligonucleotide. A hetero-duplex is an identifier oligonucleotide hybridised to an oligonucleotide showing one, two, or more mis-matching nucleotides. A mis-matching nucleotide is a nucleotide not paired in accordance with the Watson-Crick base-pairing rules, in which A pairs with T (or U) and C pairs with G. In Fig. 2, the mis-matching nucleotide(s) are illustrated by a filled circle.

The homo- and hetero-duplexes may be formed by denaturing a PCR product of the result of the selection shown on Fig. 1 and subsequently allow the mixture to hybridise again. The denaturing is usually performed by heating to a temperature above the melting point of the PCR product. The hybridisation conditions are usually obtained by lowering the temperature well below the melting point of the homo-duplexes.

Fig. 3 illustrates various steps of the present invention. In a first step the library is subjected to a selection process as described in Fig. 1, in which the straight line depicts the identifier oligonucleotide from the complex of the binding display molecule. The output of the selection process is in a subsequent step subjected to a melting and reannealing step. Due to the excess of identifier oligonucleotides from the binding display molecule, the mixture of homo- and hetero-duplexes comprises a relatively higher content of homo-duplexes from the identifiers of the binding display molecules than homo-duplexes from other sources. In the final step shown on Fig. 3, the homo-duplexes are separated from the mixture, e.g. by cleavage of hetero-duplexes with an enzyme. The result is an enrichment of identifier oligonucleotides from binding display molecules.

Fig. 4 discloses a diagram of the overall principle of mis-match selection. Initially, a library of bifunctional molecules is subjected to a selection process. According to the broadest scope of the invention, any type of bifunctional complex library can be used, including phage display, ribosome display, and small molecule display. Following the selection, the identifier oligonucleotide is amplified using PCR or a similar method in order to generate more copies of the individual identifier oligonucleotides. Preferably, the amplification retains the proportion between the individual identifier oligonucleotides.

A number of times the following cycle can be repeated: The PCR amplicons are heated to a temperature above the melting point of the homo-duplexes and subsequent cooled to form a mixture of hetero-duplexes and homo-duplexes. The homo-duplexes are recovered from the mixture by i) binding the mis-matched duplexes to a protein, such as MutS, ii) cleaving the hetero-duplex using an appropriate enzyme, such as Cel I, or iii) physical means, such as DHPLC. If the result of the mis-match selection still comprises too much noise, i.e. it is not possible when sequencing a small amount

of sequences to deduce one or more sequences, which occurs more frequently than others, the cycle may be repeated, starting with a PCR amplification of the output of the mis-match selection.

The final step of the method includes an analysis of the output. A variety of techniques are available to the skilled person in the art, including sequencing using capillary electrophoresis, bead array, high-density microarray etc. If the mis-match selection has been successful, an analysis of a relatively few oligonucleotides will reveal which display molecules of the library that have the highest binding affinity, because the identifier oligonucleotides of complexes having display molecules with high binding affinity occurs more frequent.

As item 1 there is provided a method for obtaining display molecule(s) having affinity towards a target, comprising the steps of

a. providing a library comprising a plurality of different display molecules, each display molecule being associated with an identifier oligonucleotide, which codes for the identity of said display molecule,
b. contacting the library with a target to allow for an interaction between the display molecules of the library with the target,
c. partitioning a fraction enriched in identifier oligonucleotides of display molecules interacting with the target,
d. subjecting the fraction to conditions at which hetero-duplexes and homo-duplexes are formed,
e. recovering the homo-duplexes, and
f. deducing from the homo-duplexes the identity of the display molecule(s) interacting with the target.

Item 2 concerns the method of item 1, wherein in step c the identifier oligonucleotides of the library members are provided in homo-duplex form.
Item 3 concerns the method of items 1 and 2, wherein in step d, the conditions effect separation of the strands of the duplex identifier oligonucleotides, and subsequently hybridisation.
Item 4 concerns the method of any of items 1 to 3, wherein the homo-duplexes in step e are recovered by removal of hetero-duplexes.
Item 5 concerns the method of item 4, wherein the hetero-duplexes are removed by enzymatically degradation.
Item 6 concerns the method of item 5, wherein the enzyme is a nuclease.
Item 7 concerns the method of any of items 4 to 6, wherein the enzyme is selected from T4 endonuclease VII, T4 endonuclease I, CEL I, nuclease S1, or variants thereof.
Item 8 concerns the method of any of the items 5 to 7, wherein the enzyme is thermostable.
Item 9 concerns the method of item 1, wherein the display molecule is a reaction product of two or more chemical entities and the identifier oligonucleotide comprises codons identifying the chemical entities.
Item 10 concerns the method of item 9, wherein the chemical entities are precursors for a structural unit appearing in the display molecule.
Item 11 concerns the method of any of the items 9 to 10, wherein some or all of the chemical entities are not naturally occurring $\alpha$-amino acids or precursors thereof.
Item 12 concerns the method of item 9, wherein each codon comprises 4 or more nucleotides.
Item 13 concerns the method of any of the items 1 to 12, wherein the display molecules of the library are non-a-polypeptides.
Item 14 concerns the method of any of the items 1 to 13, wherein the display molecules of the library are non-nucleic acids.
Item 15 concerns the method of any of the items 1 to 14, wherein the display molecule has a molecular weight less than 2000 Dalton, preferably less than 1000, and most preferred less than 500 Dalton.
Item 16 concerns the method of any of the preceding items, wherein the identifier oligonucleotide uniquely identifies the display molecule.
Item 17 concerns the method of any of the items 1 to 16, wherein one or more chemical entities are transferred to the nascent display molecule by a chemical building block further comprising an anti-codon.
Item 18 concerns the method of item 17, wherein the information of the anti-codon is transferred in conjunction with the chemical entity to the nascent complex.
Item 19 concerns the method of any of the preceding items, wherein the chemical entities are reacted without enzymatic interaction.
Item 20 concerns the method of any of the items 1 to 19, wherein the codons are separated by a framing sequence.
Item 21 concerns the method of any of the items 1 to 20, wherein a selectively cleavable linker joins the display molecule and the identifier oligonucleotide.
Item 22 concerns the method of item 21, wherein the linker is cleaved by irradiation.
Item 23 concerns the method of any of the items, wherein the library comprises one, two or more different library members.

Item 24 concerns the method of any of the items 1 to 23, wherein the library comprises 1,000 or more different member.
Item 25 concerns the method of item 1, wherein the molecular target is of a biological origin.
Item 26 concerns the method of any of the items 1 to 25, wherein the molecular target is immobilized on a solid support.
Item 27 concerns the method of item 26, wherein the target immobilized on the support forms a stable or quasi-stable dispersion.
Item 28 concerns the method of items 26 or 27, wherein a cleavable linker is present between the solid support and the molecular target.
Item 29 concerns the method of any of the items 1 to 28, wherein the molecular target is a protein.
Item 30 concerns the method of item 29, wherein the protein is selected from the group consisting of kinases, proteases, phosphatases, and anti-bodies.
Item 31 concerns the method of any of the items 1 to 29, wherein the molecular target and/or the display molecule is a nucleic acid.
Item 32 concerns the method of item 31, wherein the nucleic acid is a DNA or RNA aptamer.
Item 33 concerns the method of any of the items 29 to 32, wherein the target protein is attached to the nucleic acid responsible for the formation thereof.
Item 34 concerns the method of any of the items 1 to 33, wherein the contacting step includes that a target is mixed with a library of complexes.
Item 35 concerns the method of item 34, wherein a target is saturated with a known ligand prior to the mixing step.
Item 36 concerns the method of item 1, wherein the recovered homo-duplexes of step e are amplified prior to decoding the identity of the display molecule.
Item 37 concerns the method of any of the items 1 to 36, wherein the partitioned fraction of identifier oligonucleotides of step c is amplified by PCR prior to step d.
Item 38 concerns the method of item 37, wherein the identifier oligonucleotides are proportionally amplified.
Item 39 concerns the method of any of the items 1 to 38, wherein the recovered homo-duplexes of step e one or more times are recycled to step d. Item 40 concerns the method of item 39, wherein the recovered homo-duplexes are amplified prior to the treatment according to step d.
Item 41 concerns the method of items 38 or 39, wherein a decoding occurs before recycling to step d.
Item 42 concerns the method of any of the items 39 to 41, wherein the information obtained from the decoding is used to modify the composition of the identifier oligonucleotides or complements thereof before recycling to step d.
Item 43 concerns the method of item 41, wherein the modification includes extinction of certain identifiers.

**Examples**

EXAMPLE 1. Statistical calculations of mismatch selection (MISE treatment in a library after initial selection.

*Theoretical procedure:*

This example describes the statistical calculation of MISE treatment simulating various libraries with certain size and diversity and different enrichment factors in the initial selection process. Below is definition of the parameters used in the calculations.

Steps (x):

1 : initial library
2 : library after selection
3 : library after first MISE round
4 : library after second MISE round
5 : library after third MISE round
6 : library after fourth MISE round

N(t,x) : Total number of molecules in the library in step x
N(p,x) : Total number of molecules in pool p in step x
A(t,x) : Total number of molecules formed in PCR amplification in step x
D(t,x) : Diversity (number of different molecules in library) in step x
D(p,x) : Diversity of pool p (number of different molecules in library) in step x
R(p) : Relative enrichment factor of pool p

SO(x) : Fraction of homoduplexes surviving MISE treatment
SE(x) : Fraction of heteroduplexes surviving MISE treatment

STEP 1: Selection.

In this step the initial library is contacted with target. Unbound templates are washed off and bound templates are recovered. The templates will be recovered according to the binding efficiency of their displayed ligand to the target.

$$N(p,1) = N(p,s) * R(p,s)$$

STEP 2: Calculation of diversity after selection.

The selection step can reduce diversity of the library if for some of the background binders less than 1 molecule survives selection.
If N(p,1) is less than D(p,s) then N(p,1) is set equal to D(p,s) [loss of diversity]

STEP 3: PCR Amplification of selection output.

By addition of a known amount of primers in the PCR amplification, the total amount of molecules in the library after amplification can be normalized to a specific amount [A(t,1)]
After amplification $N(t,1)_{[post\text{-}amplification]}$ equals A(t,1)
And $N(p,1)_{[post\text{-}amplification]}$ equals $N(p,1)_{[pre\text{-}amplification]} * A(t,1) / N(t,1)_{[pre\text{-}amplification]}$

STEP 4: Homo- and hetero-duplex separation.

The resulting duplexes are denatured by heating and the individual strands are reannealed in a random fashion. Thus the re-formation of duplexes takes place stochastically.
The frequency of a species in the total library is calculated by

$$F(p,1) = N(p,1) / N(t,1)$$

Since all species in a pool behave in the same fashion in this simulation, the frequency is equal for all species in a pool.
The number of homoduplexes formed in pool [p] is

$$O(p,1) = [N(t,1) * (F(p,1))\char`^2 * D(p,1) ] / 2$$

The number of heteroduplexes involving strands from pool [p] is

$$E(p,1) = 2 * F(p,1) * (1-F(p,1)) * D(p,1) * N(t,1)$$

STEP 5: Homo- and hetero-duplex separation.

All duplexes (homo- and heteroduplexes) are reacted with a mismatch-specific enzyme that specifically degrades heteroduplexes. It is expected that a small fraction of homoduplexes is also degraded by the mismatch-specific enzyme.
The number of surviving homoduplexes in pool p is

$$O(p,1) = SO\ (1)$$

If O(p,1) is then less than zero O(p,1) is set to zero [less than one molecule exists]

The number of surviving heteroduplexes in pool p is

$$E(p,1) = SE\ (1)$$

If O(p,1) is then less than zero O(p,1) is set to zero

STEP 6: Amplification

The total library is normalized to N(t,2) corresponding to a PCR step.

Fraction in pool p of number of total molecules:

*Calculated example A*

A model library composed of 5 pools (b-f) that contain species [pool b contains 16 species, pool c contains 8 species etc.] that bind target specifically and can be enriched from 0,1 fold (pool f) to 6,25e-3 fold (pool b). Pool a contains 1e8 species (minus the sum of the other pools) that bind unspecifically and therefore are depleted relatively from the library during selection: R(a) = 1e-7. In this case the selection step reduces the number of molecules in pool a from approximately 1e11 to 1e11*R(a) = 1 e4 and the number of molecules in pool f from 1000 to 100.

N(t,x) = 1e11 : Total number of molecules in the library in step x
A(t,x) = 1e11 : Total number of molecules formed in PCR amplification in step x
D(t,x) = 1e8 : Diversity (number of different molecules in library) in step x
D(a,1) = 1e8, D(b,1) = 16, D(c,1) = 8, D(d,1) = 4, D(e,1) = 2, D (f,1) = 1
R(a) = 1e-7, R(b) = 6,25e-3, R(c) = 1,25e-2, R(d) = 2,5e-2, R(e) = 5e-2, R (f) = 1e-1
SO(x) = 0,8 : Fraction of homoduplexes surviving MISE treatment
SE(x) = 1e-4 : Fraction of heteroduplexes surviving MISE treatment

Table 1.1. Fraction of molecules in pool p in step x [N(p,x) divided by N(t,x)] after each amplification step. (Numbers in percent).

| | | | | | | | | |
|---|---|---|---|---|---|---|---|---|
| Step (x) | | Pool (p) | | | | | | |
| | | a | b | c | d | e | f | $\Sigma_{b-f}$ |
| 1 | | 99,999969 | 0.000016 | 0,000008 | 0,000004 | 0,000002 | 0,000001 | 0,000031 |
| 2 | | 95,238095 | 0,952381 | 0,952381 | 0,952381 | 0,952381 | 0,952381 | 4,761905 |
| 3 | | 63,670137 | 1,558650 | 2,656209 | 4,851337 | 9,241583 | 18,022084 | 36,329863 |
| 4 | | 0,529398 | 0,050570 | 0,251337 | 1,591524 | 11,378731 | 86,198439 | 99,470602 |
| 5 | | 0,000177 | 0,000019 | 0,000189 | 0,008976 | 0,867189 | 99,123449 | 99,999823 |
| 6 | | 0,000000 | 0,000000 | 0,000000 | 0,000002 | 0,004046 | 99,995951 | 100,000000 |

As can be seen the library consisting of > 95 % background binders (pool a) after selection (step 2) consists of only 0,5 % background binders and 99 % binders (the sum of pools b-f [$\Sigma_{b-f}$]) after selection and 2 rounds of MISE (steps 3 and 4) using the described conditions.

*Calculated example B (lower enrichment factor)*

A model library composed of 5 pools (b-f) that contain species [pool b contains 16 species, pool c contains 8 species etc.] that bind target specifically and can be enriched from 0,01 fold (pool f) to 6,25e-4 fold (pool b). Pool a contains 1 e8 species (minus the sum of the other pools) that bind unspecifically and therefore are depleted relatively from the library during selection: R(a) = 1e-7. In this case the selection step reduces the number of molecules in pool a from approximately 1e11 to 1e11*R(a) = 1e4 and the number of molecules in pool f from 1000 to 10.

N(t,x) =1e11 : Total number of molecules in the library in step x
A(t,x) = 1e11 : Total number of molecules formed in PCR amplification in step x
D(t,x) = 1 e8 : Diversity (number of different molecules in library) in step x
D(a,1) = 1e8, D(b,1) = 16, D(c,1) = 8, D(d,1) = 4, D(e,1) = 2, D (f,1) = 1
R(a) = 1e-7, R(b) = 6,25e-4, R(c) = 1,25e-3, R(d) = 2,5e-3, R(e) = 5e-3, R (f) = 1e-2
SO(x) = 0,8 : Fraction of homoduplexes surviving MISE treatment
SE(x) =1e-4 : Fraction of heteroduplexes surviving MISE treatment

Table 1.2. Fraction of molecules in pool p in step x [N(p,x) divided by N(t,x)] after each amplification step. (Numbers in percent).

| | | | | | | | | |
|---|---|---|---|---|---|---|---|---|
| Step (x) | | Pool (p) | | | | | | |
| | | a | b | c | d | e | f | $\Sigma_{b-f}$ |
| 1 | | 99,999969 | 0,000016 | 0,000008, | 0,000004 | 0,000002 | 0,000001 | 0,000031 |
| 2 | | 99,502488 | 0,099502 | 0,099502 | 0,099502 | 0,099502 | 0,099502 | 0,497512 |
| 3 | | 99,088563 | 0,099085 | 0,106137 | 0,141386 | 0,211904 | 0,352926 | 0,911437 |
| 4 | | 95,128061 | 0,095119 | 0,111690 | 0,234614 | 0,763023 | 3,667493 | 4,871939 |
| 5 | | 19,132181 | 0,019130 | 0,025357 | 0,114356 | 1,807232 | 78,901744 | 80,867819 |
| 6 | | 0,008267 | 0,000008 | 0,000011 | 0,000098 | 0,026939 | 99,964675 | 99,991733 |

As can be seen the library consisting of > 99,5 % background binders (pool a) after selection (step 2) consists of only 19 % background binders and > 80 % binders (the sum of pools b-f [$\Sigma_{b-f}$]) after selection and 3 rounds of MISE (steps 3,4 and 5) using the described conditions.

*Calculated example C (Effect of more efficient mismatch cleavage)*

A model library composed of 5 pools (b-f) that contain species [pool b contains 16 species, pool c contains 8 species etc.] that bind target specifically and can be enriched from 0,1 fold (pool f) to 6,25e-3 fold (pool b). Pool a contains 1e8 species (minus the sum of the other pools) that bind unspecifically and therefore are depleted relatively from the library during selection: R(a) = 1e-7. In this case the selection step reduces the number of molecules in pool a from approximately 1e11 to 1e11*R(a) = 1e4 and the number of molecules in pool f from 1000 to 100.

N(t,x) = 1e11 : Total number of molecules in the library in step x
A(t,x) = 1e11 : Total number of molecules formed in PCR amplification in step x
D(t,x) = 1 e8 : Diversity (number of different molecules in library) in step x
D(a,1) = 1e8, D(b,1) = 16, D(c,1) = 8, D(d,1) = 4, D(e,1) = 2, D (f,1) = 1
R(a) = 1 e-7, R(b) = 6,25e-3, R(c) = 1,25e-2, R(d) = 2,5e-2, R(e) = 5e-2, R (f) = 1e-1
SO(x) = 0,8 : Fraction of homoduplexes surviving MISE treatment
SE(x) = 1e-5 : Fraction of heteroduplexes surviving MISE treatment

Table 1.3. Fraction of molecules in pool p in step x [N(p,x) divided by N(t,x)] after each amplification step. (Numbers in percent).

| | | | | | | | | |
|---|---|---|---|---|---|---|---|---|
| Step (x) | | Pool (p) | | | | | | |
| | | a | b c | | d | e | f | $\Sigma_{b\text{-}f}$ |
| 1 | | 99,999969 | 0.000016 | 0,000008 | 0,000004 | 0,000002 | 0,000001 | 0,000031 |
| 2 | | 95,238095 | 0,952381 | 0,952381 | 0,952381 | 0,952381 | 0,952381 | 4,761905 |
| 3 | | 39,292097 | 2,026837 | 3,971975 | 7,862268 | 15,642835 | 31,203988 | 60,707903 |
| 4 | | 0,022674 | 0,023500 | 0,177898 | 1,388845 | 10,985145 | 87,401938 | 99,977326 |
| 5 | | 0,000001 | 0,000001 | 0,000057 | 0,006308 | 0,783933 | 99,209701 | 99,999999 |
| 6 | | 0,000000 | 0,000000 | 0,000000 | 0,000000 | 0,003142 | 99,996858 | 100,000000 |

As can be seen the library consisting of > 95 % background binders (pool a) after selection (step 2) consists of only < 40 % background binders and > 60 % binders (the sum of pools b-f [$\Sigma_{b\text{-}f}$]) after selection and 1 round of MISE (step 3) using the described conditions.

These theoretical examples show that one can extract specific oligonucleotides from a diverse pool based on formation of homo- and hetero-duplexes. These examples also illustrate that parameters such as diversity, enrichment factor, library size and degree of separation of homo-and hetero-duplexes will influence the outcome of the mismatch selection treatment.

EXAMPLE 2. *General experimental procedure and material for single codon oligonucleotides*

Double stranded DNA species are formed by extension of oligonucleotide primer F on single stranded templates $N_0$ and $N_{12}$ using Sequenase 2.0 (Amersham Biosciences) and buffer.

Heat-denaturation and annealing of samples:

A sample of either double stranded $N_0$ or double stranded $N_{12}$ or a mixture of these in 5 μl of water containing 40 mM HEPES pH 7.5, 50 mM NaCl, 16 mM $MgCl_2$ was heated to 95°C for 10 minutes and allowed to anneal by lowering the temperature.

If only double stranded $N_0$ is present in the sample, this treatment is expected to result in the re-formation of homoduplexes.

If only double stranded $N_{12}$ (4^12 = 1,7*$10^7$ different species) is present in the sample, this treatment is expected to result in the re-formation of homoduplexes and the formation of heteroduplexes.

If both double stranded $N_0$ and double stranded $N_{12}$ is present in the sample, this treatment is expected to result in the re formation of $N_0$ and $N_{12}$ homoduplexes and the formation of $N_{12}$-$N_{12}$ and $N_0$-$N_{12}$ heteroduplexes (containing 1 or more mismatched base pairs).

Treatment of samples with mismatch-specific enzyme:

To a sample consisting of either double stranded $N_0$ or double stranded $N_{12}$ or a mixture of these was added 1 μl (10x) Surveyor reaction buffer, 0.5 μl Enhancer, 1 μl Surveyor Nuclease, and 2.5 μl H2O. The samples were then mixed and incubated 1 hour at 42°C. Then the samples were heated to 95°C for 10 minutes and allowed to anneal by lowering the temperature. To 3 μl of each sample was added 1 μl 5xEXT buffer [100 mM HEPES pH 7.5, 750 mM NaCi, 40 mM $MgCl_2$], 4 μl $H_2O$, and 1 μl E. coli Exonuclease VII (10 U/μl) (USB). The samples were then incubated at 37°C for 16 hours.

QPCR (quantitative real-time polymerase chain reaction) analyses to estimate number of molecules of each species before and after treatment. The QPCR was performed using a Taqman probe which is a fluorescence resonance energy transfer (FRET) probe consisting of a short oligonucleotide complementary to one of the amplified strands. The probe contains a fluorofor and a quencher molecule at the 5' and 3' end of the probe, respectively. This probe is included in the real-time PCR reaction along with the required forward and reverse PCR primers. The quencher molecule quenches the fluorescence of the fluorofor due to its close proximity on the probe. As the Taq polymerase replicates the new strand

of the DNA, its 5'-3' exonuclease activity degrades the FRET probe from the 5'-end. This degradation releases the reporter flurofor from its proximity to the quencher, resulting in fluorescence of the reporter. Accumulation of fluorescence as a result of target amplification was detected in real time in an ABI 7900 HT sequence detection system (Applied Biosystems) which contains an optical detection systems During the exponential amplification phase, the amount of target should be doubling every cycle. Quantification analyses use the $C_T$ value, which is the point (cycle number) at which the fluorescence signal reaches a specific threshold level of detection in the exponential phase. The more abundant the template, the earlier this point is reached. The quantity of DNA in the sample can be obtained by interpolation of its $C_T$ value vs. a linear standard curve of $C_T$ values obtained from a serially diluted standard solution.

Q-PCR reactions

For 5 ml premix (for one 96-well plate):

2.5 ml Taqman Universal PCR Master Mix (Applied Biosystems)
450 μl RPv2 (10 pmol/μl)
25 μl Taqman probe (50 μM)
1075 μl $H_2O$
40.5 μl premix was aliquoted into each well and 4.5 μl of relevant upstream PCR primer (Primer F for standard curve and $N_{12}$) or the $N_0$ specific primers QP1-3 and 5 μl sample ($H_2O$ in wells for negative controls) was added.

The samples for the standard curve was prepared by diluting Temp4 to $10^8$ copies/5 μl and subsequently performing a 10-fold serial dilution of this sample. 5 μl was used for each Q-PCR reaction.

Thermocycling/measurement of fluoresence was performed on an Applied Biosystems ABI Prism 7900HT real-time instrument utilizing the cycling parameters:

95°C 10 min
40 cycles of
95°C 15 sec
64°C 1 min

Oligonucleotides

$N_{12}$ oligo:
5'-GTCAGAGACGTGGTGGAGGAAGTCTTCCTAGAAGCTGGA
NNNNNNNNNNNNTCTAGCAGCTAGTATGACGTGGTGTCCAAGCTG-3'

$N_0$ oligo:
5'-GCTAGAGACGTGGTGGAGGAAGTCTTCCTAGAAGCTGGA
TATCTTCAGTTCTCGACTCCTGAGTATGACGTGGTGTCCAAGCTG-3'

Primer F:
5'- CAGCTTGGACACCACGTCATAC -3'

Primer R:
5'- GTCAGAGACGTGGTGGAGGAA-3'

QP1-3:
5'-TCATACTCAGGAGTCGAGAACTGAAGATA-3'

Temp4:

**5'-**
**GCTAGAGACGTGGTGGAGGAAGTCTTCCTAGAAGCTGGATATCTGACG**
**TGTTGAC**
**GTACACAGTATGACGTGGTGTCCAAGCTG-3'**

TaqMan probe:

5'-6FAM-TCCAGCTTCTAGGAAGAC-MGB-NFQ (Applied Biosystems) 6FAM : 6-Carboxyfluorescein
MGB : Minor groove binder
NFQ : Non-fluorescent quencher

*EXAMPLE 3: Discrimination between homo- and heteroduplexes in an identifier oligonucleotide containing a codon.*

This example shows the possibility to specifically remove/degrade/separate heteroduplexes from homoduplexes. This is illustrates using a mismatch cleavage enzyme but other techniques such as for example gel separation, mismatch binding and column separation can also be used.

Two samples were subjected to the experimental procedure described in Example 2:

Sample 2A: 1 pmol double stranded $N_0$ in 5 μl of water containing 40 mM HEPES pH 7.5, 50 mM NaCi, 16 mM $MgCl_2$
Sample 2B: 1 pmol double stranded $N_{12}$ in 5 μl of water containing 40 mM HEPES pH 7.5, 50 mM NaCl, 16 mM $MgCl_2$

Results of QPCR analyses of sample 2A:

Number of homoduplexes before MISE treatment: 6, 71 E+08 (6, 00E+08 expected)
Number of homoduplexes after MISE treatment: 4, 54E+08
Thus (4, 54 / 6, 71) = 67 % of homoduplexes have survived the MISE treatment

Results of QPCR analyses of sample 2B:

Number of heteroduplexes before MISE treatment: 7, 35E+08 (6,00E+08expected)
Number of heteroduplexes after MISE treatment: 1, 16E+07

Thus (1, 16 / 73, 5) = 1, 5 % of heteroduplexes have survived the MISE treatment
That is the relative survival factor of homoduplexes is (67 / 1, 5) = 45

The relative survival factor obtain in this example is dependent on the experimental conditions. This can be future optimized if required by tuning the mismatch treatment conditions. The relative survival factor could also be different using other techniques in specifically remove/degrade/separate heteroduplexes from homoduplexes, as for example gel separation, mismatch binding and column separation.

*EXAMPLE 4: Enrichment of sequences in excess over a background sequence population.*

This example describes the possibility to enrich a specific oligonucleotide among a diverse background of sequences.
Samples were subjected to the experimental procedure described in Example 2:

A mixed sample containing 1 pmol sample $N_{12}$ ($4^{12} = 1,7*10^7$ different species of double stranded DNA) and 0,001 pmol of sample $N_0$ in 5 μl of water containing 40 mM HEPES pH 7.5, 50 mM NaCl, 16 mM $MgCl_2$ was subjected to general procedure 2. Then samples were diluted 300 times and analyzed by QPCR (input) and the result from one mismatch selection treatment (output) as described in Example 2

QPCR analysis result:

| Sample | # $N_{12}$ | # $N_0$ | Fold excess $N_{12}$ |
|---|---|---|---|
| Input | 5,11E+08 | 1,27E+05 | 4040 |
| Output | 3,80E+03 | 6,58E+01 | 58 |

The enrichment factor with the mismatch selection procedure is 70 (4040/58) in this experiment.

This example demonstrates the possibility to enrich for a specific oligonucleotide sequence ($N_0$) among a diverse pool of oligonucleotide sequences ($N_{12}$). The specific sequence ($N_0$) is in 17000-fold excess over each specific $N_{12}$ sequences but in 1000-fold (4040 experimentally) lower concentration compared to the entire population of $N_{12}$ sequences. Thus, although the background sequences are in excess, the specific sequence is able to survive the mismatch selection treatment.

*EXAMPLE 5: Sequential mismatch selection treatment*

One important feature with mismatch selection is that one can perform multiple round of treatment. This will permit extreme enrichment factors which might be important when the library size is much larger than the enrichment factor obtain in the initial selection.

A mixed sample containing 1 pmol sample $N_{12}$ (4^12 = 1,7*$10^7$ different species of double stranded DNA) and 0,0001 pmol of sample $N_0$ in 5 $\mu$l of water containing 40 mM HEPES pH 7.5, 50 mM NaCl, 16 mM $MgCl_2$ was subjected to condition described in Example 2.

Following the procedure described in Example 2, the samples were run on a denaturing 10 % polyacrylamide gel and a gel slice bands containing full-length templates ($N_0$ or $N_{12}$) [estimated using 32P-labeled marker oligos of the same length as $N_0$ and $N_{12}$ were excised from the gel and placed in an eppendorph tube. 100 $\mu$l of 1 x EXT buffer [40 mM HEPES pH 7.5, 50 mM NaCl, 16 mM $MgCl_2$] was added. DNA was liberated from the gel by freeze-thawing using 2 cycles of heating to 99˚C and cooling to -20˚C. 400 $\mu$l $H_2O$ was added and 5 $\mu$l DNA mixture was used for PCR amplification with 5 pmol each of primers F and R in a 25 $\mu$l reaction using Ready-To-Go beads (Amersham Biosciences).

Approximately 1 pmol of amplified material (containing $N_0$ and $N_{12}$) was subjected to another round of mismatch selection as described in Example 2, except that the Exo VII treatment step was excluded. Then the sample was diluted 300 times and analyzed by QPCR

QPCR analysis result:

| Sample | # $N_{12}$ | # $N_0$ | Fold excess $N_{12}$ |
|---|---|---|---|
| Input | 1,54E+09 | 3,42E+04 | 45098 |
| Output 1$^{st}$ round of general procedure 2 | 1,30E+04 | 1,99E+02 | 66 |
| Output 2$^{nd}$ round of general procedure 2 | 3,53E+03 | 7,28E+02 | 5 |

The enrichment factor in the 1$^{st}$ round of mismatch selection treatment described in Example 2 is 45098 / 66 = 683 The enrichment factor in the 2$^{nd}$ round of mismatch selection treatment described in Example 2 (excluding the Exo VII treatment) is 66 / 5 = 13

The total enrichment factor from these two mismatch selection treatment is 8879 (683*13).

This example shows two rounds of mismatch selection treatment but this treatment can be continued until desired result is obtained.

This enrichment factor obtained with the mismatch selection treatment can then be multiplied with the initial enrichment factor obtained with the standard selection procedure to obtain the overall enrichment factor for the complete selection process.

EXAMPLE 6. *General experimental procedure and material for multiple codon oligonucleotides*

Double stranded DNA species are formed by extension of oligonucleotide primer ER on single stranded templates $EN_0$, $EN_6$ and $EN_{12}$ using Sequenase 2.0 (Amersham Biosciences) and buffer.

Heat-denaturation and annealing of samples:

A mixture of double stranded $EN_0$, double stranded $EN_6$ and double stranded $EN_{12}$ in 5 $\mu$l of water containing 40

mM HEPES pH 7.5, 50 mM NaCl, 16 mM $MgCl_2$ was heated to 95˚C for 10 minutes and allowed to anneal by lowering the temperature.

This treatment is expected to result in the re-formation of $EN_0$-$EN_0$, $EN_6$-$EN_6$, and $EN_{12}$-$EN_{12}$ homoduplexes and the formation of $EN_0$-$EN_6$, $EN_0$-$EN_{12}$, $EN_6$-$EN_6$, $EN_6$-$EN_{12}$ and $EN_{12}$-$EN_{12}$ heteroduplexes (containing 1 or more mismatched base pairs).

Treatment of samples with mismatch-specific enzyme:

To the 5 µl mixture was added 1 µl (10x) Surveyor reaction buffer, 0.5 µl Enhancer, 1 µl Surveyor Nuclease, and 2.5 µl H2O. The samples were then mixed and incubated 1 hour at 42˚C. Then the sample were heated to 95˚C for 10 minutes and allowed to anneal by lowering the temperature. To 3 µl of each sample was added 1 µl 5xEXT buffer [100 mM HEPES pH 7.5, 750 mM NaCl, 40 mM $MgCl_2$], 4 µl $H_2O$, and 1 µl E. coli Exonuclease VII (10 U/µl) (USB). The samples were then incubated at 37˚C for 16 hours.

Then the samples were run on a denaturing 10 % polyacrylamide gel and a gel slice bands containing full-length templates ($EN_0$, $EN_6$ and $EN_{12}$) [estimated using 32P-labeled marker oligos of the same length as the templates) were excised from the gel and placed in an eppendorph tube. 100µ l of 1 x EXT buffer [40 mM HEPES pH 7.5, 50 mM NaCl, 16 mM $MgCl_2$] was added. DNA was liberated from the gel by freeze-thawing using 2 cycles of heating to 99˚C and cooling to -20˚C. 400 µl $H_2O$ was added and 5 µl DNA mixture was used for PCR amplification with 5 pmol each of primers EF and ER in a 25 µl reaction using Ready-To-Go beads (Amersham Biosciences).

A TOPO-TA (Invitrogen) ligation reaction was assembled with 4 µl PCR product, 1 µl salt solution (Invitrogen), and 1 µl vector. Water was added to 6 µl. The reaction was incubated at RT for 30 min. Heat-shock competent TOP10 E.coli cells were thawed on ice. 5 µl ligation reaction was added to the thawed cells and these were incubated 30 min on ice, heatshocked in 42˚C water for 30 sec, and then put on ice. 250 µl of growth medium was added and the mixture was incubated 1 h at 37oC. The mixture was then spread on a growth plate containing 100 µg / ml ampicillin and incubate ON at 37oC. Individual *E.coli* clones were picked and transferred to PCR wells containing 50 µl water. These 50 µl were incubated at 94˚C for 5 minutes and used in a 20 µl in a 25 µl PCR reaction with 5 pmol of each TOPO primer M13 forward & M13 reverse and Ready-To-Go PCR beads (Amersham Biosciences). The following PCR profile was used: 94oC 2 min, then 30 x (94oC 4 sec, 50oC 30 sec, 72oC 1 min) then 72oC 10 min. Primers and nucleotides were degraded by adding 1 µl 1:1 EXO/SAP mixture (USB corp.) to 2 µl PCR product and incubating at 37˚C for 15 min and then 80˚C for 15 min to heat-inactivate the enzymes. 5 pmol T7 primer was added and water was added to 12 µl. Add 8 µl DYEnamic ET cycle sequencing Terminator Mix (Applied biosystems). A thermocycling profile of 30 x (95oC 20 sec, 50oC 15 sec, 60oC 1 min) was run. Then 5 µl water was added to each well and sequencing reactions were purified using seq96 spinplates (Amersham Biosciences). Reactions were run on a MegaBace capillary electrophoresis instrument (Molecular Dynamics) using injection parameters 2 kV, 50 sec and run parameters: 9 kV 45 min and analyzed using Contig Express software (Informax).

Oligonucleotides

**$EN_0$ oligo:**

**5'GTCGAATGCTGTAGCGGTAGGCAGCAATGACGTCGAATGACAGCAAA**
**TGAGTCGATGTGCTGAGCTAGAT-3'**

**$EN_6$ oligo:**

**5'-**
**GTCGAATGCTGTAGCGGTAGGCAGCANNGACGTCGNANGACAGCANAT**
**NAGTC**
**GATGTGCTGAGCTAGAT -3'**

**$EN_{12}$ oligo:**

**5'-**

**GTCGAATGCTGTAGCGGTAGGCAGCNNTNNCGTCGANNNNCAGCANNN GNGTC**

**GATGTGCTGAGCTAGAT-3'**

EF oligo:
5'-GTCGAATGCTGTAGCGGTAG
ER oligo:
5'-ATCTAGCTCAGCACATCGAC
M13 forward:
5'-GTAAAACGACGGCCAG
M 13 reverse:
5'-CAGGAAACAGCTATGAC
T7 primer:
5'-TAATACGACTCACTATAGGG

*EXAMPLE 7: Mismatch selection treatment and composition of oligonucleotide containing multiple codons.*

Libraries of bifunctional complexes will preferably contain more than one codon allowing encoding of multiple functional entities in the displayed molecule. This example describes an identifier containing three variable regions that represent three individual encoding codons. In the homo- hetero-duplex separation step in the mismatch cleavage procedure multiple short fragments are produced that could potentially recombine through overlapping sequences and generated shuffled variants. These shuffled identifiers would then contain codons originated from different original identifiers. This is tested in this example.

Selected libraries with identifier oligos containing 3 codons was modelled using templates $EN_0$, $EN_6$, and $EN_{12}$. It is expected that a library initially containing 4^12 = 1,6e7 different species will contain an enriched best binder $EN_0$ and a pool of not-best binders $EN_6$. The enriched pool of not-best is expected to be diverse (in this case the $EN_6$ pool contains 4^6 = 4096 species but not as diverse as the background of non-specific binders ($EN_{12}$ contains 1,6e7 different species).

Two samples were mixed to model selected libraries:

Sample 3.1 A

1 pmol $EN_{12}$, 0.1 pmol $EN_6$, 0.1 pmol $EN_0$. This corresponds to a 1,6e7 member library that has been selected so that the best binder ($EN_0$) has been enriched 1,6e6 fold and the not-best binders ($EN_6$) have been enriched 390 fold (1,6e6 / 4096).

Sample 3.1 B

1 pmol $EN_{12}$, 0.01 pmol $EN_6$, 0.01 pmol $EN_0$. This corresponds to a 1,6e7 member library that has been selected so that the best binder ($EN_0$) has been enriched 1,6e5 fold and the not-best binders ($EN_6$) have been enriched 39 fold (1,6e5 / 4096).

The samples were subjected mismatch selection as described in Example 6.

Sample 3.1A

| | $EN_{12}$ | $EN_6$ | $EN_0$ |
|---|---|---|---|
| Input (sequencing) | 11 | 2 | 2 |
| Output (sequencing) | 5 | 0 | 6 |

This corresponds to an $EN_0$ enrichment fold of (6/11)/(2/(2+2+11)) = 4

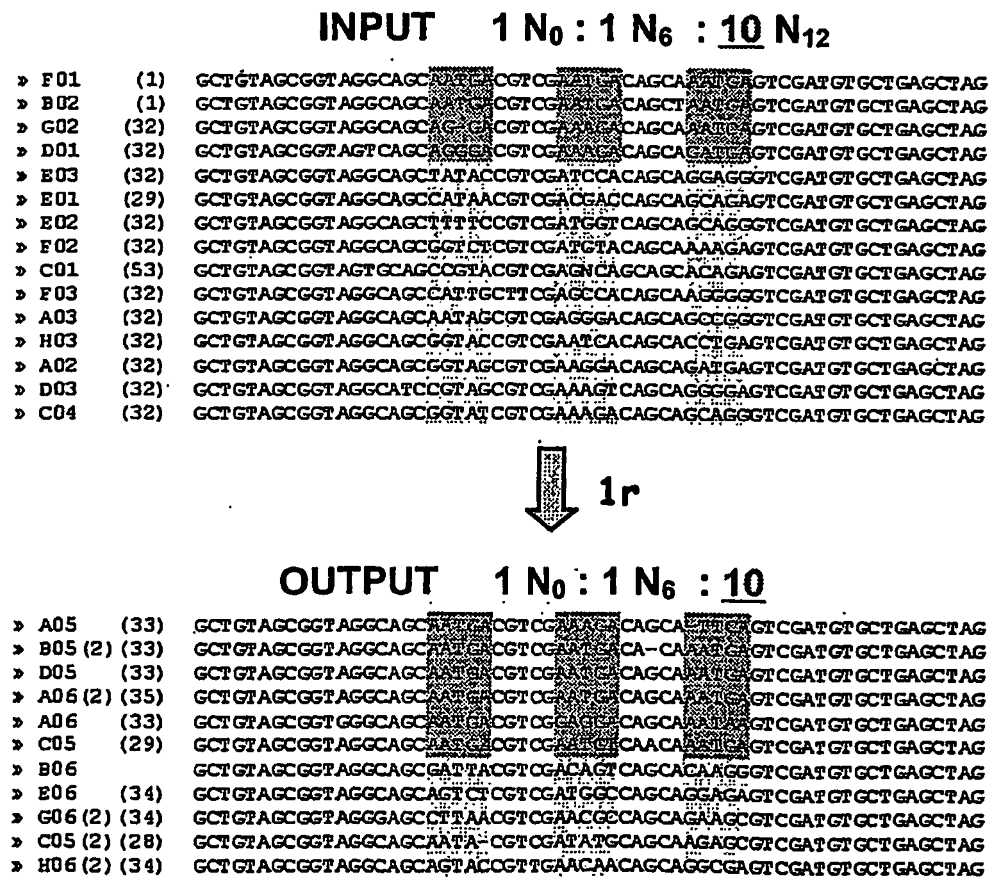


Sample 3.1B

| | $EN_{12}$ | $EN_6$ | $EN_0$ |
|---|---|---|---|
| Input (theoretical) | 100 | 1 | 1 |
| Output (sequencing) | | 6 3 | 1 |

This corresponds to an $EN_0$ enrichment fold of (1/(6+3+1))/(1/100) = 10 and an $EN_6$ enrichment fold of (3/(6+3+1))/(1/100) = 30

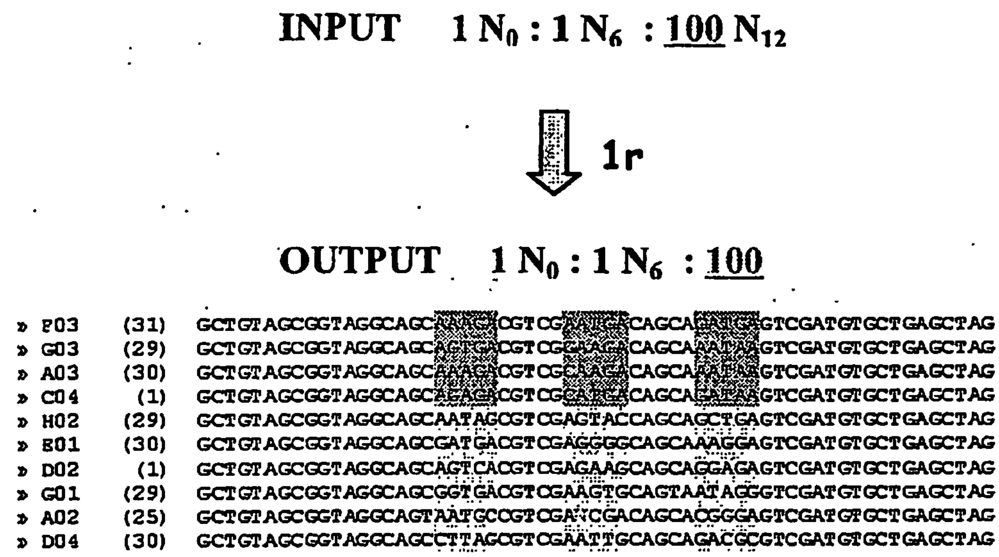


These two results shows that enrichment of the expected sequences ($N_0$, $N_6$) are identified by sequencing and the composition of the identifier sequences are kept intact. There is no shuffling between the codons from the $N_0$, $N_6$, $N_{12}$ oligonucleotides.

*Example 8 : Identification of a ligand from a library composed of 61875 different small molecules each associated with a unique identifer oligonucleotide by selection and subsequently using mismatch selection (MISE).*

General arrangement of each complex composed of display molecule and identifier oligonucleotide in the library generation:

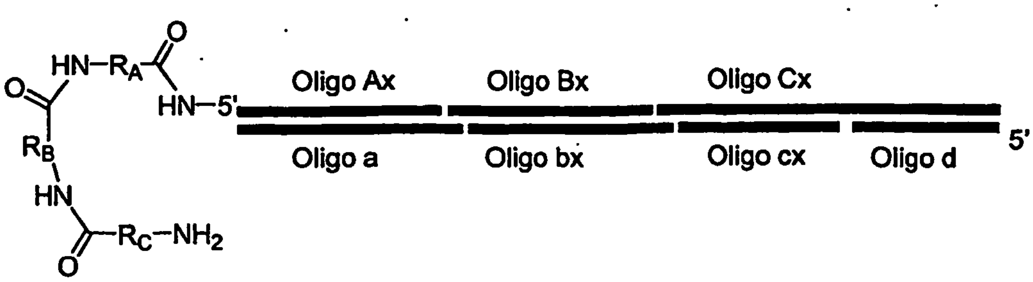


*Overview of the library generation procedure:*

First round of library generation (round A) :

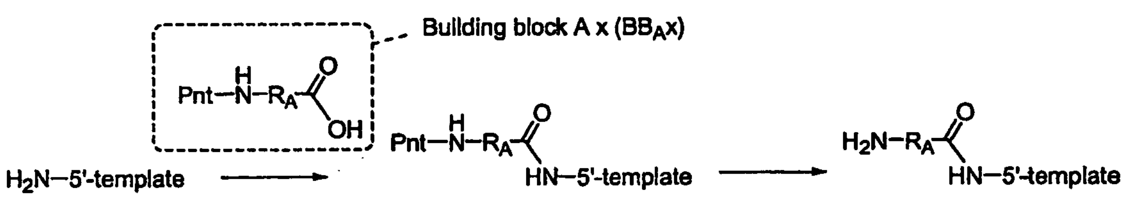


"Pnt" corresponds to pentenoyl - an amine protecting group. "R" can by any molecule fragment. The chemical

used in library generation comprise a primary (shown) or a secondary amine.

Second round of library generation (round B) :

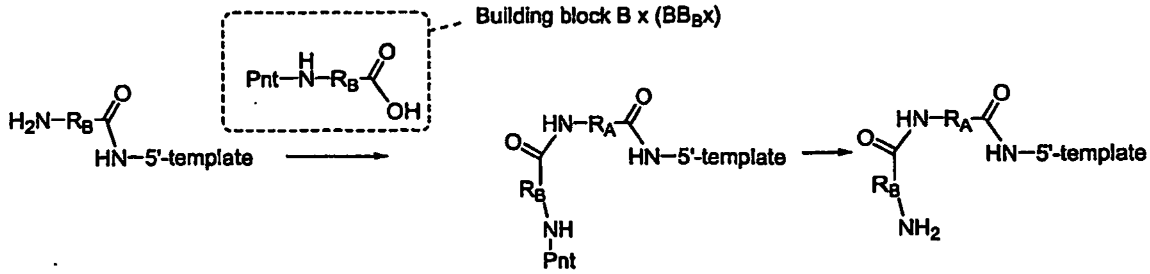


Third round of library generation (round C):

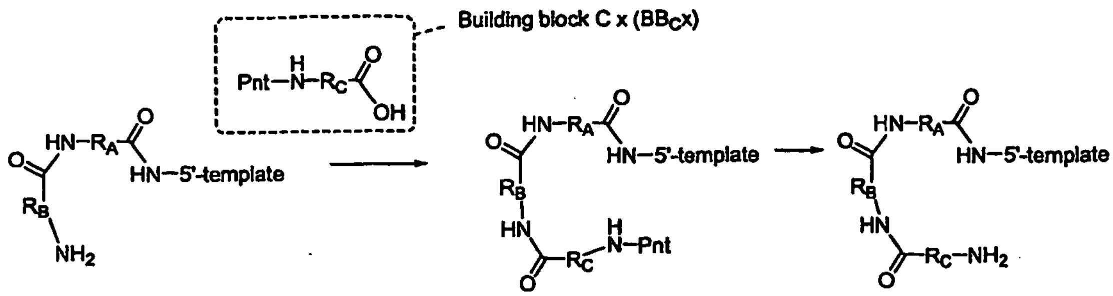


*General procedure : Library generation, selection and mismatch subsequent selection*

*First round of library generation (round A) :*

First oligonucleotides of the A series are each modified by adding to each type of oligo a small molecule building block ($BB_Ax$) to the 5' amine forming an amide bond. After this step the template is comprised of oligo Ax.

*Second round of library generation (round B) :*

4 nmol of a mixture of different modified A oligos are then split into a number tubes corresponding to the number of different building blocks to be used in round B. 190 pmol Oligo a and 2 μl heering DNA is added to each tube and the DNA material in each tube is lyophilized. The lyophilized DNA is then redissolved in 50 μl water and purified by spining through Biospin P-6 columns (Biorad) equilibrated with water.

*Addition of building block:*

The DNA material in each tube is again lyophilized and redissolved in 2 μl 100 mM Na-borate pH 8.0/100 mM sulfo N-hydroxy succinimide (sNHS). For each tube 10 μl building block $BB_Bx$ (100 mM in dimethyl sulfoxide [DMSO]) is preactivated by mixing with 10 μl 1-Ethyl-3-(3-dimethylaminopropyl)-carbodiimide (EDC) (90 mM in dimethylformamide [DMF]) and incubating at 30°C for 30 min. 3 μl of this preactivated mixture is then mixed with the 2 μl in each tube and allowed to react 45 min at 30 °C. Then an additional 3 μl freshly preactivated BB is added and the reaction is allowed to proceed for 45 min at 30 °C. The resulting mixture is then purified by spinning through Bio-Rad P6 DG (Desalting gel).

*Addition of codon oligonucleotide :*

The DNA material is then lyophilized and redissolved in 10 μl water containing 200 pmol oligo Bx (eg. B1) and the corresponding oligo bx (eg. b1). This is done so that the codon in oligo Bx identifies the $BB_Bx$ added to the DNA template. 10 units of T4 DNA ligase (Promega) and 1.2 μl T4 DNA ligase buffer is then added to each tube and the mixture is incubated at 20°C for 1 hour. The DNA template linked to the small molecules now comprises an Ax oligo with a Bx oligo ligated to its 3' end. The reactions are then pooled, an appropiate volume of water is allowed to evaporate and the remaining sample is purified by spining through Biospin P-6 columns (Biorad) equilibrated with water.

*Removal of building block protecting group:*

The pooled sample (~ 50 μl) is adjusted to 10 mM Na-acetate (pH 5). 0.25 volumes of 25 mM Iodine in tetrahydrofuran/water (1:1) is added and the sample is incubate at 37 °C for 2h. The reaction is then quenched by addition of 2 μl of 1 M $Na_2S_2O_3$ and incubation at room temperature for 5 min. The complexes are then purified by spining through Biospin P-6 columns (Biorad) equilibrated with water

To remove sulphonamide protecting groups, the sample is adjusted to 50 μl 100 mM sodium borate pH 8.5 and 20 μl 500 mM 4-methoxy thiophenol (in acetonitrile) is added and the reaction is incubated at 25°C overnight. Then the complexes are purified by spinning through Biospin P-6 columns (Biorad) equilibrated with water and then lyophilized.

<u>*Third round of library generation (round C) :*</u>

The samples are dissolved in 175 μl 100 mM Na-borate pH 8.0 and distributed into 25 wells (7 μl / well). 2 μl 100 mM $BB_Cx$ in water/DMSO and 1 μl of 250 mM DMT-MM is added to each reaction and incubated at 30 °C ovemigth. Water is added to 50 μl and the reactions are then spin purified using Bio-Rad P6 DG (Desalting gel) and subsequently water is allowed to evaporate so that the final volume is 10 μl.

*Addition of building block:*

The DNA material is then lyophilized and redissolved in 10 μl water containing 200 pmol oligo Cx (eg. C1) and the corresponding oligo cx (eg. c1). This is done so that the codon in oligo Cx corresponds to the $BB_Cx$ added to the DNA template. 10 units of T4 DNA ligase (Promega) and 1.2 μl T4 DNA ligase buffer is then added to each tube and incubated at 20°C for 1 hour. The DNA template linked to the small molecules now comprises and Ax oligo with a Bx ligated to its 3' end and a Cx oligo ligated to the 3' end of the Bx oligo. The reactions are then pooled, the pooled sample volume is reduced by evaporation and the sample is purified by spining through Biospin P-6 columns (Biorad) equilibrated with water. The pooled sample (~ 50 μl) is adjusted to 10 mM Na-acetate (pH 5). 0.25 volumes of 25 mM Iodine in tetrahydrofuran/water (1:1) is added and the sample is incubate at 37 °C for 2h. The reaction is then quenched by addition of 2 μl of 1M $Na_2S_2O_3$ and incubation at RT for 5 min. Then the DNA templates (carrying small molecules) are purified by spinning through Biospin P-6 columns (Biorad) equilibrated with water and then lyophilized.

*Final deprotection step*

Some building blocks contain methyl esters that are deprotected to acids by dissolving the pooled sample in 5 μl 20 mM NaOH, heating to 80 °C for 10 minutes and adding 5 μl of 20 mM HCl.

*Final extension step*

To ensure that the DNA templates are double stranded prior to selection oligo d is extended along the template by adding to the sample 10 μl of 5 X sequenase EX-buffer [100 mM Hepes, pH 7.5, 50 mM $MgCl_2$, 750 mM NaCl] and 4000 pmol oligo d. Annealing is performed by heating to 80°C and cooling to 20 °C. To the sample is then added 500 μL dNTP , water to 50 μl and 39 units of Sequenase version 2.0 (USB). The reaction is incubated at 37°C for 1 hour.

<u>*Selection*</u>

This library is subjected to selection, whereby binders to the selection target are enriched.
Maxisorp ELISA wells (NUNC A/S, Denmark) were coated with each 100 μL 2μg/mL integrin αVβ3 in PBS buffer [2.8 mM $NaH_2PO_4$, 7.2 mM $Na_2HPO_4$, 0.15 M NaCl, pH 7.2] overnight at 4°C. Then the integrin solution was substituted for 200 μl blocking buffer [TBS, 0.05% Tween 20 (Sigma P-9416), 1% bovine serum alnumin (Sigma A-7030), 1 mM $MnCl_2$] which was left on for 3 hours at room temperature. Then the wells were washed 10 times with blocking buffer and the

encoded library was added to the wells after diluting it 100 times with blocking buffer. Following 2 hours incubation at room temperature the wells were washed 10 times with blocking buffer. After the final wash the wells were cleared of wash buffer and subsequently inverted and exposed to UV light at 300-350 nm for 30 seconds using a trans-illuminator set at 70% power. Then 100 μl blocking buffer without Tween-20 was immediately added to each well, the wells were shaken for 30 seconds, and the solutions containing eluted templates were removed for PCR amplification and then used for mismatch selection (MISE).

*Mismatch selection (MISE) :*

The double stranded sample is denatured by heating and allowed to cool whereby hetero- and homoduplexes are formed:

*Treatment of samples with nucleases:*

To the 5 μl mixture ia added 1 μl (10x) Surveyor reaction buffer (Transgenomic), 0.5 μl Enhancer (Transgenomic), 1 μl Surveyor Nuclease (Transgenomic), and 2.5 μl $H_2O$. The samples are then mixed and incubated 1 hour at 42˚C. Then the sample is heated to 95˚C for 10 minutes and duplexes are allowed to form by lowering the temperature. To 3 μl of each sample is added 1 μl 5 x EXT buffer [100 mM HEPES pH 7.5, 750 mM NaCl, 40 mM $MgCl_2$], 4 μl $H_2O$, and 1 μl E. coli Exonuclease VII (10 U/μl) (USB). The samples are then incubated at 37˚C for 16 hours.

*Purification by polyacrylamide gel electrophoresis:*

Then the samples are mixed with loading buffer and run on a denaturing 10 % polyacrylamide gel and gel slices containing full-length templates (estimated using 32P-labeled marker oligos of the same length as the templates) are excised from the gel and placed in an eppendorph tube. 100 μl of 1 x EXT buffer [40 mM HEPES pH 7.5, 50 mM NaCl, 16 mM $MgCl_2$] are added. DNA is liberated from the gel by freeze-thawing using 2 cycles of heating to 99˚C and cooling to -20˚C. 400 μl $H_2O$ is added and 5 μl DNA mixture is used for PCR amplification with 5 pmol each of forward and reverse primers and in a 25 μl reaction using Ready-To-Go beads (Amersham Biosciences).

*Cloning of MISE products*

A TOPO-TA (Invitrogen) ligation reaction is assembled with 4 μl PCR product, 1 μl salt solution (Invitrogen) and 1 μl vector. Water is added to 6 μl. The reaction is then incubated at RT for 30 min. Heat-shock competent TOP10 E.coli cells are then thawed on ice and 5 μl of the ligation reaction is added to the thawed cells. The cells are then incubated 30 min on ice, heatshocked in 42˚C water for 30 sec, and then put on ice again. 250 μl of growth medium is added to the cells and they are incubated 1 h at 37˚C. The medium containting cells is then spread on a growth plate containing 100 μg /ml ampicillin and incubated at 37˚C for 16 hours.

*Sequencing of MISE products:*

Individual *E.coli* clones are then picked and transferred to PCR wells containing 50 μl water. These 50 μl were incubated at 94˚C for 5 minutes and used in a 20 μl in a 25 μl PCR reaction with 5 pmol of each TOPO primer M13 forward & M13 reverse and Ready-To-Go PCR beads (Amersham Biosciences). The following PCR profile is used: 94˚C 2 min, then 30 x (94˚C 4 sec, 50˚C 30 sec, 72˚C 1 min) then 72˚C 10 min. Primers and nucleotides are then degraded by adding 1 μl 1:1 EXO/SAP mixture (USB corp.) to 2 μl PCR product and incubating at 37˚C for 15 min and then 80˚C for 15 min to heat-inactivate the enzymes. 5 pmol T7 primer is added and water is added to 12 μl. Then 8 μl DYEnamic ET cycle sequencing Terminator Mix (Applied biosystems) is added to each well. A thermocycling profile of 30 x (95˚C 20 sec, 50˚C 15 sec, 60˚C 1 min) is then run. Then 10 μl water is added to each well and sequencing reactions are purified using seq96 spinplates (Amersham Biosciences). Reactions are then run on a MegaBace capillary electrophoresis instrument (Molecular Dynamics) using injection parameters 2 kV, 50 sec and run parameters: 9 kV 45 min and analyzed using Contig Express software (Informax).

*Example 8.1 (general procedure 8): Generation of a library containing 61875 different small molecules and identification of a binder among these by subjecting the library to target selection and subsequent mismatch selection (MISE) :*

*First round of library generation (round A) :*

99 different A oligos were used :

Oligo Ax general structure :
5'- **NSP**-ACCTCAGCTGTGTATCGAGCGGCAGCTGTTCCGTCG-3'

The underlined part corresponds to the 5 nucleotide sequence that varies among different A oligos, ie. the codon. The remaining sequence is identical among the A oligos.

N : 5'-Amino-Modifier 5 (Glen research cat# 10-1905-90)
S : Spacer C3 CPG (Glen research cat# 20-2913-01)
P : PC Spacer Phosphoramidite (Glen research cat# 10-4913-90)
Oligo a: 5'- TGTGCGACGIIIIIGCTGCCGCTCGATACACAGCTGAGGT
I : inosine

Onto the free $NH_2$ of the 5' amino modifier on these oligos were loaded the listed $BB_A$x building blocks:

| Oligo | Codon | Building block | Building block structure |
|---|---|---|---|
| Oligo A1 | TGTTC | $BB_A1$ | 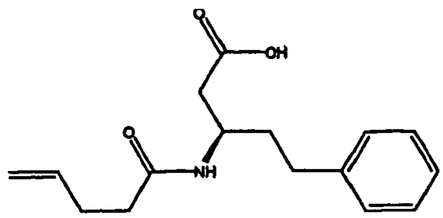 |
| Oligo A2 | CGAGC | $BB_A2$ | 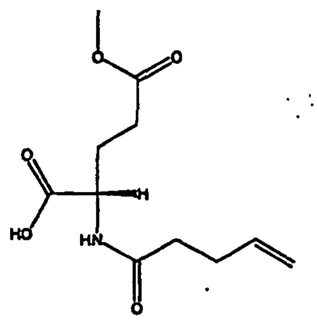 |
| Oligo A3 | GGATA | $BB_A3$ | 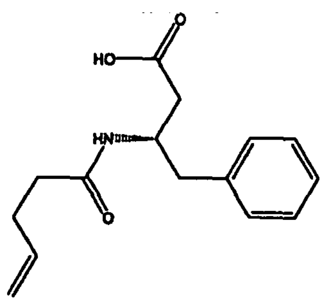 |
| Oligo A4 | CGCTG | $BB_A4$ | 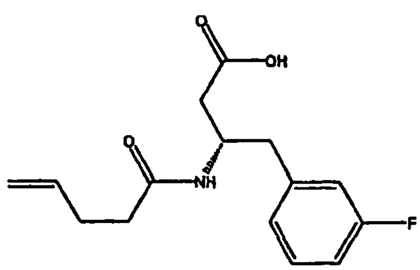 |

(continued)

| Oligo | Codon | Building block | Building block structure |
|---|---|---|---|
| Oligo A5 | GTTAT | $BB_A5$ | 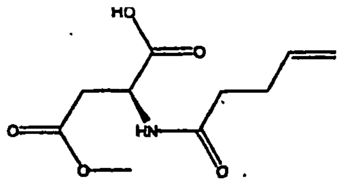 |
| Oligo A6 | AGTGC | $BB_A6$ | 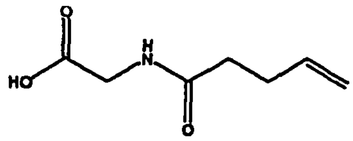 |
| Oligo A7 | ACCTG | $BB_A7$ | 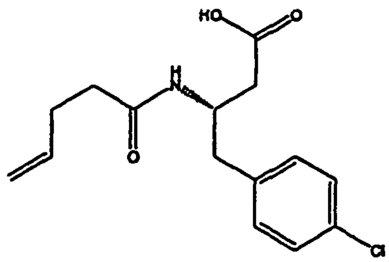 |
| Oligo A8 | CTGGT | $BB_A8$ | 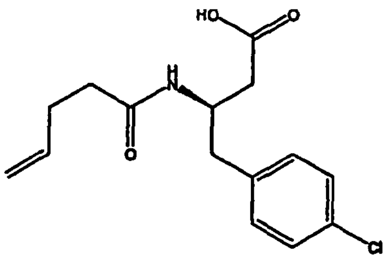 |
| Oligo A9 | TAGGA | $BB_A9$ | 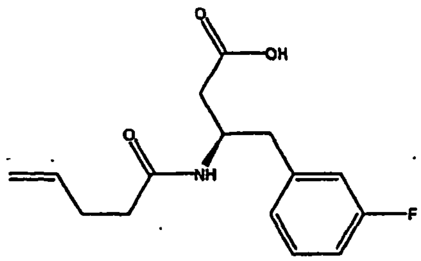 |
| Oligo A10 | ACTCA | $BB_A10$ | 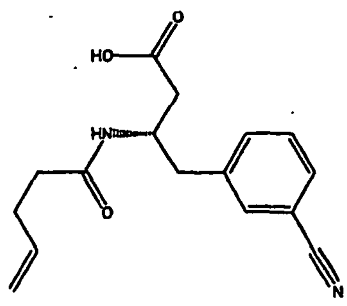 |

(continued)

| Oligo | Codon | Building block | Building block structure |
|---|---|---|---|
| Oligo A11 | CTTAC | $BB_A11$ | 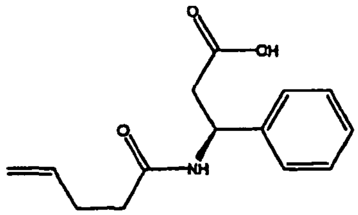 |
| Oligo A12 | CGCAC | $BB_A12$ | 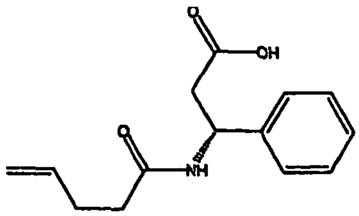 |
| Oligo A13 | TCGCG | $BB_A13$ | 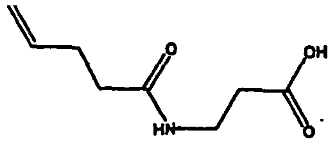 |
| Oligo A14 | CGGAT | $BB_A14$ | 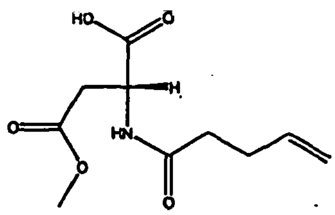 |
| Oligo A15 | GAGAT | $BB_A15$ | 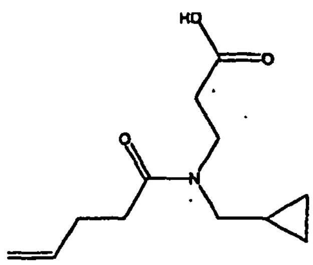 |
| Oligo A16 | TGTAG | $BB_A16$ | 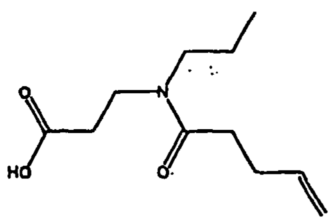 |

(continued)

| Oligo | Codon | Building block | Building block structure |
|---|---|---|---|
| Oligo A17 | GTGTT | $BB_A17$ | 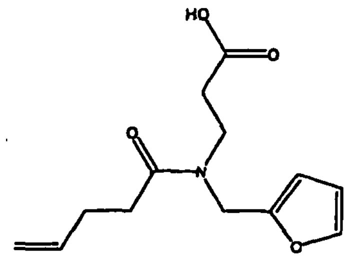 |
| Oligo A18 | AGATG | $BB_A18$ | 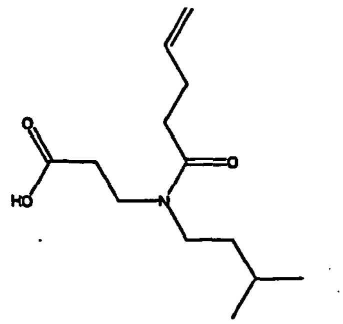 |
| Oligo A19 | ATCCT | $BB_A19$ | 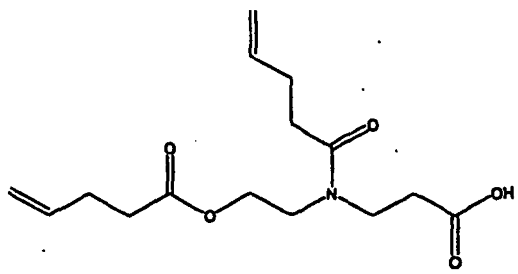 |
| Oligo A20 | TTGCT | $BB_A20$ | 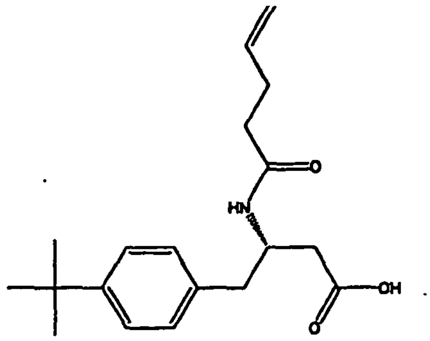 |
| Oligo A21 | ACGTA | $BB_A21$ | 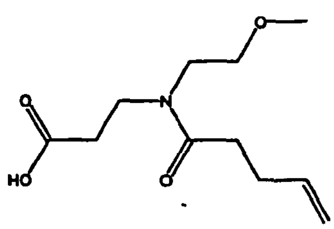 |

(continued)

| Oligo | Codon | Building block | Building block structure |
|---|---|---|---|
| Oligo A22 | ATCAC | $BB_A22$ | 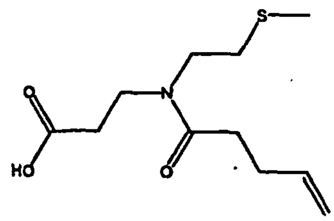 |
| Oligo A23 | TATCC | $BB_A23$ | 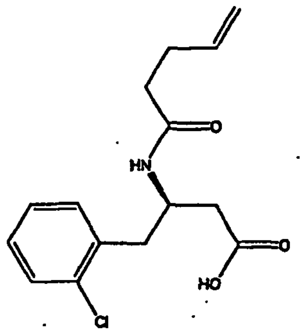 |
| Oligo A24 | GGAAG | $BB_A24$ | 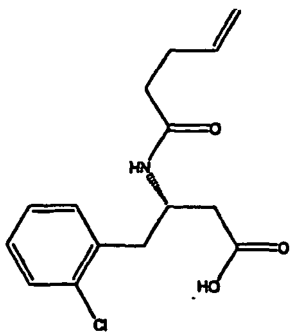 |
| Oligo A25 | CGGTC | $BB_A25$ | 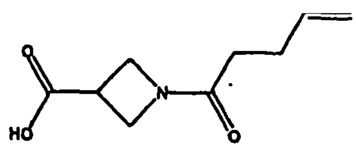 |
| Oligo A26 | TGCTT | $BB_A26$ | 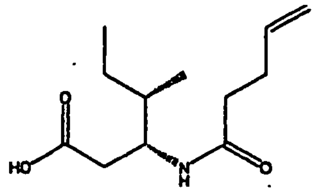 |
| Oligo A27 | TTAGC | $BB_A27$ | 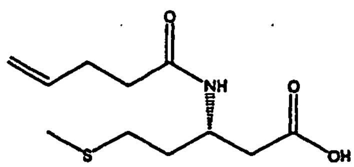 |

(continued)

| Oligo | Codon | Building block | Building block structure |
|---|---|---|---|
| Oligo A28 | GCTGA | $BB_A28$ | 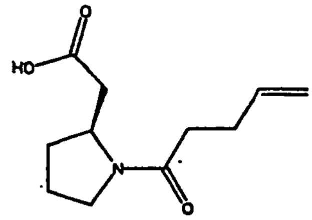 |
| Oligo A29 | GAACG | $BB_A29$ | 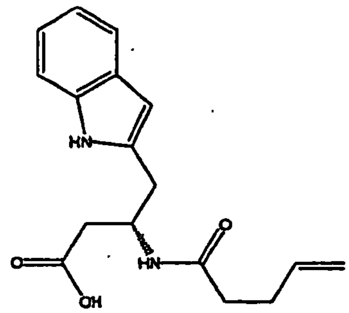 |
| Oligo A30 | CATGG | $BB_A30$ | 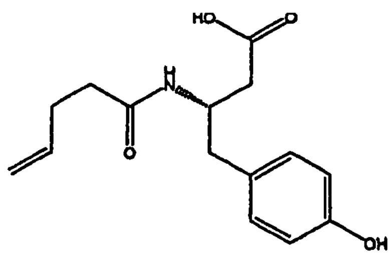 |
| Oligo A31 | TGGTA | $BB_A31$ | 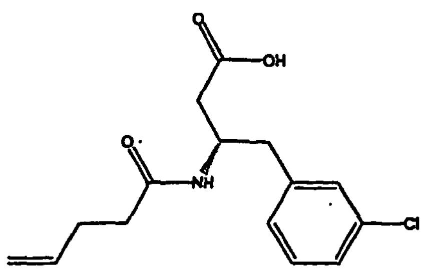 |
| Oligo A32 | TCAAG | $BB_A32$ | 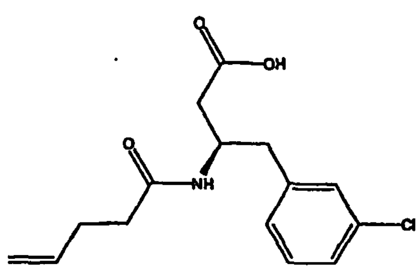 |

(continued)

| Oligo | Codon | Building block | Building block structure |
|---|---|---|---|
| Oligo A33 | ATCGA | $BB_A33$ | 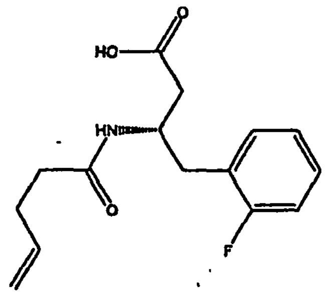 |
| Oligo A34 | ATGCA | $BB_A34$ | 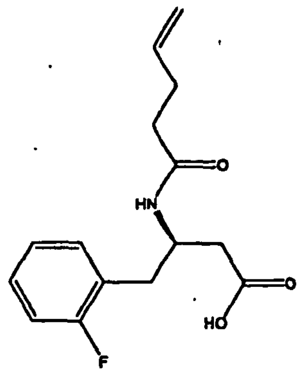 |
| Oligo A35 | ACTAG | $BB_A35$ | 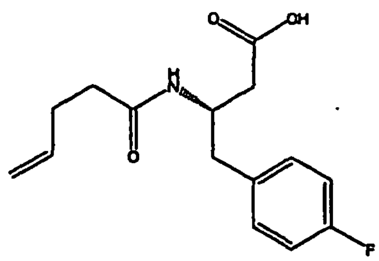 |
| Oligo A36 | TACCT | $BB_A36$ | 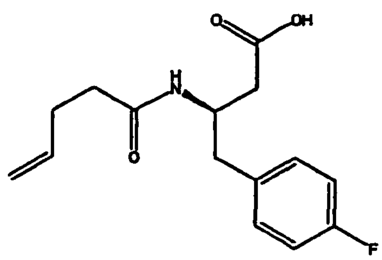 |
| Oligo A37 | TACGA | $BB_A37$ | 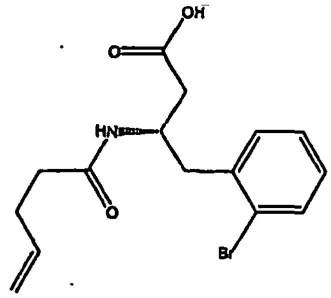 |

(continued)

| Oligo | Codon | Building block | Building block structure |
|---|---|---|---|
| Oligo A38 | CTTCA | $BB_A38$ | 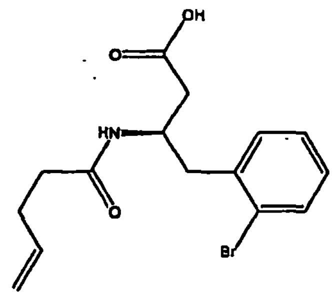 |
| Oligo A39 | CTCTT | $BB_A39$ | 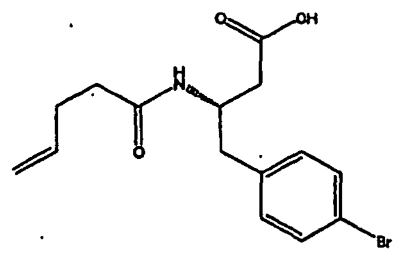 |
| Oligo A40 | TCATC | $BB_A40$ | 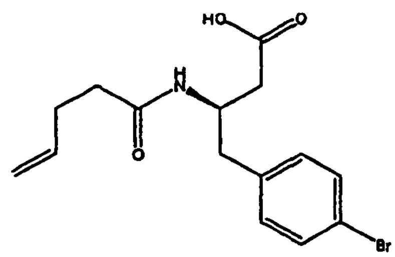 |
| Oligo A41 | ATTCC | $BB_A41$ | 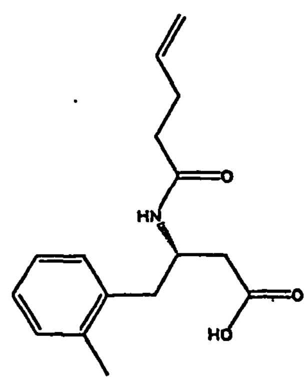 |

(continued)

| Oligo | Codon | Building block | Building block structure |
|---|---|---|---|
| Oligo A42 | CGACG | $BB_A42$ | 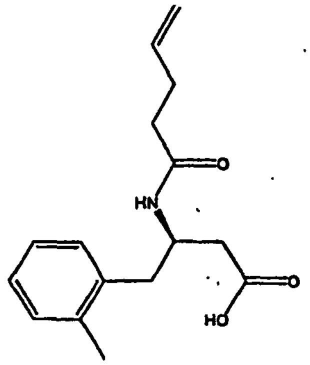 |
| Oligo A43 | CCTGT | $BB_A43$ | 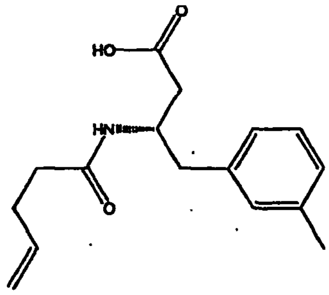 |
| Oligo A44 | CCTTC | $BB_A44$ | 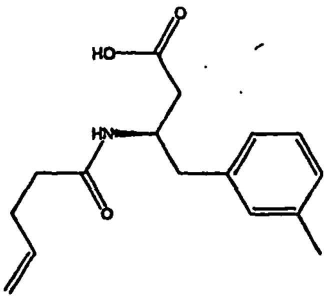 |
| Oligo A45 | ACACC | $BB_A45$ | 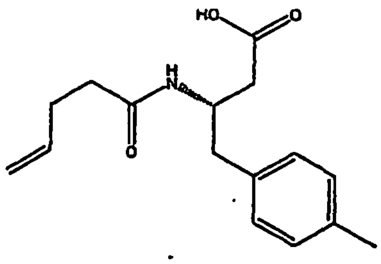 |
| Oligo A46 | TAACA | $BB_A46$ | 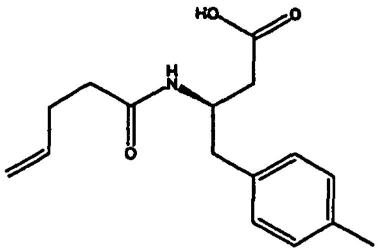 |

(continued)

| Oligo | Codon | Building block | Building block structure |
|---|---|---|---|
| Oligo A47 | TAACA | $BB_A47$ | 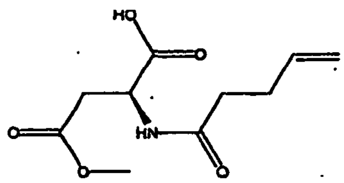 |
| Oligo A48 | CCAGG | $BB_A48$ | 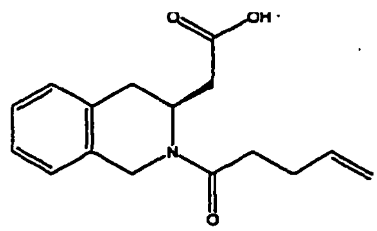 |
| Oligo A49 | ATGTC | $BB_A49$ | 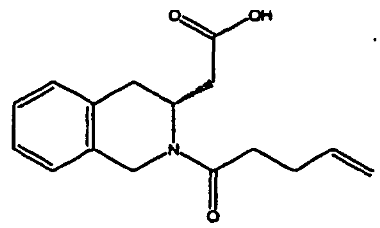 |
| Oligo A50 | GAGGA | $BB_A50$ | 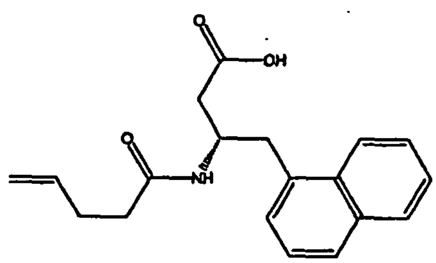 |
| Oligo A51 | GGTCA | $BB_A51$ | 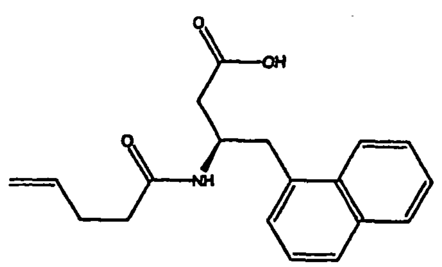 |
| Oligo A52 | GACTT | $BB_A52$ | 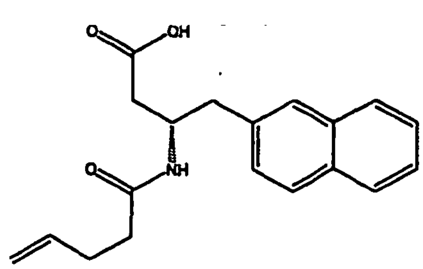 |

(continued)

| Oligo | Codon | Building block | Building block structure |
|---|---|---|---|
| Oligo A53 | GGTGG | $BB_A53$ | 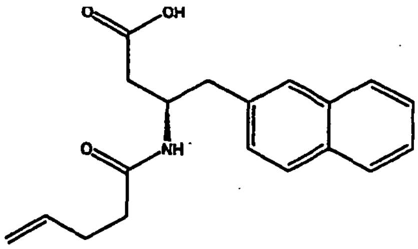 |
| Oligo A54 | CAACT | $BB_A54$ | 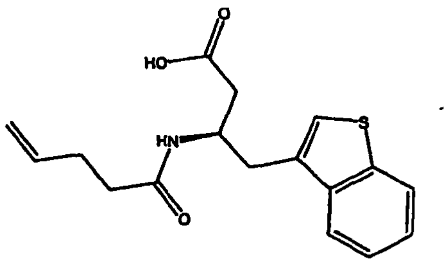 |
| Oligo A55 | ATGAG | $BB_A55$ | 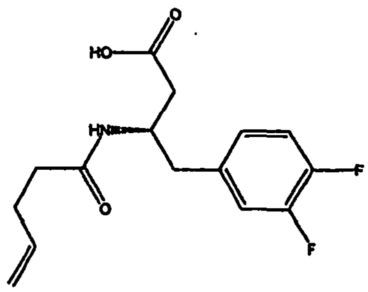 |
| Oligo A56 | TCTGC | $BB_A56$ | 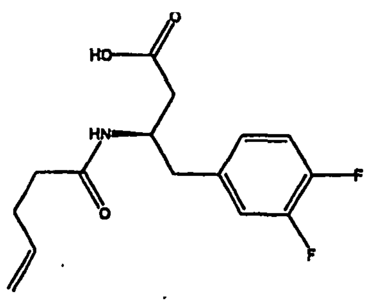 |
| Oligo A57 | ATAGG | $BB_A57$ | 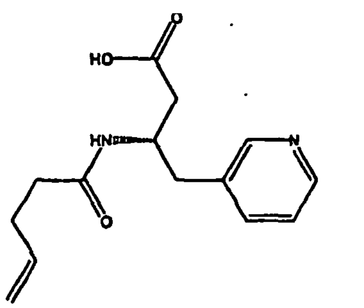 |

(continued)

| Oligo | Codon | Building block | Building block structure |
|---|---|---|---|
| Oligo A58 | CTACC | $BB_A58$ | 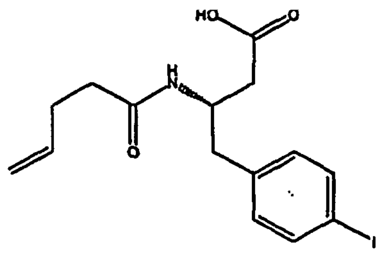 |
| Oligo A59 | AAGTG | $BB_A59$ | 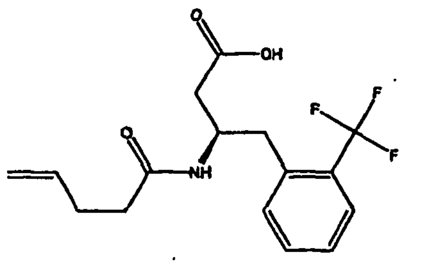 |
| Oligo A60 | TCCAA | $BB_A60$ | 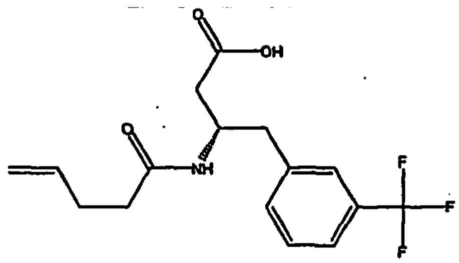 |
| Oligo A61 | GCTCT | $BB_A61$ | 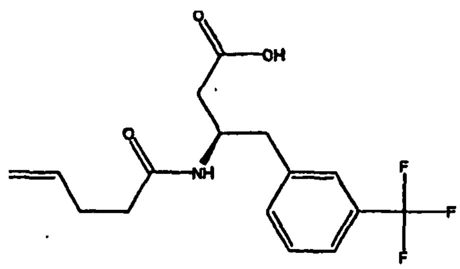 |
| Oligo A62 | GGAGT | $BB_A62$ | 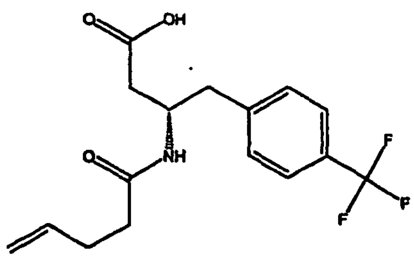 |

(continued)

| Oligo | Codon | Building block | Building block structure |
|---|---|---|---|
| Oligo A63 | AATCG | $BB_A63$ | 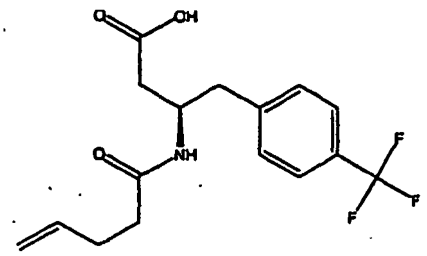 |
| Oligo A64 | AAGCT | $BB_A64$ | 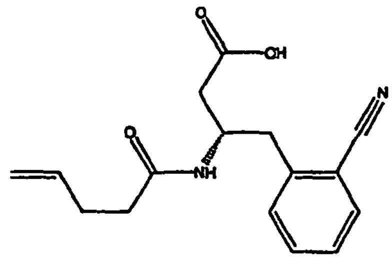 |
| Oligo A65 | CCGAA | $BB_A65$ | 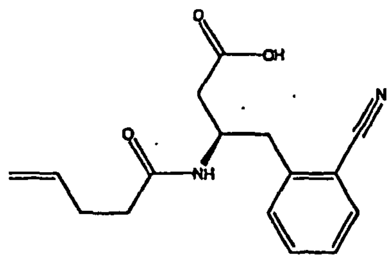 |
| Oligo A66 | TTTGT | $BB_A66$ | 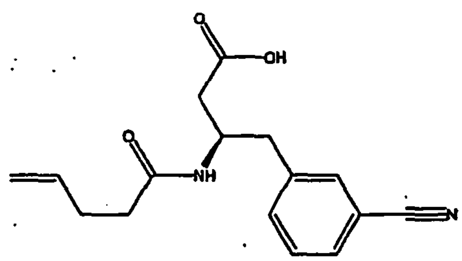 |
| Oligo A67 | CCGTG | $BB_A67$ | 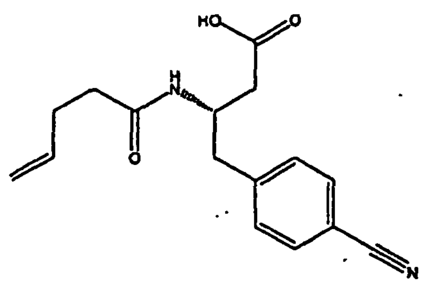 |

(continued)

| Oligo | Codon | Building block | Building block structure |
|---|---|---|---|
| Oligo A68 | TTTCG | $BB_A68$ | 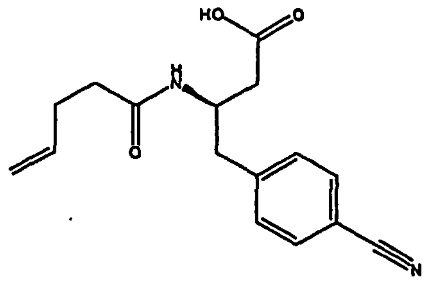 |
| Oligo A69 | TGAGG | $BB_A69$ | 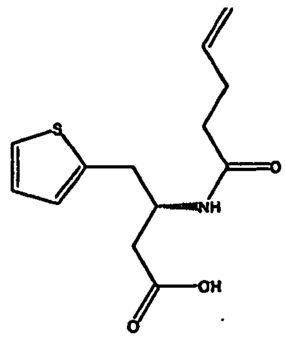 |
| Oligo A70 | GTTGC | $BB_A70$ | 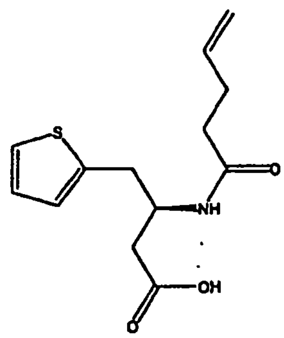 |
| Oligo A71 | AACTA | $BB_A71$ | 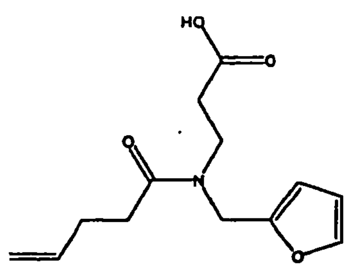 |

(continued)

| Oligo | Codon | Building block | Building block structure |
|---|---|---|---|
| Oligo A72 | AACTA | $BB_A72$ | 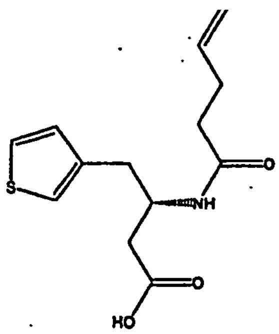 |
| Oligo A73 | CCTCG | $BB_A73$ | 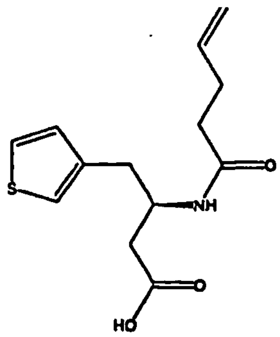 |
| Oligo A74 | AGCAA | $BB_A74$ | 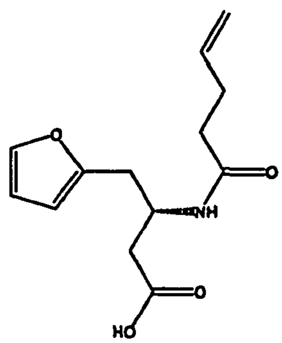 |
| Oligo A75 | TTCCA | $BB_A75$ | 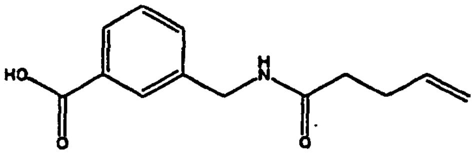 |
| Oligo A76 | AGACT | $BB_A76$ | 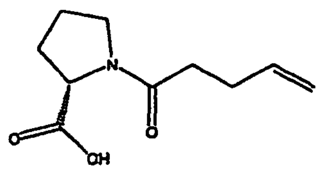 |

(continued)

| Oligo | Codon | Building block | Building block structure |
|---|---|---|---|
| Oligo A77 | AGGTT | $BB_A77$ | 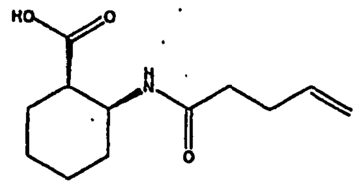 |
| Oligo A78 | GCGTC | $BB_A78$ | 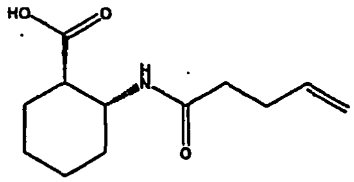 |
| Oligo A79 | AACGT | $BB_A79$ | 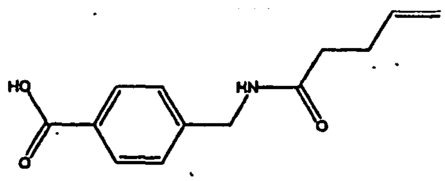 |
| Oligo A80 | CAAGA | $BB_A80$ | 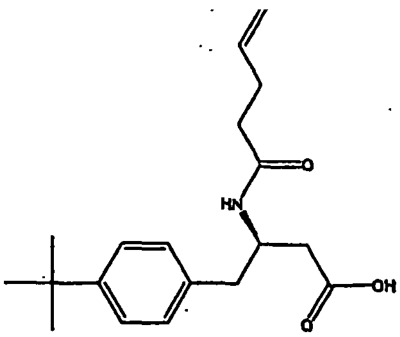 |
| Oligo A81 | AGAGA | $BB_A81$ | 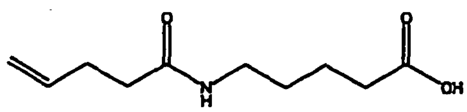 |
| Oligo A82 | GTACT | $BB_A82$ | 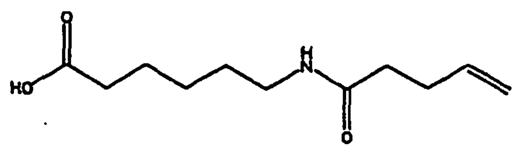 |
| Oligo A83 | TAGAG | $BB_A83$ | 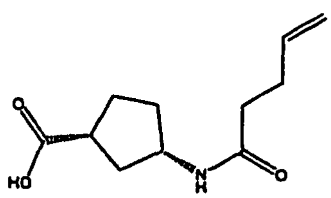 |

(continued)

| Oligo | Codon | Building block | Building block structure |
|---|---|---|---|
| Oligo A84 | ACGAT | $BB_A84$ | 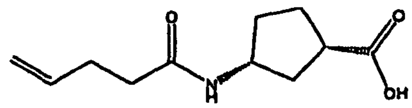 |
| Oligo A85 | GACCA | $BB_A85$ | 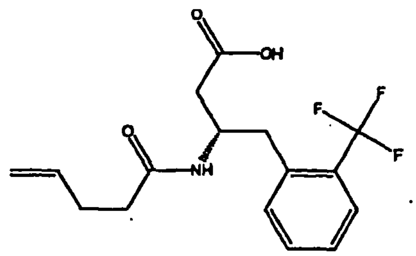 |
| Oligo A86 | TCGTT | $BB_A86$ | 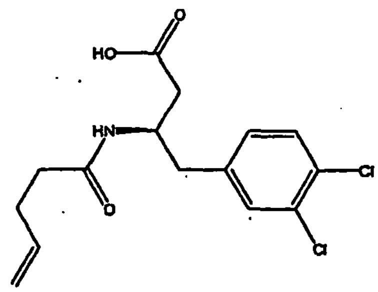 |
| Oligo A87 | GTCTC | $BB_A87$ | 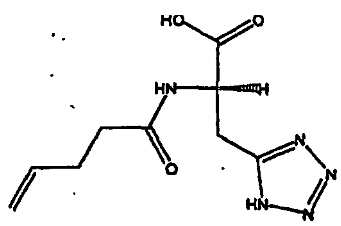 |
| Oligo A88 | CAGCA | $BB_A88$ | 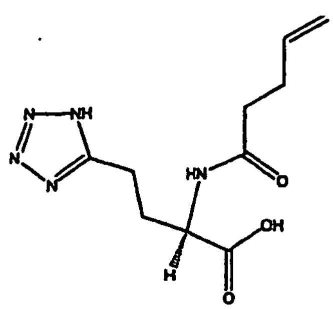 |
| Oligo A89 | TAGTC | $BB_A89$ | 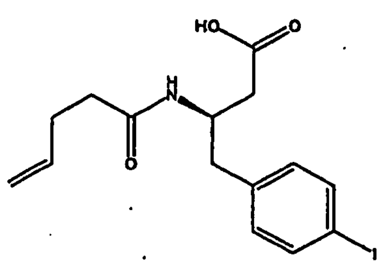 |

(continued)

| Oligo | Codon | Building block | Building block structure |
|---|---|---|---|
| Oligo A90 | GGGTG | $BB_A90$ | 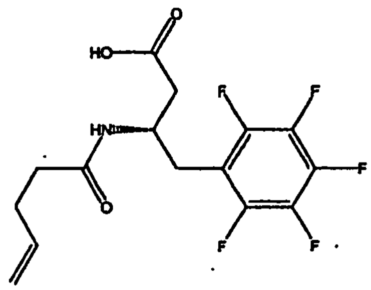 |
| Oligo A91 | CTCAG | $BB_A91$ | 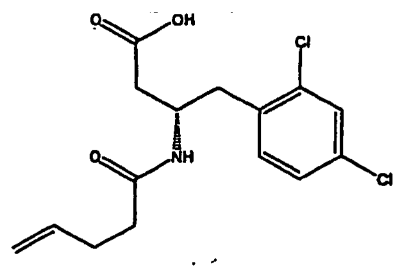 |
| Oligo A92 | AGAAC | $BB_A92$ | 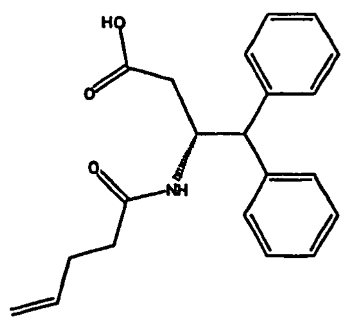 |
| Oligo A93 | GCGAG | $BB_A93$ | 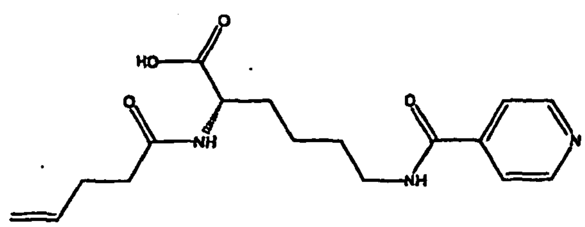 |
| Oligo A94 | GATGT | $BB_A94$ | 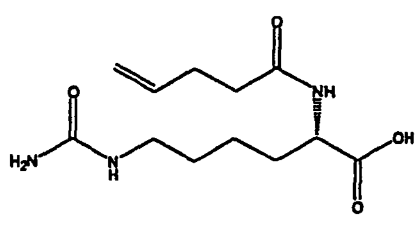 |

(continued)

| Oligo | Codon | Building block | Building block structure |
|---|---|---|---|
| Oligo A95 | TCACT | $BB_A95$ | 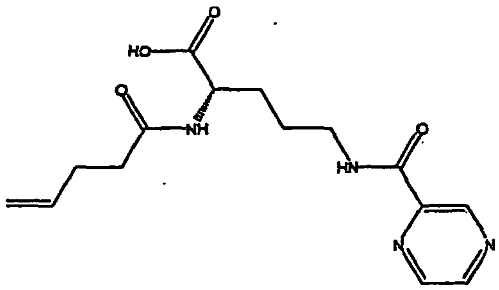 |
| Oligo A96 | CGTCT | $BB_A96$ | 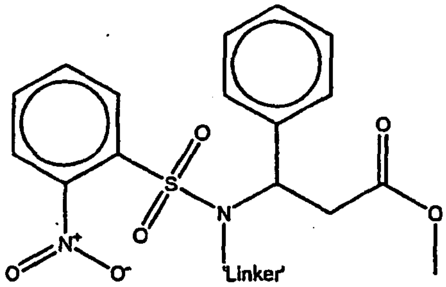 |
| Oligo A97 | AGCTC | $BB_A97$ | 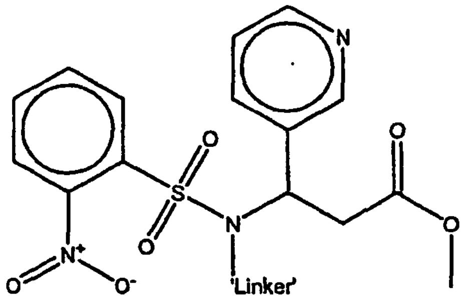 |
| Oligo A98 | CACTC | $BB_A98$ | 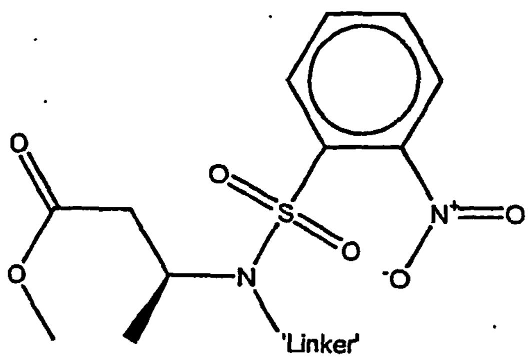 |

(continued)

| Oligo | Codon | Building block | Building block structure |
|---|---|---|---|
| Oligo A99 | CAGTT | $BB_A99$ | 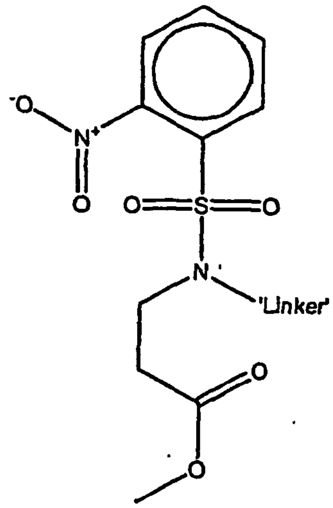 |

*Second round of library generation (round B) :*

25 different combinations of $BB_Bx$ and [oligo Bx-oligo bx] pairs were used.

Onto the free $NH_2$-group of the above loaded and deprotected $BB_Ax$ building blocks were loaded the below listed $BB_Bx$ building blocks:

Oligo Bx general structure :
5'- $HPO_3$-CACAAGTACGAACGTGCATCAGAG-3'

The underlined part corresponds to the 20 nucleotide sequence that varies among different B oligos, ie. the codon. The remaining sequence is identical among the B oligos.

The corresponding b oligos used have the general structure :

5'-$HPO_3$- TCCTCTCTGATGCACGTTCGTACT

Every b oligo can anneal to a specific B oligo. As can be seen the above shown B oligo can anneal to the above shown b oligo.

| Oligo | Codon | Building block | Building block structure |
|---|---|---|---|
| Oligo B1 | AGTACGAACGTGCATCAGAG | $BB_B1$ | 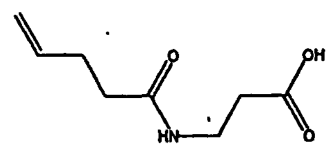 |

(continued)

| Oligo | Codon | Building block | Building block structure |
|---|---|---|---|
| Oligo B2 | TAGTCTCCTCCACTTCCATG | $BB_B2$ | 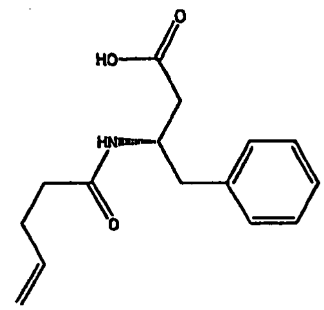 |
| Oligo B3 | TACATCGTTCCAGACTACCG | $BB_B3$ | 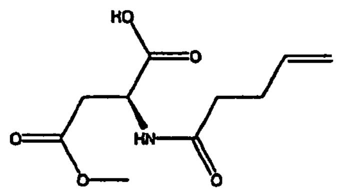 |
| Oligo B4 | TCCAGTGCAAGACTGAACAG | $BB_B4$ | 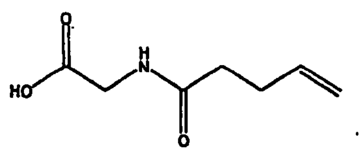 |
| Oligo B5 | AGCATCACTACTCTGTCTGG | $BB_B5$ | 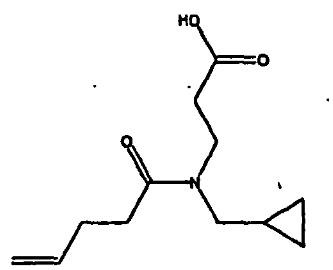 |
| Oligo B6 | TCTTGTCAACCTTCCATGCG | $BB_B6$ | 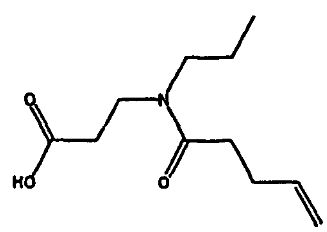 |

(continued)

| Oligo | Codon | Building block | Building block structure |
|---|---|---|---|
| Oligo B7 | AAGGACGTTCCTAGTAGGTG | $BB_B7$ | 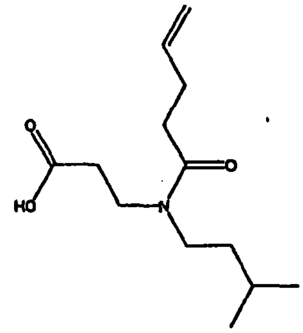 |
| Oligo B8 | GGAACCATCAAGATCCTGAG | $BB_B8$ | 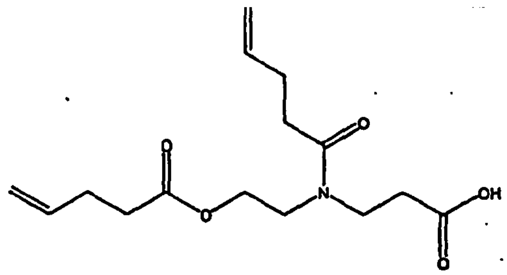 |
| Oligo B9 | ATCTCTGACGAGATCCAAGG | $BB_B9$ | 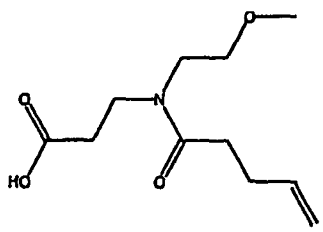 |
| Oligo B10 | TCAAGGTTGGTGGTGTACTG | $BB_B10$ | 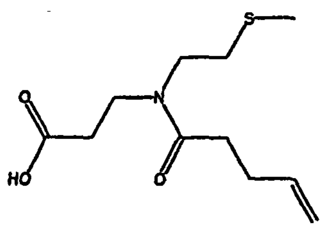 |
| Oligo B11 | TCGAACTTGTTGCTTCCTCG | $BB_B11$ | 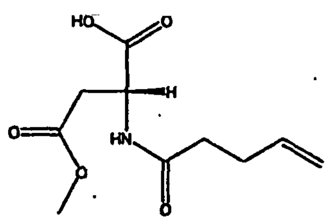 |
| Oligo B12 | CTGAGTGTGTAGTACCAACG | $BB_B12$ | 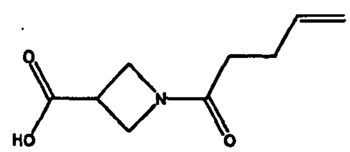 |

(continued)

| Oligo | Codon | Building block | Building block structure |
|---|---|---|---|
| Oligo B13 | ATCTTGGTTGTTCTCCTGCG | $BB_B13$ | 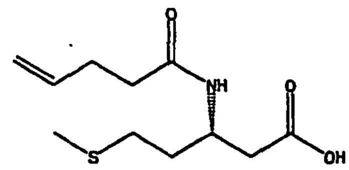 |
| Oligo B14 | TAGTAGCTTGGAGTAGACCG | $BB_B14$ | 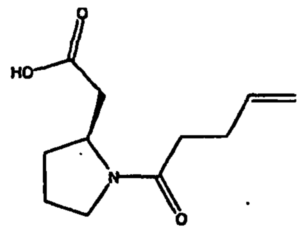 |
| Oligo B15 | TTCACTCCATGCAGCATGTG | $BB_B15$ | 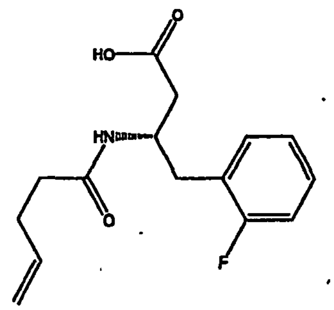 |
| Oligo B16 | ACGATGGTGATCGATCAACG | $BB_B16$ | 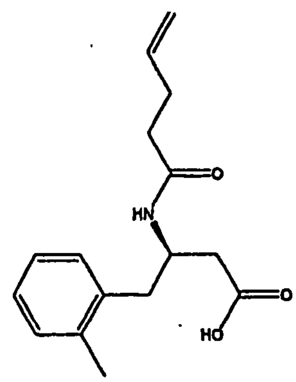 |
| Oligo B17 | TTCAGTGCTTGAGCTACCTG | $BB_B17$ | 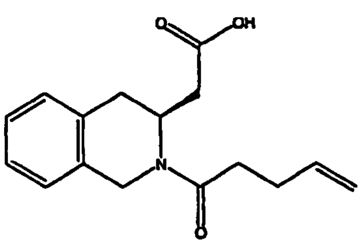 |
| Oligo B18 | TTGGACTCTTCTTGCACCAG | $BB_B18$ | 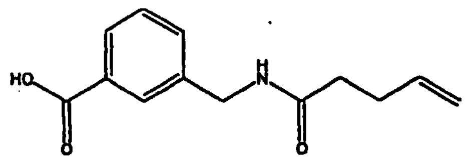 |

(continued)

| Oligo | Codon | Building block | Building block structure |
|---|---|---|---|
| Oligo B19 | TCAACCAACTGGTTCTTGGG | $BB_B19$ | 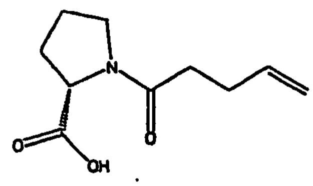 |
| Oligo B20 | TAGTACTCTACACTGCTGCG | $BB_B20$ | 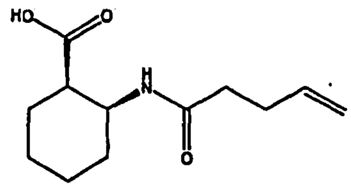 |
| Oligo B21 | TACACCATGACTTGCAGACG | $BB_B21$ | 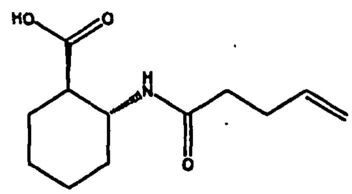 |
| Oligo B22 | GCATCTTGAGTCGTTGAACG | $BB_B22$ | 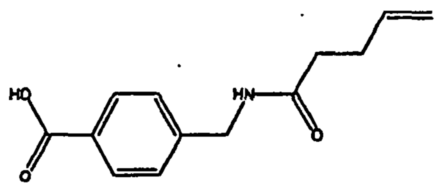 |
| Oligo B23 | GACTCATCTCACTGGAGTTG | $BB_B23$ | 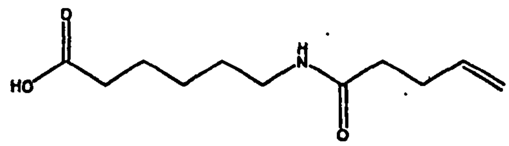 |
| Oligo B24 | TCCAGCTTCTAGGAAGACAG | $BB_B24$ | 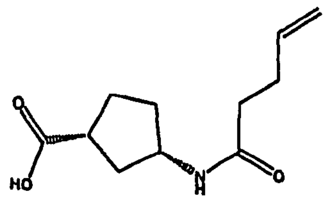 |
| Oligo B25 | CTTCTTGAGTGCACTAGCAG | $BB_B25$ | 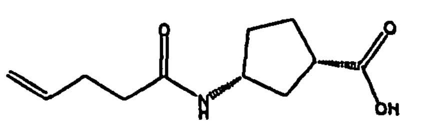 |

*Third round of library generation (round C) :*

25 different C oligos were used :

Oligo Cx general structure :
5'- **$HPO_3$**-CACAAGTACGAACGTGCATCAGAG-3'

The underlined part corresponds to the 20 nucleotide sequence that varies among different C oligos, ie. the codon. The remaining sequence is identical among the C oligos.

Onto these oligos were loaded the listed $BB_C$x building blocks:

Oligo Cx example : 5'-$HPO_3$-AGGACGAGCAGGACCTGGAACCTGGTGCGTTCCTCCACCACGTCTCCG

Oligo cx example : 5'- GCACCAGGTTCCAGGTCCTGCTCG

| Oligo | Codon | Building block | Building block structure |
|---|---|---|---|
| Oligo C1 | CGAGCAGGACCTGGAACCTGGTGC | $BB_C1$ | 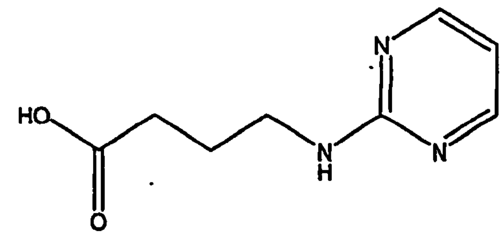 |
| Oligo C2 | CTCGACCACTGCAGGTGGAGCTCC | $BB_C2$ | 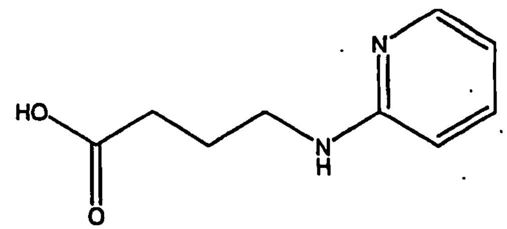 |
| Oligo C3 | CGTGCTTCCTCTGCTGCACCACCG | $BB_C3$ | 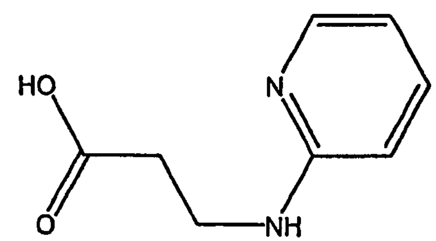 |
| Oligo C4 | CCTGGTGTCGAGGTGAGCAGCAGC | $BB_C4$ | 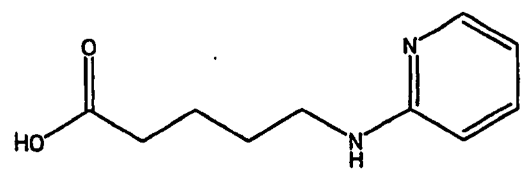 |

(continued)

| Oligo | Codon | Building block | Building block structure |
|---|---|---|---|
| Oligo C5 | CTCGACGAGGTCCATCCTGGTCGC | $BB_C5$ | 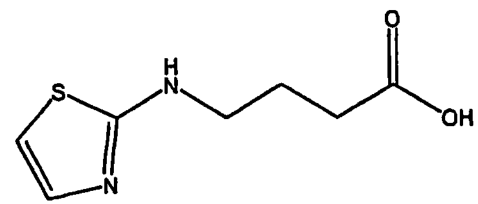 |
| Oligo C6 | CGTGAGGAGCAGGTCCTCCTGTCG | $BB_C6$ | 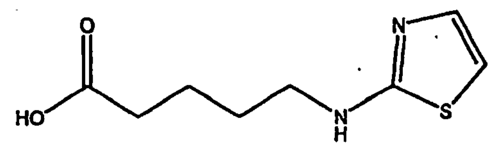 |
| Oligo C7 | CCTGACACTGGTCGTGGTCGAGGC | $BB_C7$ | 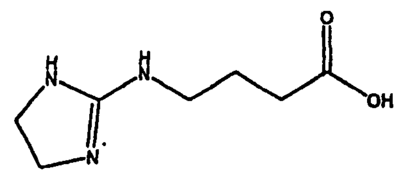 |
| Oligo C8 | CCATCTCGACGACCTGCTCCTGGG | $BB_C8$ | 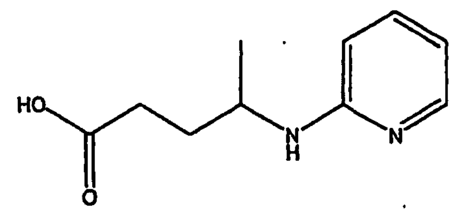 |
| Oligo C9 | CCACGAGGTCTCCACTGGTCCAGG | $BB_C9$ | 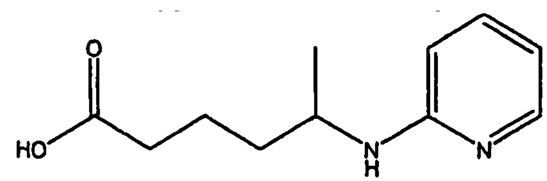 |

(continued)

| Oligo | Codon | Building block | Building block structure |
|---|---|---|---|
| Oligo C10 | CCACTGAGCTGCTCCTCCAGGTGG | $BB_C10$ | 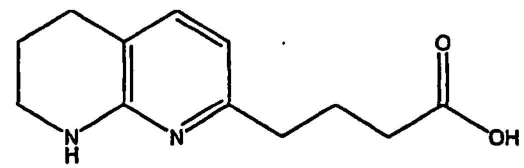 |
| Oligo C11 | CCTCCTGTCCTGCACGTCCATCCG | $BB_C11$ | 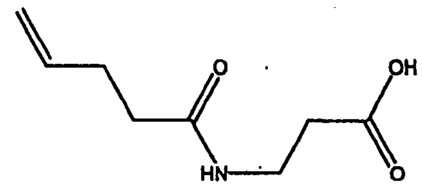 |
| Oligo C12 | CAGCACCTGGAGGTAGGACCACGG | $BB_C12$ | 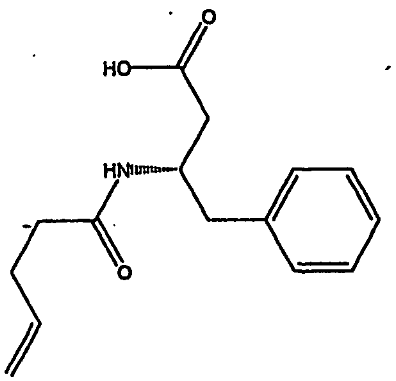 |

(continued)

| Oligo | Codon | Building block | Building block structure |
|---|---|---|---|
| Oligo C13 | CGACCAGACGAGGACCAGGTAGGC | $BB_C13$ | 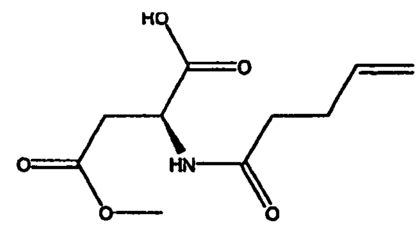 |
| Oligo C14 | CCAGGTTCGAGGACCTCGTCAGCC | $BB_C14$ | 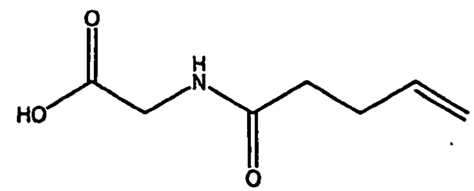 |
| Oligo C15 | CGAGCACGAGGAGCACGTGTCCAG | $BB_C15$ | 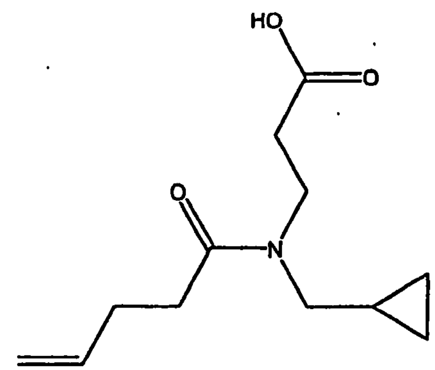 |

(continued)

| Oligo | Codon | Building block | Building block structure |
|---|---|---|---|
| Oligo C16 | CCACGTCCACAGGTGCACCAGGTG | $BB_C16$ | 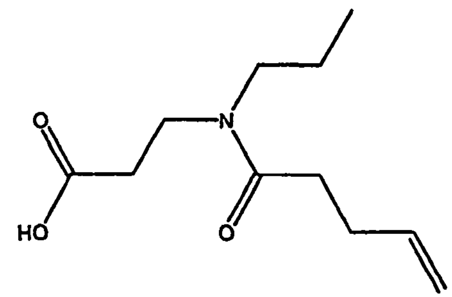 |
| Oligo C17 | CCTGGTGCTCCACGACGTGCTTCG | $BB_C17$ | 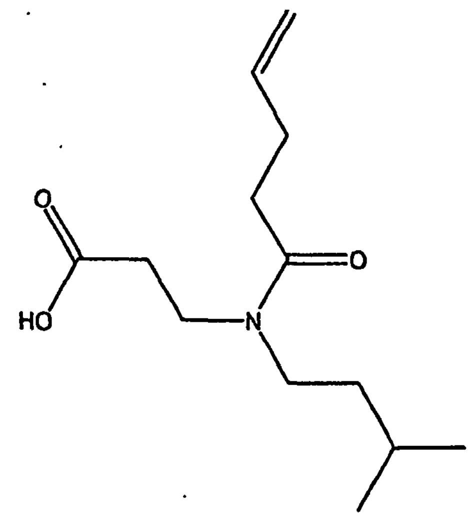 |

(continued)

| Oligo | Codon | Building block | Building block structure |
|---|---|---|---|
| Oligo C18 | CACGTGACGACCTGGTCAGGTGGG | $BB_C18$ | 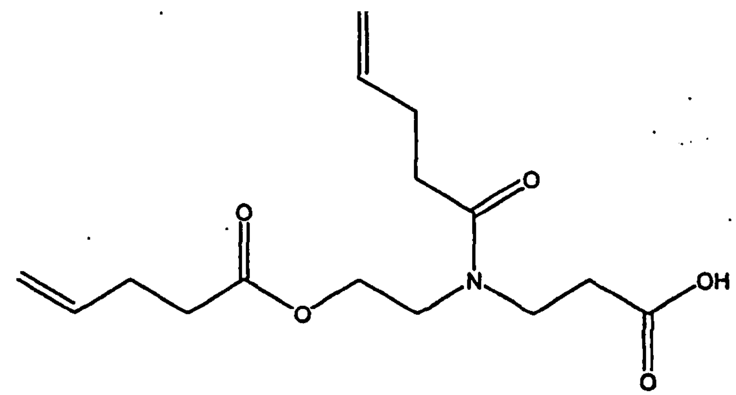 |
| Oligo C19 | CGTAGCTCGTGCTGGTCCTCCTGG | $BB_C19$ | 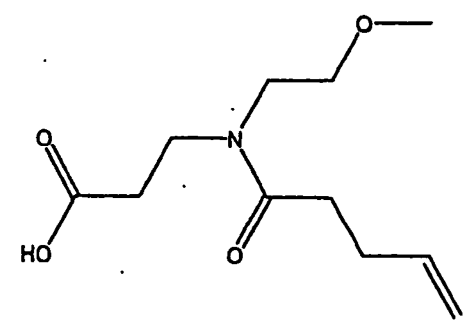 |
| Oligo C20 | CGACGACCACCACCTTGGACACCC | $BB_C20$ | 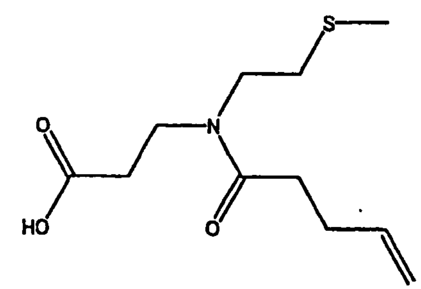 |

(continued)

| Oligo | Codon | Building block | Building block structure |
|---|---|---|---|
| Oligo C21 | CCTACGTCGTGCTCACGTCCTGCC | $BB_C21$ | 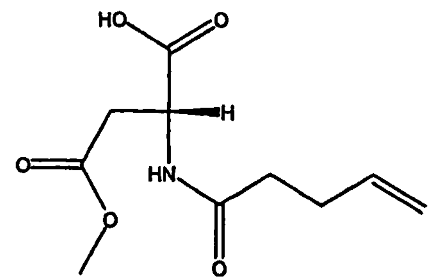 |
| Oligo C22 | CGACGACAGCTAGGAGGAGGTGGG | $BB_C22$ | 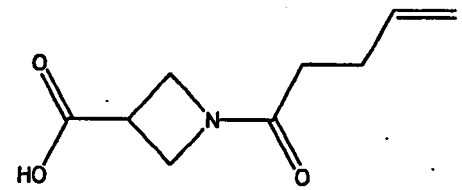 |
| Oligo C23 | CTGGTGGAGCTGCACGAGCACAGC | $BB_C23$ | 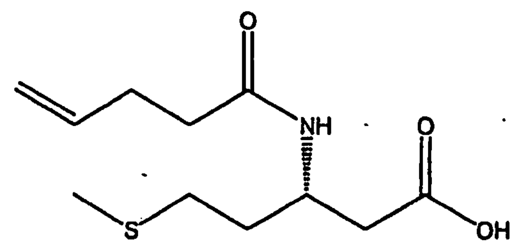 |

(continued)

| Oligo | Codon | Building block | Building block structure |
|---|---|---|---|
| Oligo C24 | CAGGACTGGACGACGACCAGGTCG | $BB_C24$ | 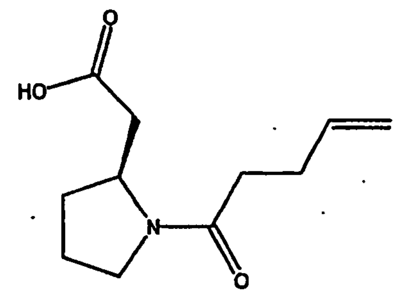 |
| Oligo C25 | CGATGCTGCAGACGACCAGCACCC | $BB_C25$ | 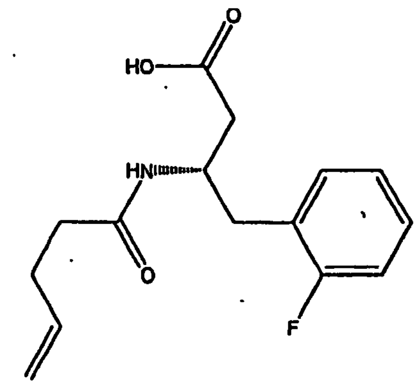 |

*Final extension*

Oligo d: 5'-CGGAGACGTGGTGGAGGAAC

Results of sequencing the identifier oligonucleotides that result from the target selection

Fraction of templates encoding any binder before selection (estimated): 1 in 61.875

Sequenced identifier oligonucleotides after selection (codons corresponding to small molecule X are shown in bold italics):

**» G052.abd (53)**

**CGGCAGCCATGGCGTCGCACAATCTTGGTTGTTCTCCTGCGAGGACCATCTCGAACGACCTGGGGTT**

**» F012.abd (53)**

**CGGCAGCACGATCGTCGCACAGGAACCATCAAGATCCTGAGGGGACCTGACACTGGTCGTGGTCGAGGCGTT**

**» E02.abd (53)**

**CGGCAGCAACTACGTCGCACATAGTCTCCTCCACTTCCATGAGGACCATCTCGACGACCTACTCCTGGGGTT**

**» E032.abd (52)**

**CGGGTGCCAAGGTGGTGGTCGTCGTCTCGCAGGAGAACAACCAAGATTGTGCGACGGCTAAGCTGCCGCT**

**» E03.abd (53)**

**CGGCAGCACACCCGTCGCACATTCACTCCATGCAGCATGCGAGGACCTGACACTGGTCGTGGTCGAGGCGTT**

**» H022.abd (52)**

**CGGCAGCGTACTCGTCGCACATCAAGGTTGGTGGTGTAACTGAGGACTCGACCACTGCAGGTGGAGTTCCGTT**

**» E01.abd (53)**

**CGGCAGCTTTGTCGTCGCACATCGAACTTGTTGCTTCCTCGAGGACCATCTCGACGACCTGCTCCTGGGGTT**

**» H042.abd (52)**

**CGGCAGCCTTACCGTCGCACATCCAGTGCAAGACTGAACAGAGGACCTGGTTGTCGAGGTGAGCAGCAGCGTT**

**» F042.abd (53)**

**CGGCAGCCTTACCGTCGCACATCAACAACTGGTTCTTGGGAGGACCACGTCCACAGGTGCACCAGGTGGTT**

**» E05.abd (53)**

**CGGCAGCCCTCGCGTCGCACATAGTCTCCTCCACTTCCATGAGGACCTGGTGCGCCACGACGTGCTTCGGTT**

**» E04.abd (52)**

**CGGCAGCGTTATCGTCGCACATCCAGTGCAAGACTGAACAGAGGACCACTGAGCTGCTCCTCCAGGTGGGTT**

**» G032.abd (53)**

**CGGCAGCTAACACGTCGCACATCCAGCTTCTAGGAAGACAGAGGACCTACCTCGTGCTCACGTCCTGCCGTT**

**» F062.abd (52)**

**CGGGTGCTGGTCGTCTGCAGCATCGTCCTCTGCTAGTGCACTCAAGAAGTGTGCGACGGGAATGCTGCCGCT**

**» H062.abd (53)**

**CGGCAGCGGATACGTCGCACAAGTACGAACGTGCATCAGAGAGGTCGACCCTGCAGGTGGAGCTCCGTT**

**» G062.abd (54)**

**CGGCAGCAGCTCCGTCGCACATCCAGTGCAAGACTGAACAGAGGACAGCACCTGGAGGTAGGACCACGGGTT**

» E022.abd (53)
CGGCAGCTAACACGTCGCACATCGAACTTGTTGCTTCCTCGAAGGACCACTGAGCTGCTCCTCCAGGTGGGTT
» E012.abd (53)
CGGGTGCTGGTCGTCTGCAGCATCGTCCTCTGCTAGTGCACTCAAGAAGTGTGCGACGGGAATGCTGCCGCT
» H012.abd (53)
CGGCAGCA*TTCC*CGTCGCACA*CTTCTTGAGTGCACTAGCAG*AGGA*CGATGCTGCAGACGACCAGCACCC*GTT
» E062.abd (53)
CGCGACCAGGATGGACCTCGTCGAGTCCTCTGTTCAGTCTTGCACTGGATGCGACGGCACTGCTGCCGCT
» F032.abd (53)
GGAACCTGGACAGTTGGAGACCTCGTGGTCCTCGTCTACAAGTCATGGTGTATGTGCGACGACCAGGCTGCCGCT
» F022.abd (52)
CGGCAGCCGCACCGTCGCACAGCATCTTGAGTCGTTGAACGAGGACTCGACCACTGCAGGTGGAGCTCCGTT
» H052.abd (53)
CGGCAGCACACCCGTCGCACAGGAACATCAAAGATCCTGAGAGGACCATCTCGACGACCTGCTCCTGGGGTT
» G022.abd (53)
CGGCTGACGAGGTCCTCGAACTGGTCCTCACCTAGTAGGAACGTCCTTTGTGCGACGAGTTGGCTGCCGCT
» G042.abd (53)
CGGCAGCTCTGCCGTCGCACACTTCTTGAGTGCACTAGCAGAGGACCACGAGGTCTCCACTGGTCCAGGTT
» E042.abd (55)
CGCTGCTGCTCACCTCGACACCAGGTCCTCCTTGGATCTCGTCAGAGATTGTGCGACGGCTCGGCTGCCGCT
» E052.abd (54)
CGGCAGCCACTCCGTCGCACACTGAGTGTGTAGTACCAACGAGGACGAGCACGAGGAGCACGTGTCCAGCGTT
» G012.abd (54)
CGGCAGCGTTCCGTCGCACATTCAGTGCTTGAGCTAACTGAGGACACTCGTNGATGATCCTGCTACCGTNGGGTT
» H032.abd (53)
CGGCAGCATCCTCGTCGCACATAGTAGCTTGGTACGTATGACCGAGGACCACAGAAGGTCTCCACGTGGTCCAGGTT

Fraction of identifiers encoding binder X after selection before MISE treatment: 1 out of 28. Enrichment fold in selection : (1/27)/(1/61.875) = 2210 fold (theoretical maximum is 61875 fold).

Results of sequencing the identifier oligonucleotides that result from mismatch selection (MISE) (codons corresponding to small molecule X are shown in bold italics):

» MISE5_r140 (69) GGCAGCA*TTCC*CGGTCGCACA*CTTCTTGAGTGCA*-
*CTAGCAG*AGGA*CGATGCTGCAGACGACCATGCACCC*GTTC
« MISE5_r112 (192) GGCAGCA*TTCC*CGTCGCACA*CTTCTTGAGTGCA*-
*CTAGCAG*AGGA*CGATGCTGCAGACGACCAGCACCC*GTTC
« MISE5_r117 (183) GGCAGCTA*TTCC*ACTGTCGCTACA*CTTCTTGTAGTGCA*-
*CTAGCAG*AGGA*CGATGCTGCATACAGACCAGCACCC*GTTC

« MISE5_r115 (201)

GGCAGCATTCTCTCGTCGCACACTTCTTGAGTGCAGCTAGCAGAGGATCGATGCTGCATGACGATCCAGCACCCGT

« MISE5_r111 (194)

GCTAGCATTCGCCGTTGCACACTTCTTGAGTGCAGTAGCAGAGGACGATGCTGCAGACGAGCCAGCACCCGTTC

« MISE5_r148 (232)

GGCAGCATTCCCGTCGCACACTTCTTGAGTGCATTAGCAGAGGACGATGCTGCAGAGCGACCAGCACCCGTTC

« MISE5_r123 (208)

GGCAGCGTTTCGCGTGCGCACACTTCGTTGAGTGCAATCTAGCAGAGGACTGATGCTGCTAGACGACCAGCACCCGTT

« MISE5_r137 (212)

GGCAGCATTCCCGTCGCACACTTCTTGAGTGCAGTAGCAGAGGACGATGCTGCANACGAGCCAGCACCCGTTC

« MISE5_r144 (228)

GGCAGCATTCCACGTCGCTACACTTCTTGAGTGCACTAGTCAGAGGANGATGCTGCANACGACCCAGCACCCGTTC

« MISE5_r132 (215)

TGGCAGCATTCCCGTCGCACACTTNNTGAGTGCACTAGCATGAGGATCGATGCTGCAGAGCTACCAGCACCCGTT

« MISE5_r126 (235)

GGCAGCATGTCCCGTCGCTACGCTTCTTGAGTGCATCTAGGCAGAGGACGATGGGCTGCAGACGACCAGCACCCGTT

« MISE5_r105 (247)

GGCAGCATTCCCGTCGCACACTTTCTTGAGTGCAACTAGCAGAGGACGATGTCTGCAGACGACCAGCACCCGTTC

« MISE5_r131 (298)

GGCAGCATTCCCGTCGCACACTTCTTGAGTGCACTAGCAGAGGACGATGCTGTGCAGACGACCAGCACCCGTT

Fraction of templates encoding binder X after MISE treatment : 12 out of 13 Enrichment fold : (12/13)/(1/28) = 26 (theoretical maximum is 28 fold).

The output of the selection process shows 26 different sequences. Thus, it is not possible to rank the corresponding display molecule in accordance with their affinity towards the integrin target. The subsequent Mismatch Selection allows the clear conclusion that a single display molecule prevails over all others.

*EXAMPLE 9 : Using mismatch selection to identify thombin-binding ligands from a 1.105.920-member small molecule library.*

A library of 1.105.920 small molecules displayed from identifier oligonucleotides was provided essentially as outlined in example 8 except 4 rounds of library generation were performed (rounds 1, B, C and D) each including addition of a building block and addition of the corresponding codon oligonucleotide. The first, third and fourth additions of building blocks (rounds A, C and D) were performed using acylation reactions forming amide bonds whereas addition of the second building block (round B) was performed using reductive amination forming a carbon-nitrogen bond. The library included 10 different building blocks at position 1 (round A), 48 building blocks at position 2 (round B), 48 building blocks at position 3 (round C), and 48 building blocks at position 4 (round D).

After library generation a fraction of the library contacted with a target. In the present example the target is the protein Thrombin. However, any target may be used.

The unbound library fraction was removed by washing and the remaining bound fraction was recovered from the target by specific elution, i.e., displacement of the target-bound library fraction from the target by addition of a specific high affinity thrombin binding molecule at an appopriate concentration. The eluted library fraction (hereafter referred to as the output) was then purified by spin column filtration. The process of contacting the library with thrombin followed by elution (this process is hereafter referred to as pseudorounding) was repeated a 4 times. A fraction of the eluted library from pseudoround 4 was either a) applied to a further pseudoround (pseudoround 5) or b) subjected to mismatch selection conditions.

Among the 1.105.920 display molecules in the library are molecules that correspond to the building block combinations BB572-BB1023-BB1025 and BB479-BB1023-BB1025 both correspond to thrombin binders, e.g., the Kd (equilibrium dissociation constant) of the binding between thrombin and a small molecule derived from the BB479-BB1023-BB1025 building block combination was determined to be 1.1 μM using surface plasmon resonance technology (Biacore).

PCR amplification of a fraction of the library before pseuodoround 1 and outputs from pseudoround 4 and pseudoround 5 was performed followed by cloning and sequencing as described in example 8. The fraction of identifier

oligonucleotide sequences codons corresponding to the mentioned building block combinations BB572-BB1023-BB1025 and BB479-BB1023-BB1025 are shown in table 9.1.

TABLE 9.1 : Relative abundances of building blocks as defined by their corresponding identifer oligonucletide codons

| Building block (Position) | Library before pseudoround 1 | Pseudoround 4 output | Pseudoround 5 Output | Output of mismatch selection performed on pseudoround 4 output |
|---|---|---|---|---|
| BB479 (2) | 0.01 | 0.09 | 0.13 | 0.16 |
| BB572 (2) | 0.01 | 0.09 | 0.10 | 0.16 |
| BB1023 (3) | 0.01 | 0.23 | 0.31 | 0.33 |
| BB1025 (4) | 0.03 | 0.16 | 0.20 | 0.25 |

The relative abundances of codons corresponding to building blocks which are part of thrombin-binding molecules in the pseudoround 4 output are significantly different than that of the library before pseudoround 1 ($p = 0.028$, student's t-test, 2-tailed, paired). This is in agreement with the expectation that pseudorounds will decrease the relative abundance of codons corresponding to building blocks which are not part of thrombin-binding small molecules and thereby increase the relative abundance of codons corresponding to building blocks which are part of thrombin-binding small molecules.

The output from pseudoround 5 was not significantly different that the output of pseudoround 4 ($p = 0.19$, student's t-test, 2-tailed, paired).

This is not unexpected because after a number of pseudorounds the majority of the remaining library fraction will constitute display molecules with significant affinity for thrombin. As the abundance of a specific codon combination is positively correlated with the affinity of the displayed molecule, i.e., a high relative abundance of an identifier oligonucleotide indicates a significant affinity of its corresponding display molecule, it is of interest to identify the codon combinations with highest abundance. As the output furthermore contains a large number of binders the fraction of an individual binder will not change significantly once displayed molecules with low affinity for Thrombin have been removed by selection/ pseudorounding. Mismatch selection is a tool to circumvent this problem as it is useful for distinguishing between compounds represented with many templates (which will generally be binders of higher affinity) and compounds represented with few templates (which will generally be binders of relatively lower affinity).

The mismatch selection strategy was applied to the PCR amplified output from pseudoround 4 using the following conditions: To 5 μl PCR product of pseudoround 4 was added 1 μl (10x) Surveyor reaction buffer (Transgenomic), 0.5 μl Enhancer (Transgenomic), 1 μl Surveyor Nuclease (Transgenomic), and 2.5 μl $H_2O$. The reaction components were mixed and incubated for 2 hours at 42˚C. Then the sample was heated to 95˚C for 10 minutes and identifier oligonucleotide duplexes were allowed to form by lowering the temperature. To 3 μl of the resulting sample was added 1 μl 5 x EXT buffer [100 mM HEPES pH 7.5, 750 mM NaCl, 40 mM $MgCl_2$], 4 μl $H_2O$, and 1 μl E. coli Exonuclease I (10 U/μl) (USB). The reaction components were mixed and incubated at 37˚C for 4 hours followed by enzyme inactivation by incubation at 95˚C for 15 minutes.

PCR amplification of the output of mismatch selection on the output from pseudoround 4 was performed followed by cloning and sequencing as described in example 8.

The relative abundances of building blocks corresponding to display molecules with significant thrombin affinity in the output from mismatch selection performed on output from pseudoround 4 were significantly different that those of the output of pseudoround 4 ($p = 0.035$, student's t-test, 2-tailed, paired).

Moreover, The relative abundances of building blocks corresponding to display molecules with significant thrombin affinity in the output from mismatch selection performed on output from pseudoround 4 were also significantly different that those of the output of pseudoround 5 ($p = 0.0083$, student's t-test, 2-tailed, paired).

In conclusion, the relative abundance of codons corresponding to building blocks forming display molecules which bind thrombin were significantly increased by performing mismatch selection on the output from pseudoround 4. In contrast the relative abundance of codons corresponding to building blocks which bind thrombin were not significantly increased by performing pseudoround 5 using the output from pseudoround 4. This demonstrates the advantage provided by the mismatch selection strategy in the attempt to identify molecules or molecular fragments which bind a given target,

*Example 10: Factors affecting MISE efficiency and use of MISE to identify identifier oligonucleotides corresponding to display molecules which bind a target from a library encoding 10^10 small molecule species.*

In this example a (hypothetical) library containing 10^10 small molecules is provided by the method outlined in example 8.

With libraries of this size it is likely that a single step of contacting the library with a target is not sufficient to partition binders from non-binders to an extend that decoding identifier oligonucleotides corresponding to binding small molecules is practically feasible, e.g., it might be necessary to decode more than 1.000.000 identifier oligonucleotides to observe that the relative abundance of a specific identifier oligonucleotide corresponding to a target-binding display molecule is significantly different from the average relative abundances of identifier oligonucleotides corresponding to display molecules with insignificant affinity for target.

An attempt to increase the feasibility of decoding might include performing multiple rounds of contacting a target with the library followed by partitioning of the bound library fraction, i.e., to perform pseudorounds as described in example 9. But as demonstrated in example 9 it is probable that even after performing several pseudorounds (i.e. 4 pseudorounds in example 9) no identifier oligonucleotide is significantly more abundant than other identifier oligonucleotide when performing extensive decoding of identifier oligonucleotides by PCR, cloning and sequencing.

In this case the mismatch selection strategy provides a method for enriching, i.e., increasing the relative abundance of identifier oligonucleotides corresponding to display molecules which bind a target to an extent that identification of these oligonucleotides becomes feasible using PCR, cloning and sequencing, e.g., by decoding a total of 100, 1000, or 10.000 identifier oligonucleotides.

The efficiency of mismatch selection is dependent on a number of probable factors. One such factor is the extend to which one or more rounds of contacting the library with target followed by partitioning of the target-binding library fraction alters the relative abundances of the identifier oligonucleotides corresponding to display molecules that bind target. This is again dependent on the display molecules present in the library and the identity of the target used. Furthermore, a library may contain many binders to a specific target type but essentially no binders to other target types. The efficiency of MISE also depends on the efficiency of degrading or removing heteroduplexes while retaining homoduplexes during the MISE procedure as describe in example 1 calculated example C.

Although a single round of MISE can provide significant enrichment of a specific identifier oligonucleotide as shown in examples 4 and 8 it is possible and indeed very easy and affordable both in terms of time and monetary cost to perform multiple rounds of MISE as described in example 5.

It is likely that performing pseudorounds on a library containing 10^10 small molecules can result in a 1.000.000-fold enrichment, i.e., a 1.000.000-fold increase in the relative abundance, of a specific identifier oligonucleotide corresponding to a display molecule which binds target compared to identifier oligonucleotides which do not bind target significantly. Because a library initially containing 10^10 individual display molecule species is likely to contain a large number of display molecule species that bind a given target it is likely that further pseudorounding at some point will fail to enrich a specific identifier oligonucleotide. That is the relative abundances of identifier oligonucleotides corresponding to display molecules with significant target binding affinity will remain essentially unaltered in the library fraction recovered from pseudoround to pseudoround. Such a situation is described in example 9. However, it is likely that homo- and heteroduplexes can be recovered from such a library fraction during the MISE method with efficiencies of 50 % and 0.1 %, respectively. Importantly, such recovery efficiencies can be easily manipulated and optimized. Using these values in a calculation as outlined in example 1 results in the enrichment of a specific target binding display molecule of 10.000 fold with six rounds of MISE. This results in a total enrichment of 10.000.000.000 fold by combining the enrichment fold achievable by pseudorounding with the enrichment fold achievable by MISE. As the majority or essentially all of the remaining identifier oligonucleotides obtained after 6 rounds of MISE will correspond to a display molecule which binds target it becomes easy to identify this identifier oligonucleotide by PCR, cloning and sequencing. It is possible to decode identifier oligonucleotides by PCR, cloning and sequencing after each MISE step and thereby follow the MISE enrichment process and identify a diverse set of display molecules with affinity for target.

Importantly, the ability of MISE to ease or indeed make possible the identification of identifier oligonucleotides corresponding to display molecules that bind target is not dependent on initial use of several pseudorounds. The only prerequisite for MISE to enable such identification is that the relative abundances of identifier oligonucleotides corresponding to target-binding display molecules is increased from that in the initial library, e.g., by contacting the library with the target and recovering a target-bound library fraction.

## Claims

1. A method for identifying a display molecule of a bifunctional complex further comprising an identifier oligonucleotide linked to the display molecule, said method comprising the steps of

i) providing an initial library of bifunctional complexes comprising a plurality of different display molecules each linked to an identifier oligonucleotide identifying a display molecule,
wherein the display molecule is obtained by a method comprising the steps of

a. providing an oligonucleotide comprising a tag addition site and one or more reactive site(s) for functionalization,
b. reacting said one or more reactive sites with two or more chemical entities, and
c. adding for each chemical entity reacted with a reactive site a unique tag residue to the tag addition site,

thereby obtaining a bifunctional complex comprising a display molecule resulting from the chemical entity reactions, said display molecule being linked to an identifier oligonucleotide comprising the individual unique tag residues,
ii) contacting the initial library with a target and allowing for an interaction between display molecules of the initial library and the target,
iii) partitioning, from said initial library, identifier oligonucleotides identifying display molecules having interacted with the target, wherein the identifier oligonucleotides are in a single stranded form or in a double stranded form comprising complementary single stranded identifier oligonucleotides,
iv) converting identifier oligonucleotides in single stranded form to double stranded identifier oligonucleotides comprising complementary single stranded identifier oligonucleotides, when the identifier oligonucleotides in step iii) are in single stranded form,
v) separating identifier oligonucleotides from display molecules by amplifying the oligonucleotides by polymerase chain reactions or by cleaving the linkers linking identifier oligonucleotides and display molecules in the partitioned bifunctional complexes,
vi) displacing the complementary single stranded identifier oligonucleotides of individual identifier oligonucleotides,
vii) hybridising the single stranded identifier oligonucleotides obtained in step vi),
thereby generating a composition comprising homo-duplex identifier oligonucleotides and hetero-duplex identifier oligonucleotides,
wherein homo-duplex identifier oligonucleotides hybridise without generating any mis-matches between the complementary oligonucleotide strands, and
wherein one or more mis-matches are present when complementary identifier oligonucleotides form hetero-duplexes,
viii) separating homo-duplexes from hetero-duplexes,
ix) obtaining a fraction comprising predominantly homo-duplexes,
x) decoding the identifier oligonucleotides of the homo-duplexes, and
xi) identifying, based on the decoding in step x), the one or more display molecules identified by the decoded identifier oligonucleotides.

**2.** The method of claim 1, wherein the duplex identifier oligonucleotide comprising complementary single stranded identifier oligonucleotides is obtained by hybridisation of a complementary identifier oligonucleotide with the identifier oligonucleotide linked to the display molecule of the bifunctional complex.

**3.** The method of claim 1, wherein the duplex identifier oligonucleotide comprising complementary single stranded identifier oligonucleotides is obtained by hybridising a probe oligonucleotide with the identifier oligonucleotide of the bifunctional complex, enzymatically extending said probe oligonucleotide, thereby obtaining a complementary identifier oligonucleotide hybridised with the identifier oligonucleotide linked to the display molecule of the bifunctional complex.

**4.** The method of any of claims 2 and 3, wherein said duplex identifier oligonucleotide comprising complimentary single stranded identifier oligonucleotides is obtained prior to, during or concomitantly with the step of partitioning the bifunctional complexes.

**5.** The method of any of claims 2 and 3, wherein said duplex identifier oligonucleotide comprising complementary single stranded identifier oligonucleotides is obtained after the step of partitioning the bifunctional complexes.

**6.** The method of claim 1, wherein the identifier oligonucleotide linked to the display molecule is selected from the group consisting of a) a single stranded identifier oligonucleotide and b) a duplex identifier oligonucleotide comprising complementary single stranded identifier oligonucleotides, said method comprising the preliminary steps of

i) providing a composition of bifunctional complexes comprising different display molecules,
ii) providing one or more target molecule(s) having an affinity for one or more of said display molecules,
iii) contacting the composition of bifunctional complexes provided in step i) with the target molecule provided in step ii)
iv) obtaining an enriched fraction of bifunctional complexes comprising one or more display molecules having an affinity for said target molecule,
v) partitioning said enriched fraction obtained in step iv) from bifunctional complexes not having an affinity for said target molecule, and
vi) complementing single stranded identifier oligonucleotides of different bifunctional complexes in which the display molecule is linked to a) a single stranded identifier oligonucleotide, thereby obtaining a duplex identifier oligonucleotide comprising complementary identifier oligonucleotides, with the proviso that no single stranded complementation occurs for bifunctional complexes comprising b) duplex identifier oligonucleotides comprising complementary identifier oligonucleotides:

**7.** The method of claim 6 wherein the display molecule of a bifunctional complex provided in step i) or obtained in step iv) is linked to a single stranded identifier oligonucleotide, said method comprising the further step of complementing said single stranded identifier oligonucleotides of the different bifunctional complexes, thereby obtaining a duplex identifier oligonucleotide comprising complementary identifier oligonucleotides, wherein said complementation of said single stranded identifier oligonucleotides of the different bifunctional complexes takes place prior to partitioning of the bifunctional complexes or after partitioning of the bifunctional complexes or concomitantly with the partitioning of the bifunctional complexes.

**8.** The method of claim 1, wherein the hetero-duplexes are removed by enzymatically degrading the mis-matched, single stranded part of the hetero-duplexes.

**9.** The method fo claim 8, wherein the enzyme comprises nuclease activity or consists of polypeptide having nuclease activity.

**10.** The method of any of claims 8 and 9, wherein the enzyme is selected from the group of enzymes consisting of T4 endonuclease VII, T4 endonuclease I, CEL I, nuclease S1, including variants and combinations thereof.

**11.** The method of any of claims 1 to 10, wherein the linker linking the display molecule and the identifier oligonucleotides is selectively cleavable.

**12.** The method of any of claims 1 to 10, wherein the composition of bifunctional complexes comprise more than 1000 different display molecules.

**13.** The method of any of claims 1 to 10, wherein the partitioned fraction of identifier oligonucleotides is amplified by PCR.

**14.** The method of any of claims 1 to 10, wherein the complementary identifier oligonucleotide strands of homo-duplexes and hetero-duplexes in the fraction comprising predominantly homo-duplexes are separated and allowed to hybridise, said hybridisation resulting in a further fraction comprising predominantly homo-duplexes.

**15.** The method of any of the preceding claims, wherein the display molecules have a a molecular weight of less than 2000 Dalton, such as less than 1000 Dalton, for example less than 500 Dalton.

**16.** The method of any one of the preceding claims, wherein the contacting of the target molecule and the composition of different bifunctional complexes is achieved by mixing the target molecule with the composition of different bifunctional complexes.

**17.** The method of claim 16, wherein the target is saturated with a known ligand prior to contacting the different bifunctional complexes.

**18.** A method for identifying a display molecule of a bifunctional complex further comprising an identifier oligonucleotide linked to the display molecule, said method comprising the steps of

i) providing an initial library of bifunctional complexes comprising a plurality of different display molecules each linked to an identifier oligonucleotide identifying a display molecule,

wherein said display molecule is a small molecule,
ii) contacting the initial library with a target and allowing for an interaction between display molecules of the initial library and the target,
iii) partitioning, from said initial library, identifier oligonucleotides identifying display molecules having interacted with the target, wherein the identifier oligonucleotides are in a single stranded form or in a double stranded form comprising complementary single stranded identifier oligonucleotides,
iv) converting identifier oligonucleotides in single stranded form to double stranded identifier oligonucleotides comprising complementary single stranded identifier oligonucleotides, when the identifier oligonucleotides in step iii) are in single stranded form,
v) separating identifier oligonucleotides from display molecules by amplifying the oligonucleotides by polymerase chain reactions or by cleaving the linkers linking identifier oligonucleotides and display molecules in the partitioned bifunctional complexes,
vi) displacing the complementary single stranded identifier oligonucleotides of individual identifier oligonucleotides,
vii) hybridising the single stranded identifier oligonucleotides obtained in step vi),
thereby generating a composition comprising homo-duplex identifier oligonucleotides and hetero-duplex identifier oligonucleotides,
wherein homo-duplex identifier oligonucleotides hybridise without generating any mis-matches between the complementary oligonucleotide strands, and
wherein one or more mis-matches are present when complementary identifier oligonucleotides form hetero-duplexes,
viii) separating homo-duplexes from hetero-duplexes,
ix) obtaining a fraction comprising predominantly homo-duplexes,
x) decoding the identifier oligonucleotides of the homo-duplexes, and
xi) identifying, based on the decoding in step x), the one or more display molecules identified by the decoded identifier oligonucleotides.

**19.** The method of claim 18, wherein said small molecule display molecule is a drug candidate.

**20.** The method of claim 18 or 19, wherein said display molecule is a scaffolded molecule.

**21.** The method of any one of the claims 18-20, wherein said display molecule is an artificial substance.

**22.** The method of any one of the claims 18-21, wherein said display molecule has a molecular weight of less than 2000 Dalton, such as less than 1000 Dalton, for example less than 500 Dalton.

**23.** The method of any one of the claims 18-22, wherein said display molecule is synthesised by reacting 4-8 chemical entities.

**24.** The method of any one of the claims 18-23, wherein said display molecule has affinity for said target and said display molecule has a Kd value for said target of less than 100 μM, such as less than 10 μM, such as less than 1 μM, such as less than 100 nM, such as less than 10 nM, such as less than 1 nM.

**25.** The method of any one of the claim 18-24, wherein said target is selected from the group consisting of a target of biological origin, a derivatised target of biological origin, a synthetic molecular target, a protein, a small molecular hormone, a lipid, a polysaccharide, a whole cell, a nucleic acid, a metabolite, a heme group, an enzyme, a hormone, a structural element, a regulatory protein; a membrane channel or pump, a part of a signal transducing cascade, an antibody, a kinase enzymes, a phosphatase enzyme, a protease enzyme, a soluble or insoluble agglomerate of one or more proteins and one or more substituents occurring in the body or artificial component. DNA, RNA, an aptamer, and a ribozyme.

**Patentansprüche**

**1.** Verfahren zum Identifizieren eines Anzeige-Moleküls eines bifunktionalen Komplexes, der ferner ein Identifizier-Oligonucleotid umfasst, das mit dem Anzeige-Molekül verbunden ist, wobei das Verfahren die Schritte umfasst:

i) das Bereitstellen einer initialen Bibliothek bifunktionaler Komplexe, die eine Vielzahl von unterschiedlichen

Anzeige-Molekülen umfasst, wobei jedes mit einem Identifizier-Oligonucleotid verbunden ist, das ein Anzeige-Molekül identifiziert,
wobei das Anzeige-Molekül durch ein Verfahren erhalten wird, das die Schritte umfasst

a. das Bereitstellen eines Oligonucleotids, das eine Anhänger-Hinzufügungsstelle und eine oder mehrere reaktive Stelle(n) zur Funktionalisierung umfasst,
b. das Reagieren der einen oder mehreren reaktiven Stellen mit zwei oder mehreren chemischen Einheiten, und
c. für jede chemische Einheit, die mit einer reaktiven Stelle reagiert hat, das Hinzufügen eines einzigartigen Anhängerrestes an die Anhänger-Hinzufügungsstelle,

wodurch ein bifunktionaler Komplex erhalten wird, der ein Anzeige-Molekül umfasst, das von den Reaktionen der chemischen Einheiten herrührt, wobei das Anzeige-Molekül mit einem Identifizier-Oligonucleotid verbunden ist, das die individuellen einzigartigen Anhängerreste umfasst,
ii) das Inkontaktbringen der initialen Bibliothek mit einem Ziel und das Zulassen einer Interaktion zwischen Anzeige-Molekülen der initialen Bibliothek und dem Ziel,
iii) das Abteilen, aus der initialen Bibliothek, von Identifizier-Oligonucleotiden, die Anzeige-Moleküle identifizieren, die mit dem Ziel interagiert haben, wobei die Identifizier-Oligonucleotide in einer einzelsträngigen Form oder in einer doppelsträngigen Form, die komplementäre einzelsträngige Identifizier-Oligonucleotide umfasst, vorliegen,
iv) das Konvertieren von Identifizier-Oligonucleotiden in einzelsträngiger Form zu doppelsträngigen Identifizier-Oligonucleotiden, die komplementäre einzelsträngige Identifizier-Oligonucleotide umfassen, wenn die Identifizier-Oligonucleotide in Schritt iii) in einzelsträngiger Form vorliegen,
v) das Abtrennen von Identifizier-Oligonucleotiden von Anzeige-Molekülen durch Amplifizieren der Oligonucleotide mittels Polymerase-Kettenreaktion oder mittels Spalten der Linker, die Identifizier-Oligonucleotide und Anzeige-Moleküle in den abgeteilten bifunktionalen Komplexen verbinden,
vi) das Entfernen der komplementären einzelsträngigen Identifizier-Oligonucleotide der individuellen Identifizier-Oligonucleotide,
vii) das Hybridisieren der in Schritt vi) erhaltenen einzelsträngigen Identifizier-Oligonucleotide,
wobei eine Zusammensetzung erzeugt wird, die Homoduplex-Identifizier-Oligonucleotide und Heteroduplex-Identifizier-Oligonucleotide umfasst,
wobei Homoduplex-Identifizier-Oligonucleotide hybridisieren, ohne dass Fehlpaarungen zwischen den komplementären Oligonucleotidsträngen entstehen, und
wobei eine oder mehrere Fehlpaarungen vorliegen, wenn komplementäre Identifizier-Oligonucleotide Heteroduplexe bilden,
viii) das Abtrennen der Homoduplexe von Heteroduplexen,
ix) das Erhalten einer Fraktion, die hauptsächlich Homoduplexe umfasst,
x) das Decodieren der Identifizier-Oligonucleotide der Homoduplexe, und
xi) das Identifizieren des einen oder der mehreren Anzeige-Moleküle, die von den decodierten Identifizierer-Oligonucleotiden identifiziert wurden, auf der Basis des Decodierens in Schritt x).

**2.** Verfahren gemäß Anspruch 1, wobei das Duplex-Identifizier-Oligonucleotid, das komplementäre einzelsträngige Identifizier-Oligonucleotide umfasst, durch Hybridisierung eines komplementären Identifizier-Oligonucleotids mit dem Identifizier-Oligonucleotid, das mit dem Anzeige-Molekül des bifunktionalen Komplexes verbunden ist, erhalten wird.

**3.** Verfahren gemäß Anspruch 1, wobei das Duplex-Identifizier-Oligonucleotid, das komplementäre einzelsträngige Identifizier-Oligonucleotide umfasst, durch Hybridisierung eines Sonden-Oligonucleotids mit dem Identifizier-Oligonucleotid des bifunktionalen Komplexes, enzymatisches Erweitern des Sonden-Oligonucleotids, erhalten wird, wobei ein komplementäres Identifizier-Oligonucleotid erhalten wird, das mit dem Identifizier-Oligonucleotid, das mit dem Anzeige-Molekül des bifunktionalen Komplexes verbunden ist, hybridisiert ist.

**4.** Verfahren gemäß einem der Ansprüche 2 und 3, wobei das Duplex-Identifizier-Oligonucleotid, das komplementäre einzelsträngige Identifizier-Oligonucleotide umfasst, vor dem, während des oder begleitend zum Schritt(s) des Abteilens der bifunktionalen Komplexe erhalten wird.

**5.** Verfahren gemäß einem der Ansprüche 2 und 3, wobei das Duplex-Identifizier-Oligonucleotid, das komplementäre einzelsträngige Identifizier-Oligonucleotide umfasst, nach dem Schritt des Abteilens der bifunktionalen Komplexe

erhalten wird.

6. Verfahren gemäß Anspruch 1, wobei das Identifizier-Oligonucleotid, das mit dem Anzeige-Molekül verbunden ist, ausgewählt ist aus der Gruppe bestehend aus a) einem einzelsträngigen Identifizier-Oligonucleotid und b) einem Duplex-Identifizier-Oligonucleotid, das komplementäre einzelsträngige Identifizier-Oligonucleotide umfasst, wobei das Verfahren die vorausgehenden Schritte umfasst:

   i) das Bereitstellen einer Zusammensetzung bifunktionaler Komplexe, die unterschiedliche Anzeige-Moleküle umfassen,
   ii) das Bereitstellen eines oder mehrerer Zielmoleküls (Zielmoleküle), das (die) Affinität zu einem oder mehreren der Anzeige-Moleküle hat (haben),
   iii) das Inkontaktbringen der Zusammensetzung bifunktionaler Komplexe wie bereitgestellt in Schritt i) mit dem Zielmolekül wie bereitgestellt in Schritt ii)
   iv) das Erhalten einer angereicherten Fraktion bifunktionaler Komplexe, die ein oder mehrere Anzeige-Moleküle umfassen, die eine Affinität zu dem Zielmolekül haben,
   v) das Abteilen der in Schritt iv) erhaltenen angereicherten Fraktion bifunktionaler Komplexe, die keine Affinität zu dem Zielmolekül haben, und
   vi) das Komplementieren einzelsträngiger Identifizier-Oligonucleotide unterschiedlicher bifunktionaler Komplexe, in denen das Anzeige-Molekül verbunden ist mit a) einem einzelsträngigen Identifizier-Oligonucleotid, wobei ein Duplex-Identifizier-Oligonucleotid erhalten wird, das komplementäre Identifizier-Oligonucleotide umfasst, mit der Maßgabe, dass keine einzelsträngige Komplementation für bifunktionale Komplexe auftritt, welche b) Duplex-Identifizier-Oligonucleotide umfassen, die komplementäre Identifizier-Oligonucleotide umfassen.

7. Verfahren gemäß Anspruch 6, wobei das Anzeige-Molekül eines bifunktionalen Komplexes wie bereitgestellt in Schritt i) oder wie erhalten in Schritt iv) mit einem einzelsträngigen Identifizier-Oligonucleotid verbunden ist, wobei das Verfahren den zusätzlichen Schritt des Komplementierens der einzelsträngigen Identifizier-Oligonucleotide der unterschiedlichen funktionalen Komplexe umfasst, wobei ein Duplex-Identifizier-Oligonucleotid erhalten wird, das komplementäre Identifizier-Oligonucleotide umfasst, wobei die Komplementation der einzelsträngigen Identifizier-Oligonucleotide der unterschiedlichen bifunktionalen Komplexe vor dem Abteilen der bifunktionalen Komplexe oder nach dem Abteilen der bifunktionalen Komplexe oder begleitend mit dem Abteilen der bifunktionalen Komplexe stattfindet.

8. Verfahren gemäß Anspruch 1, wobei die Heteroduplexe durch enzymatischen Abbau des fehlgepaarten, einzelsträngigen Teils der Heteroduplexe entfernt werden.

9. Verfahren gemäß Anspruch 8, wobei das Enzym eine Nucleaseaktivität umfasst oder aus einem Polypeptid mit Nucleaseaktivität besteht.

10. Verfahren gemäß einem der Ansprüche 8 und 9, wobei das Enzym ausgewählt ist aus der Gruppe von Enzymen bestehend aus T4-Endonuclease VII, T4-Endonuclease I, CEL I, Nuclease S1, wobei Varianten und Kombinationen davon umfasst sind.

11. Verfahren gemäß einem der Ansprüche 1 bis 10, wobei der Linker, der das Anzeige-Molekül und das Identifizier-Oligonucleotid verbindet, selektiv spaltbar ist.

12. Verfahren gemäß einem der Ansprüche 1 bis 10, wobei die Zusammensetzung bifunktionaler Komplexe mehr als 1000 unterschiedliche Anzeige-Moleküle umfasst.

13. Verfahren gemäß einem der Ansprüche 1 bis 10, wobei die abgeteilte Fraktion von Identifizierer-Oligonucleotiden durch PCR amplifiziert wurde.

14. Verfahren gemäß einem der Ansprüche 1 bis 10, wobei die komplementären Identifizier-Oligonucleotidstränge von Homoduplexen und Heteroduplexen in der Fraktion, die hauptsächlich Homoduplexe umfasst, getrennt und zugelassen werden zu hybridisieren, wobei die Hybridisierung zu einer weiteren Fraktion führt, die hauptsächlich Homoduplexe umfasst.

15. Verfahren gemäß einem der vorhergehenden Ansprüche, wobei die Anzeige-Moleküle ein Molekulargewicht von weniger als 2000 Dalton, wie weniger als 1000 Dalton, zum Beispiel weniger als 500 Dalton, aufweisen.

**16.** Verfahren gemäß einem der vorhergehenden Ansprüche, wobei das Inkontaktbringen des Zielmoleküls und der Zusammensetzung unterschiedlicher bifunktionaler Komplexe durch Mischen des Zielmoleküls mit der Zusammensetzung unterschiedlicher bifunktionaler Komplexe erreicht wird.

**17.** Verfahren gemäß Anspruch 16, wobei das Ziel vor dem Inkontaktbringen mit den unterschiedlichen bifunktionalen Komplexen mit einem bekannten Liganden gesättigt wird.

**18.** Verfahren zum Identifizieren eines Anzeige-Moleküls eines bifunktionalen Komplexes, der ferner ein Identifizier-Oligonucleotid umfasst, das mit dem Anzeige-Molekül vorhanden ist, wobei das Verfahren die Schritte umfasst:

i) das Bereitstellen einer initialen Bibliothek bifunktionaler Komplexe, die eine Vielzahl von unterschiedlichen Anzeige-Molekülen umfasst, wobei jedes an ein Identifizier-Oligonucleotid geknüpft ist, das ein Anzeige-Molekül identifiziert,
wobei das Anzeige-Molekül ein kleines Molekül ist,
ii) das Inkontaktbringen der initialen Bibliothek mit einem Ziel und das Zulassen einer Interaktion zwischen Anzeige-Molekülen der initialen Bibliothek und dem Ziel,
iii) das Abteilen, aus der initialen Bibliothek, von Identifizier-Oligonucleotiden, die Anzeige-Moleküle identifizieren, die mit dem Ziel interagiert haben, wobei die Identifizier-Oligonucleotide in einer einzelsträngigen Form oder in einer doppelsträngigen Form, die komplementäre einzelsträngige Identifizier-Oligonucleotide umfasst, vorliegen,
iv) das Konvertieren von Identifizier-Oligonucleotiden in einzelsträngiger Form zu doppelsträngigen Identifizier-Oligonucleotiden, die komplementäre einzelsträngige Identifizier-Oligonucleotide umfassen, wenn die Identifizier-Oligonucleotide in Schritt iii) in einzelsträngiger Form vorliegen,
v) das Abtrennen von Identifizier-Oligonucleotiden von Anzeige-Molekülen durch Amplifizieren der Oligonucleotide mittels Polymerase-Kettenreaktion oder mittels Spalten der Linker, die Identifizier-Oligonucleotide und Anzeige-Moleküle in den abgeteilten bifunktionalen Komplexen verbinden,
vi) das Entfernen der komplementären einzelsträngigen Identifizier-Oligonucleotide der individuellen Identifizier-Oligonucleotide,
vii) das Hybridisieren der in Schritt vi) erhaltenen einzelsträngigen Identifizier-Oligonucleotide,
wobei eine Zusammensetzung erzeugt wird, die Homoduplex-Identifizier-Oligonucleotide und Heteroduplex-Identifizier-Oligonucleotide umfasst,
wobei Homoduplex-Identifizier-Oligonucleotide hybridisieren, ohne dass Fehlpaarungen zwischen den komplementären Oligonucleotidsträngen entstehen, und
wobei eine oder mehrere Fehlpaarungen vorliegen, wenn komplementäre Identifizier-Oligonucleotide Heteroduplexe bilden,
viii) das Abtrennen der Homoduplexe von Heteroduplexen,
ix) das Erhalten einer Fraktion, die hauptsächlich Homoduplexe umfasst,
x) das Decodieren der Identifizier-Oligonucleotide der Homoduplexe, und
xi) das Identifizieren des einen oder der mehreren Anzeige-Moleküle, die von den decodierten Identifizier-Oligonucleotiden identifiziert wurden, auf der Basis des Decodierens in Schritt x).

**19.** Verfahren gemäß Anspruch 18, wobei das kleines-Molekül-Anzeige-Molekül ein Arzneimittelkandidat ist.

**20.** Verfahren gemäß Anspruch 18 oder 19, wobei das Anzeige-Molekül ein eingerüstetes Molekül ist.

**21.** Verfahren gemäß einem der Ansprüche 18 bis 20, wobei das Anzeige-Molekül eine künstliche Substanz ist.

**22.** Verfahren gemäß einem der Ansprüche 18 bis 21, wobei das Anzeige-Molekül ein Molekulargewicht von weniger als 2000 Dalton, wie weniger als 1000 Dalton, zum Beispiel weniger als 500 Dalton, aufweist.

**23.** Verfahren gemäß einem der Ansprüche 18 bis 22, wobei das Anzeige-Molekül durch Reaktion von 4 bis 8 chemischen Einheiten synthetisiert wird.

**24.** Verfahren gemäß einem der Ansprüche 18 bis 23, wobei das Anzeige-Molekül eine Affinität zu dem Ziel aufweist und das Anzeigemolekül einen Kd-Wert für das Ziel von weniger als 100 μM, wie weniger als 10 μM, wie weniger als 1 μM, wie weniger als 100 nM, wie weniger als 10 nM, wie weniger als 1 nM, aufweist.

**25.** Verfahren gemäß einem der Ansprüche 18 bis 24, wobei das Ziel ausgewählt ist aus der Gruppe bestehend aus

einem Ziel biologischen Ursprungs, einem derivatisierten Ziel biologischen Ursprungs, einem synthetischen molekularen Ziel, einem Protein, einem niedermolekularem Hormon, einem Lipid, einem Polysaccharid, einer ganzen Zelle, einer Nucleinsäure, einem Metaboliten, einer Häm-Gruppe, einem Enzym, einem Hormon, einem Strukturelement, einem regulatorischen Protein, einem/einer Membrankanal oder -pumpe, einem Teil einer Signal-transduzierender Kaskade, einem Antikörper, einem Kinase-Enzym, einem Phosphatase-Enzym, einem Protease-Enzym, einem löslichen oder unlöslichen Agglomerat von einem oder mehreren Proteinen und einem oder mehreren Substituenten, wie sie im Körper oder in künstlichen Komponenten vorkommen, DNA, RNA, einem Aptamer und einem Ribozym.

**Revendications**

**1.** Procédé d'identification d'une molécule de présentation d'un complexe bifonctionnel comprenant en outre un oligonucléotide identificateur lié à la molécule de présentation, ledit procédé comprenant les étapes consistant à

i) fournir une banque initiale de complexes bifonctionnels comprenant une pluralité de molécules de présentation différentes chacune liée à un oligonucléotide identificateur identifiant une molécule de présentation,
où la molécule de présentation est obtenue par un procédé comprenant les étapes consistant à

a. fournir un oligonucléotide comprenant un site d'addition de marqueur et un ou plusieurs sites réactifs pour la fonctionnalisation,
b. faire réagir ledit un ou lesdits plusieurs sites réactifs avec deux entités chimiques ou plus, et
c. ajouter pour chaque entité chimique qui a réagi avec un site réactif un résidu marqueur unique au site d'addition de marqueur,
obtenant de cette manière un complexe bifonctionnel comprenant une molécule de présentation résultant des réactions des entités chimiques, ladite molécule de présentation étant liée à un oligonucléotide identificateur comprenant les résidus marqueurs uniques individuels,

ii) mettre la banque initiale en contact avec une cible et permettre une interaction entre les molécules de présentation de la banque initiale et la cible,
iii) séparer, à partir de ladite banque initiale, les oligonucléotides identificateurs identifiant les molécules de présentation ayant interagi avec la cible, où les oligonucléotides identificateurs sont sous une forme simple brin ou sous une forme double brin comprenant des oligonucléotides identificateurs simple brin complémentaires,
iv) convertir les oligonucléotides identificateurs sous forme simple brin en oligonucléotides identificateurs double brin comprenant des oligonucléotides identificateurs simple brin complémentaires, lorsque les oligonucléotides identificateurs dans l'étape iii) sont sous une forme simple brin,
v) séparer les oligonucléotides identificateurs des molécules de présentation par amplification des oligonucléotides par des réactions en chaîne de la polymérase ou par clivage des lieurs liant les oligonucléotides identificateurs et les molécules de présentation dans les complexes bifonctionnels séparés,
vi) déplacer les oligonucléotides identificateurs simple brin complémentaires des oligonucléotides identificateurs individuels,
vii) hybrider les oligonucléotides identificateurs simple brin obtenus dans l'étape vi),
générant de cette manière une composition comprenant des homo-duplex d'oligonucléotides identificateurs et des hétéro-duplex d'oligonucléotides identificateurs,
où les homo-duplex d'oligonucléotides identificateurs s'hybrident sans générer de mésappariements quelconques entre les brins oligonucléotidiques complémentaires, et
où un ou plusieurs mésappariements sont présents lorsque les oligonucléotides identificateurs complémentaires forment des hétéro-duplex,
viii) séparer les homo-duplex des hétéro-duplex,
ix) obtenir une fraction comprenant principalement des homo-duplex,
x) décoder les oligonucléotides identificateurs des homo-duplex, et
xi) identifier, en se basant sur le décodage dans l'étape x), la une ou les plusieurs molécules de présentation identifiées par les oligonucléotides identificateurs décodés.

**2.** Procédé selon la revendication 1, dans lequel le duplex d'oligonucléotides identificateurs comprenant des oligonucléotides identificateurs simple brin complémentaires est obtenu par hybridation d'un oligonucléotide identificateur complémentaire avec l'oligonucléotide identificateur lié à la molécule de présentation du complexe bifonctionnel.

3. Procédé selon la revendication 1, dans lequel le duplex d'oligonucléotides identificateurs comprenant des oligonucléotides identificateurs simple brin complémentaires est obtenu par hybridation d'une sonde oligonucléotidique avec l'oligonucléotide identificateur du complexe bifonctionnel, étendant de manière enzymatique ladite sonde oligonucléotidique, obtenant de cette manière un oligonucléotide identificateur complémentaire hybridé avec l'oligonucléotide identificateur lié à la molécule de présentation du complexe bifonctionnel.

4. Procédé selon l'une quelconque des revendications 2 et 3, dans lequel ledit duplex d'oligonucléotides identificateurs comprenant des oligonucléotides identificateurs simple brin complémentaires est obtenu avant, durant ou simultanément à l'étape de séparation des complexes bifonctionnels.

5. Procédé selon l'une quelconque des revendications 2 et 3, dans lequel ledit duplex d'oligonucléotides identificateurs comprenant des oligonucléotides identificateurs simple brin complémentaires est obtenu après l'étape de séparation des complexes bifonctionnels.

6. Procédé selon la revendication 1, dans lequel l'oligonucléotide identificateur lié à la molécule de présentation est choisi dans le groupe comprenant a) un oligonucléotide identificateur simple brin et b) un duplex d'oligonucléotides identificateurs comprenant des oligonucléotides identificateurs simple brin complémentaires, ledit procédé comprenant les étapes préliminaires consistant à i) fournir une composition de complexes bifonctionnels comprenant des molécules de présentation différentes,

   ii) fournir une ou plusieurs molécules cibles présentant une affinité pour une ou plusieurs desdites molécules de présentation,
   iii) mettre la composition des complexes bifonctionnels fournie dans l'étape i) en contact avec la molécule cible fournie dans l'étape ii),
   iv) obtenir une fraction enrichie de complexes bifonctionnels comprenant une ou plusieurs molécules de présentation présentant une affinité pour ladite molécule cible,
   v) séparer ladite fraction enrichie obtenue dans l'étape iv) des complexes bifonctionnels qui ne présentent pas d'affinité pour ladite molécule cible, et
   vi) complémenter les oligonucléotides identificateurs simple brin des différents complexes bifonctionnels dans lesquels la molécule de présentation est liée à a) un oligonucléotide identificateur simple brin, obtenant de cette manière un duplex d'oligonucléotides identificateurs comprenant des oligonucléotides identificateurs complémentaires, à condition qu'aucune complémentation simple brin ne se produise pour les complexes bifonctionnels comprenant b) des duplex d'oligonucléotides comprenant des oligonucléotides identificateurs complémentaires.

7. Procédé selon la revendication 6, dans lequel la molécule de présentation d'un complexe bifonctionnel fourni dans l'étape i) ou obtenu dans l'étape iv) est liée à un oligonucléotide identificateur simple brin, ledit procédé comprenant l'étape supplémentaire consistant à complémenter lesdits oligonucléotides identificateurs simple brin des différents complexes bifonctionnels, obtenant de cette manière un duplex d'oligonucléotides identificateurs comprenant des oligonucléotides identificateurs complémentaires, où ladite complémentation desdits oligonucléotides identificateurs simple brin des différents complexes bifonctionnels prend place avant la séparation des complexes bifonctionnels ou après la séparation des complexes bifonctionnels ou simultanément à la séparation des complexes bifonctionnels.

8. Procédé selon la revendication 1, dans lequel les hétéro-duplex sont éliminés par une dégradation enzymatique de la partie simple brin mésappariée des hétéro-duplex.

9. Procédé selon la revendication 8, dans lequel l'enzyme comprend une activité nucléase ou est constituée d'un polypeptide possédant une activité nucléase.

10. Procédé selon l'une quelconque des revendications 8 et 9, dans lequel l'enzyme est choisie dans le groupe d'enzymes comprenant la T4 endonucléase VII, la T4 endonucléase I, la CEL I, la nucléase S1, y compris des variants et des combinaisons de celles-ci.

11. Procédé selon l'une quelconque des revendications 1 à 10, dans lequel le lieur liant la molécule de présentation et l'oligonucléotide identificateur est clivable de manière sélective.

12. Procédé selon l'une quelconque des revendications 1 à 10, dans lequel la composition de complexes bifonctionnels comprend plus de 1000 molécules de présentation différentes.

**13.** Procédé selon l'une quelconque des revendications 1 à 10, dans lequel la fraction séparée des oligonucléotides identificateurs est amplifiée par PCR.

**14.** Procédé selon l'une quelconque des revendications 1 à 10, dans lequel les brins des oligonucléotides identificateurs complémentaires des homo-duplex et des hétéro-duplex dans la fraction comprenant principalement des homo-duplex sont séparés et laissés s'hybrider, ladite hybridation entraînant une autre fraction comprenant principalement des homo-duplex.

**15.** Procédé selon l'une quelconque des revendications précédentes, dans lequel les molécules de présentation ont une masse moléculaire inférieure à 2000 daltons, comme inférieure à 1000 daltons, par exemple inférieure à 500 daltons.

**16.** Procédé selon l'une quelconque des revendications précédentes, dans lequel la mise en contact de la molécule cible et de la composition des différents complexes bifonctionnels est obtenue en mélangeant la molécule cible avec la composition des différents complexes bifonctionnels.

**17.** Procédé selon la revendication 16, dans lequel la cible est saturée avec un ligand connu avant la mise en contact des différents complexes bifonctionnels.

**18.** Procédé d'identification d'une molécule de présentation d'un complexe bifonctionnel comprenant en outre un oligonucléotide identificateur lié à la molécule de présentation, ledit procédé comprenant les étapes consistant à

i) fournir une banque initiale de complexes bifonctionnels comprenant une pluralité de molécules de présentation différentes chacune liée à un oligonucléotide identificateur identifiant une molécule de présentation, où ladite molécule de présentation est une petite molécule,
ii) mettre la banque initiale en contact avec une cible et permettre une interaction entre les molécules de présentation de la banque initiale et la cible,
iii) séparer, à partir de ladite banque initiale, les oligonucléotides identificateurs identifiant les molécules de présentation ayant interagi avec la cible, où les oligonucléotides identificateurs sont sous une forme simple brin ou sous une forme double brin comprenant des oligonucléotides identificateurs simple brin complémentaires,
iv) convertir les oligonucléotides identificateurs sous forme simple brin en oligonucléotides identificateurs double brin comprenant des oligonucléotides identificateurs simple brin complémentaires, lorsque les oligonucléotides identificateurs dans l'étape iii) sont sous une forme simple brin,
v) séparer les oligonucléotides identificateurs des molécules de présentation par amplification des oligonucléotides par des réactions en chaîne de la polymérase ou par clivage des lieurs liant les oligonucléotides identificateurs et les molécules de présentation dans les complexes bifonctionnels séparés,
vi) déplacer les oligonucléotides identificateurs simple brin complémentaires des oligonucléotides identificateurs individuels,
vii) hybrider les oligonucléotides identificateurs simple brin obtenus dans l'étape vi),
générant de cette manière une composition comprenant des homo-duplex d'oligonucléotides identificateurs et des hétéro-duplex d'oligonucléotides identificateurs,
où les homo-duplex d'oligonucléotides identificateurs s'hybrident sans générer de mésappariements quelconques entre les brins oligonucléotidiques complémentaires, et
où un ou plusieurs mésappariements sont présents lorsque les oligonucléotides identificateurs complémentaires forment des hétéro-duplex,
viii) séparer les homo-duplex des hétéro-duplex,
ix) obtenir une fraction comprenant principalement des homo-duplex,
x) décoder les oligonucléotides identificateurs des homo-duplex, et
xi) identifier, en se basant sur le décodage dans l'étape x), la une ou les plusieurs molécules de présentation identifiées par les oligonucléotides identificateurs décodés.

**19.** Procédé selon la revendication 18, dans lequel ladite petite molécule de présentation est un médicament candidat.

**20.** Procédé selon la revendication 18 ou 19, dans lequel ladite molécule de présentation est une molécule d'échafaudage.

**21.** Procédé selon l'une quelconque des revendications 18 à 20, dans lequel ladite molécule de présentation est une substance artificielle.

**22.** Procédé selon l'une quelconque des revendications 18 à 21, dans lequel ladite molécule de présentation a une masse moléculaire inférieure à 2000 daltons, comme inférieure à 1000 daltons, par exemple inférieure à 500 daltons.

**23.** Procédé selon l'une quelconque des revendications 18 à 22, dans lequel ladite molécule de présentation est synthétisée en faisant réagir 4 à 8 entités chimiques.

**24.** Procédé selon l'une quelconque des revendications 18 à 23, dans lequel ladite molécule de présentation présente une affinité pour ladite cible et ladite molécule de présentation a une valeur de Kd pour ladite cible inférieure à 100 μM, comme inférieure à 10 μM, comme inférieure à 1 μM, comme inférieure à 100 nm, comme inférieure à 10 nM, comme inférieure à 1 nM.

**25.** Procédé selon l'une quelconque des revendications 18 à 24, dans lequel ladite cible est choisie dans le groupe comprenant une cible d'origine biologique, une cible dérivatisée d'origine biologique, une cible moléculaire synthétique, une protéine, une petite hormone moléculaire, un lipide, un polysaccharide, une cellule entière, un acide nucléique, un métabolite, un groupe hème, une enzyme, une hormone, un élément structural, une protéine régulatrice, un canal ou une pompe membranaire, une partie d'une cascade de transduction de signaux, un anticorps, une enzyme kinase, une enzyme phosphatase, une enzyme protéase, un agglomérat soluble ou insoluble d'une ou de plusieurs protéines et d'un ou de plusieurs substituants existant dans le corps ou composants artificiels, de l'ADN, de l'ARN, un aptamère et un ribozyme.

Figure 1. Selection generate specific hits and a diverse set of background binders

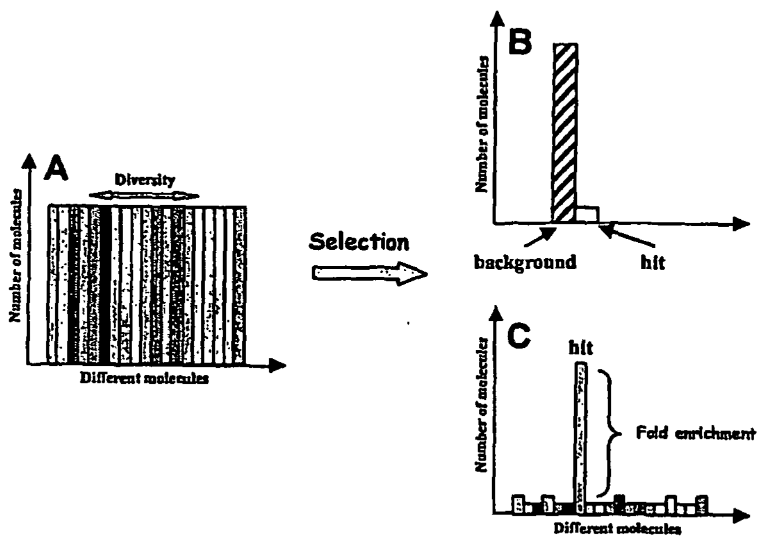

Figure 2. Homo- and heteroduplexes

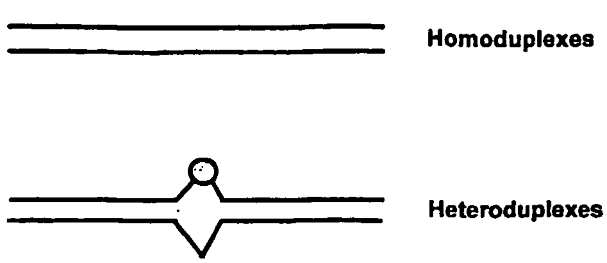

Figure 3. Homo- and Hetero-duplex formation and separation

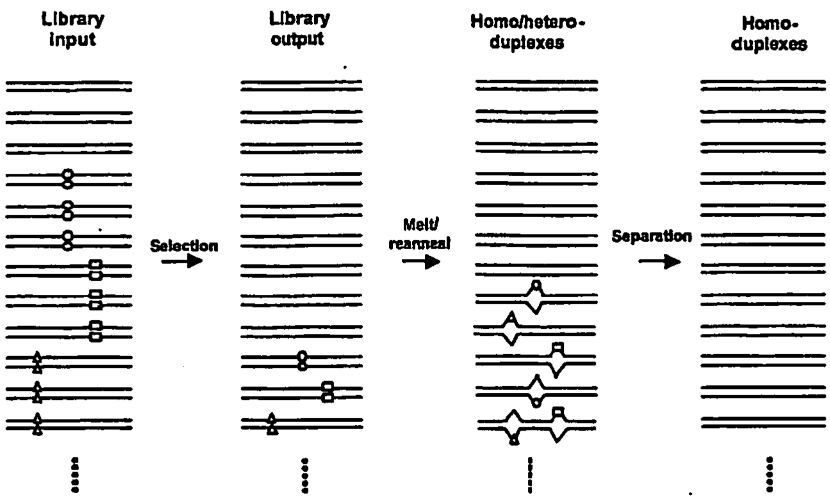

Figure 4. The overall principle of mismatch selection (MISE)

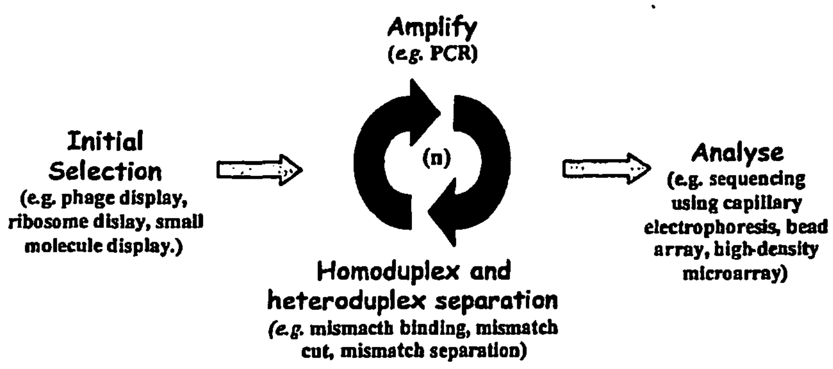

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02103008 A2
- WO 9831700 A
- WO 9303172 A
- WO 0023458 A
- EP 643778 B1
- WO 9306121 A1
- WO 2004099441 A
- WO 9641011 A
- US 5795976 A
- WO 9202536 A
- WO 9105058 A
- US 6194550 B
- WO 0032823 A
- WO 0190414 A
- WO 9320242 A
- WO 9306121 A
- WO 02074929 A
- WO 02103008 A
- DK 0300739 W
- DK PA200300430
- US 5432272 A, Benner
- WO 03078627 A2
- WO 03078626 A2
- WO 03078445 A2
- WO 03078446 A2
- US 5824471 A
- WO 02103008 PCT
- WO 2004083427 A
- WO 2004056994 A
- WO 2004039825 A


### Non-patent literature cited in the description


- **Fack, F. et al.** Heteroduplex mobility assay (HMA) pre-screening: An improved strategy for the rapid identification of inserts selected from phage displayed peptide libraries. *Mol. Divers.,* 2000, vol. 5 (1), 7-12
- Encoded combinatorial chemistry. *Proc. Natl. Acad. Sci. (US),* 1992, vol. 89, 5381-5383
- **M. Yonezawa et al.** *Nucleic acid research,* 2003, vol. 31 (19), e118
- **Holmes CP.** *J. Org. Chem.,* 1997, vol. 62, 2370-2380
- **Rajasekharan Pillai.** *V. N. Synthesis,* 1980, 1-26
- **Rajasekharan Pillai, V. N.** *Synthesis,* 1980, 1-26
- **R. O. Schoenleber ; B. Giese.** *Synlett,* 2003, 501-504
- **Kirley, T.L.** Reduction and fluorescent labeling of cyst(e)ine-containing proteins for subsequent structural analysis. *Anal. Biochem.,* 1989, vol. 180, 231
- **Levison, M.E. et al.** Reduction of biological substances by water-soluble phosphines: Gamma-globulin. *Experentia,* 1969, vol. 25, 126-127
- **Wagner.** *Nucleic Acids Research,* 1995, vol. 22, 1541-1547
- **Hsu.** *Carcinogenesis,* 1994, vol. 15, 1657-62
- **Myers et al.** *Science,* 1985, vol. 230, 1242-6
- **Grange et al.** *Nucleic Acids Research,* 1990, vol. 18, 4227-36
- **Mashal et al.** *Nature Genetics,* 1995, vol. 9, 177-83
- **Cotton et al.** *Proc Natl Acad Sci (US),* 1988, vol. 85, 4397-401
- **Liu W et al.** *Nucleic Acids Research,* 1998, vol. 26, 1396-1400
- **O'Donovan MC et al.** *Genomics,* 1998, vol. 52, 4449